(19) Europäisches Patentamt
European Patent Office
Office européen des brevets

(11) Publication number: **0 336 356**
**A2**

(12) **EUROPEAN PATENT APPLICATION**

(21) Application number: 89105864.6

(22) Date of filing: 04.04.89

(51) Int. Cl.4: C07D 209/42 , C07D 209/20 , C07D 401/12 , C07D 401/14 , C07D 403/12 , C07D 487/02 , A61K 31/40 , A61K 31/44 , A61K 31/47 , A61K 31/415 , A61K 31/495

(30) Priority: 05.04.88 US 177715

(43) Date of publication of application: 11.10.89 Bulletin 89/41

(84) Designated Contracting States: ES GR

(71) Applicant: ABBOTT LABORATORIES
One Abbott Park Road
Abbott Park, IL 60064-3500(US)

(72) Inventor: Kerwin, James F.
1301 Hampton Lane
Mundelein Illinois 60060(US)
Inventor: Nadzan, Alex M.
1690 Young Drive
Libertyville Illinois 60048(US)

(74) Representative: Modiano, Guido et al
MODIANO, JOSIF, PISANTY & STAUB
Modiano & Associati Via Meravigli, 16
I-20123 Milan(IT)

(54) **Derivatives of tryptophan as CCK antagonists.**

(57) A compound of the formula:

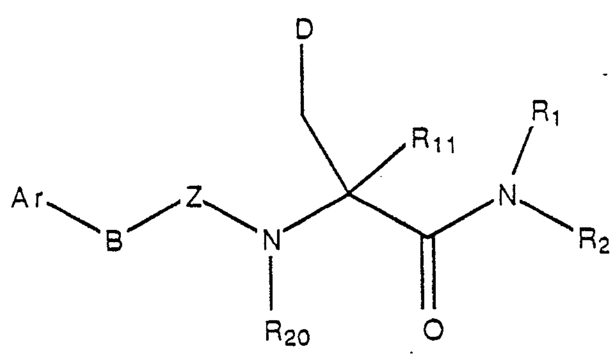

wherein $R_1$ and $R_2$ are independently selected from hydrogen, loweralkyl, cycloalkyl, loweralkenyl, adamantyl, aryl, substituted aryl, heterocyclic group, substituted alkyl, substituted amide, functionalized carbonyl, and nitrogen containing ring wherein $R_1$, $R_2$ and the adjacent nitrogen atom form a ring;
$R_{11}$ is hydrogen, loweralkyl, or loweralkenyl;

EP 0 336 356 A2

$R_{20}$ is hydrogen, loweralkyl, or loweralkenyl;
B is $-(CH_2)_m-$, substituted alkenylene, $-QCH_2-$wherein Q is O, S, NH or substituted amino, $-CH_2Q-$wherein Q is as defined or B is NH;
Z is C = O, $S(O)_2$, or C = S;
Ar is a heterocyclic group, aryl or substituted aryl;
D is unsubstituted or substituted indol-3-yl, indolin-3-yl or oxindol-3-yl; and m is 0 to 4;
or a pharmaceutically acceptable salt thereof.

# DERIVATIVES OF TRYPTOPHAN AS CCK ANTAGONISTS

## Technical Field

This is a continuation-in-part of U.S. patent application Serial No. 177,715, filed April 5, 1988.

The present invention relates to novel organic compounds and compositions which antagonize cholecystokinin and gastrin, processes for making such compounds, synthetic intermediates employed in these processes and a method for treating gastrointestinal disorders, central nervous system disorders, cancers of the pancreas and the gall bladder, hypoinsulinemia, or potentiating analgesia, or regulating appetite with such compounds.

## Background of the Invention

Cholecystokinins (CCK) are a family of amino acid polypeptide hormones. CCK and a 33 amino acid fragment of CCK ($CCK_{33}$) were first isolated from hog intestine. (Matt and Jorpes, Biochem J. 125 628 (1981)). Recently the $CCK_{33}$ fragment has been found in the brain, where it appears to be the precursor of two smaller fragments, an octapeptide $CCK_8$ and a tetrapeptide $CCK_4$. (Dockray, Nature 264 4022 (1979)).

$CCK_8$, the carboxyl terminal octapeptide fragment of CCK, is the smallest CCK fragment that remains fully biologically active. (Larsson and Rehfeld, Brain Res. 165 201-218 (1979)). The localization of CCK fragments in the cortex of the brain suggests that CCK may be an important neuromodulator of memory, learning and control of primary sensory and motor functions. CCK and its fragments are believed to play an important role in appetite regulation and satiety. (Della-Fera, Science 206 471 (1979); Gibbs et al., Nature 289 599(1981); and Smith, Eating and Its Disorders, eds., Raven Press, New York, 67 (1984)).

CCK antagonists (B.J. Gertz in Neurology and Neurobiology Vol 47, Cholecystokinin Antagonists, Wang and Schoenfeld eds., Alan R. Liss, Inc., New York, NY, 327-342, (1988); Silverman, et al., Am. J. Gastroent. 82 703-8 (1987)) are useful in the treatment and prevention of CCK-related disorders of the gastrointestinal (GI) (Lotti, et al., J. Pharm. Exp. Therap. 241 103-9 (1987)), central nervous (CNS). (Panerai, et al., Neuropharmacology 26 1285-87 (1987)) and appetite regulatory systems of animals, especially man. CCK antagonists are also useful in potentiating and prolonging opiate induced analgesia and thus have utility in the treatment of pain. (Faris et al., Science 226 1215 (1984); Rovati, et al., Clinical Research 34 406A (1986); Dourish, et al, European J. Pharmacology 147 469-72 (1988)). CCK antagonists are also useful for the treatment of disorders of gastric emptying (Stenikar, et al., Arzn. Forsch./Drug Research 37(II) 1168-71 (1987)), gastroesophageal reflux disease, pancreatitis, pancreatic and gastric carcinomas (Douglas, et al., Gastroent. 96 4629 (1989); Beauchamp, et al., Am. Surg. 202 313-9 (1985)), disorders of bowel motility, biliary dyskinesia, anorexia nervosa, hypoglycemia (Rossetti, Diabetes 36 1212-15 (1987); Reagan, Eur. J. Pharmacol. 14 241-3 (1987), gallbladder disease, and the like.

Previously four distinct chemical classes of CCK receptor antagonists have been reported. The first class comprises derivatives of cyclic nucleotides as represented by dibutyryl cyclic GMP (N. Barlas et al., Am. J. Physiol., 242, G161 (1982) and references therein). The second class is represented by C-terminal fragments of CCK (see Jensen et al. Biochem Biophys. Acta, 757, 250 (1983) and Spanarkel J Biol Chem 258, 6746 (1983)). The third class comprises amino acid derivatives of glutamic acid and tryptophan as indicated by proglumide and benzotript (see Hahne et al. Proc. Natl. Acad. Sci. U.S.A., 78, 6304 (1981) and Jensen et al. Biochem. Biophys. Acta. 761, 269 (1983)). The fourth and most recent class is comprised of 3-substituted benzodiazepines, represented by L-364,718 (see: Evans et al. Proc. Natl. Acad. Sci. U.S.A., 83 4918 (1986)).

Most of the previously known CCK antagonists are relatively weak antagonists of CCK demonstrating $IC_{50}$s between $10^{-4}$ and $10^{-6}$ M. The benzodiazepine CCK antagonists or their metabolites may have undesirable effects in vivo due to their interaction with benzodiazepine receptors.

The C-terminal pentapeptide fragment of CCK is the same as the C-terminal pentapeptide fragment of another polypeptide hormone, gastrin. Gastrin, like CCK, exists in both the GI system. Gastrin antagonists are useful in the treatment and prevention of gastrin-related disorders of the GI system such as ulcers, Zollinger-Ellison syndrome and central G cell hyperplasia. (Morley, Gut Pept. Ulcer Proc., Hiroshima Symp. 2nd, 1, (1983)). Bock,et al., J. Med. Chem. 32 13-16 (1989) discloses receptor antagonists of the in vitro effects of gastrin.

## Summary of the Invention

It has now been found that the compounds of the invention are antagonists of cholecystokinin (CCK) and bind specifically to CCK receptors. These CCK antagonists are useful in the treatment and prevention of CCK-related disorders of the gastrointestinal, central nervous and appetite regulatory systems of animals and humans. These compounds are useful in the treatment and prevention of gastrointestinal ulcers, cancers of the gall bladder and pancreas, pancreatitis, Zollinger-Ellison syndrome, central G cell hyperplasia, irritable bowel syndrome, the treatment or prevention of neuroleptic disorders, tardive dyskinesia, Parkinson's disease, psychosis, Gilles de la Tourette syndrome, disorders of appetite regulatory systems, the treatment of pain and the treatment of substance abuse.

## Disclosure of the Invention

In accordance with the present invention, there are cholecystokinin antagonists of the formula:

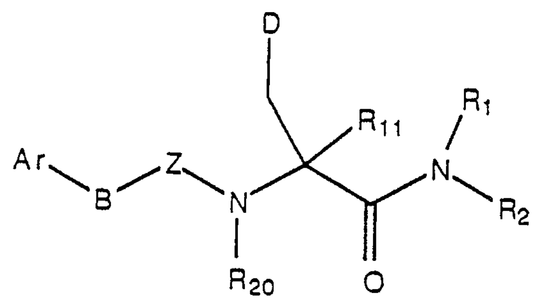

Formula I

$R_1$ and $R_2$ are independently selected from

i) hydrogen,
ii) loweralkyl,
iii) cycloalkyl,
iv) loweralkenyl,
v) adamantyl,
vi) aryl,
vii) substituted aryl,
viii) heterocyclic group,
ix) substituted alkyl,
x) substituted amide,
xi) functionalized carbonyl, and
xii) nitrogen containing ring wherein $R_1$, $R_2$ and the adjacent nitrogen atom form a ring.

Substituted alkyl groups are selected from the group consisting of

i) $-(CH_2)_{1-4}$cycloalkyl,
ii) $-(CH_2)_{1-4}CN$,
iii) alkoxyalkyl,
iv) $-(CH_2)_{1-4}(C=O)_rNR_6R_7$ wherein $R_6$ and $R_7$, are independently selected from hydrogen, loweralkyl, cycloalkyl, loweralkenyl, $-(CH_2)_m$aryl, $-(CH_2)_m$(substituted aryl)
wherein the aryl group is substituted with one, two or three substituents independently selected from loweralkyl, alkoxy, thioalkoxy, carboxy, carboalkoxy, cyano, haloalkyl, $-N_3$, $-NHP_4$ wherein $P_4$ is an N-protecting group, $-OP_5$ wherein $P_5$ is an O-protecting group, nitro, halogen, hydroxy, amino, alkylamino and dialkylamino;
v) $-(C(R_{21})(R_{18}))_{1-4}$aryl wherein $R_{21}$ is hydrogen, $-(C_1-C_6$loweralkyl), loweralkenyl, $-(O)_t(CH_2)_m$carboxyl, $-(O)_t(CH_2)_m$carboalkoxy, $-(O)_t(CH_2)_m$carboaryloxy, haloalkyl, nitro, alkoxy, aryloxy, thioalkoxy, thioaryloxy, halogen, CN, -OH, $-NH_2$ $-NR_6R_7$ wherein $R_6$ and $R_7$ are independently as defined above, $--(CH_2)_mOR_4$, and $-(CH_2)_mSR_4$
wherein $R_4$ is hydrogen, $-(C=O)_r$loweralkyl, $-(C=O)_r$loweralkenyl, $-(C=O)_r$cycloalkyl, $-(C=O)_r(CH_2)_m$aryl or

$-(C=O)_r(CH_2)_m$(substituted aryl); and
$R_{18}$ is hydrogen, $-(C_1-C_6$loweralkyl), loweralkenyl, $-(O)_t(CH_2)_m$carboxyl, $-(O)_t(CH_2)_m$carboalkoxy, $-(O)_t(CH_2)_m$carboaryloxy, haloalkyl, nitro, alkoxy, aryloxy, thioalkoxy, thioaryloxy, halogen, CN, -OH, $-NH_2$, $-NR_6R_7$ wherein $R_6$ and $R_7$ are independently as defined above, $-(CH_2)_mOR_4$ wherein $R_4$ is independently as defined above, and $-(CH_2)_mSR_4$ wherein $R_4$ is independently as defined above,

vi) $-(C(R_{21})(R_{18}))_{1-4}$(substituted aryl) wherein $R_{21}$ and $R_{18}$ are defined independently as above,

vii) $-(C(R_{21})(R_{18}))_{1-4}C(O)R_3$, wherein $R_{21}$ and $R_{18}$ are independently as defined above and wherein $R_3$ is -OH, $-OR_4$ wherein $R_4$ is independently as defined above, $NR_6R_7$ wherein $R_6$ and $R_7$ are independently as defined above, or $-NHR_4$ wherein $R_4$ is as defined above, with the proviso that when $R_3$ is $NR_6R_7$ then $R_{21}$ and $R_{18}$ are not both hydrogen and

viii) $-(C(R_{21})(R_{18}))_{1-4}R_{12}$ wherein $R_{21}$ and $R_{18}$ are independently as defined above and $R_{12}$ is a heterocyclic group. Substituted amide groups are selected from $-(C=O)NR_6R_7$ wherein
$R_6$ and $R_7$, are independently selected from hydrogen, loweralkyl, cycloalkyl, loweralkenyl, $-(CH_2)_m$aryl, $--(CH_2)_m$(substituted aryl)
wherein the aryl group is substituted with one, two or three substituents independently selected from loweralkyl, alkoxy, thioalkoxy, carboxy, carboalkoxy, cyano, haloalkyl, $-N_3$, $-NHP_4$ wherein $P_4$ is an N-protecting group, $-OP_5$ wherein $P_5$ is an O-protecting group, nitro, halogen, hydroxy, amino, alkylamino and dialkylamino. Functionalized carbonyl groups are selected from $-C(O)R_3$ wherein $R_3$ is -OH, $-OR_4$ wherein $R_4$ is independently as defined above, $-NR_6R_7$ wherein $R_6$ and $R_7$ are independently as defined above, or $-NHR_4$ wherein $R_4$ is independently as defined above.

Nitrogen containing rings wherein $R_1$, $R_2$ and the adjacent nitrogen atom form a ring are represented by the formula

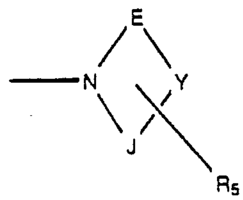

wherein E and J are independently selected from $-(CH_2)_p-$, $-(CH=CH)_r-$, $-YCH_2-$, $CH_2Y-$, -C(O)Y- and -YC(O)- wherein
Y is S, O, $CH_2$, or $-N(R_8)-$, wherein $R_8$ is selected from hydrogen, $-(C=O)_r(C_1-C_6$loweralkyl), $-(C=O)_r$-cycloalkyl, $-(C=O)_r$loweralkenyl, $-(C=O)_r(CH_2)_m$aryl, $-(C=O)_r(CH_2)_m$(substituted aryl) wherein substituted aryl is as defined above, $-(CH_2)_mSR_4$ and $-(CH_2)_mOR_4$ wherein
$R_4$ is hydrogen, $-(C=O)_r$loweralkyl, $-(C=O)_r$loweralkenyl, $-(C=O)_r$cycloalkyl, $-(C=O)_r(CH_2)_m$aryl or $-(C=O)_r(CH_2)_m$(substituted aryl)
wherein substituted aryl is as defined above, and wherein $R_5$ is hydrogen or $R_5$ is one, two or three of the substituents independently selected from the group $-(C_1-C_6$loweralkyl), loweralkenyl, $-(O)_t(CH_2)_m$carboxyl, $--(O)_t(CH_2)_m$carboalkoxy, $-(O)_t(CH_2)_m$carboaryloxy, haloalkyl, nitro, alkoxy, cyano, $-N_3$, $-NHP_4$, $-OP_5$, alkylaminocarbonyl, dialkylaminocarbonyl, aryloxy, thioalkoxy, thioaryloxy, halogen, CN, -OH, $-NH_2$, $-NR_6R_7$ wherein $R_6$ and $R_7$ are independently as defined above, $-(CH_2)_mOR_4$ wherein $R_4$ is independently as defined above, and $-(CH_2)_mSR_4$ wherein $R_4$ is independently as defined above.

$R_{11}$ is
i) hydrogen,
ii) loweralkyl, or
iii) loweralkenyl.

$R_{20}$ is
i) hydrogen,
ii) loweralkyl, or
iii) loweralkenyl.

B is
i) $-(CH_2)_m-$,
ii) substituted alkenylene,

iii) $-QCH_2-$ wherein Q is O, S, or $-N(R_8)-$ wherein $R_8$ is as defined above,
iv) $-CH_2Q-$ wherein Q is as defined above, or
v) NH.

Substituted alkenylene is represented by the formula $-(CR_{21}=CR_{18})_q-$ wherein $R_{21}$ and $R_{18}$ are independently as defined above.

Z is
i) C=O,
ii) $S(O)_2$ ,
or
iii) C=S.

Ar is a heterocyclic group, aryl or substituted aryl.

D is group of the formula

a)

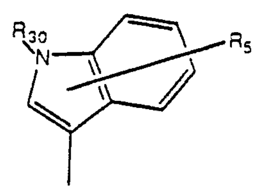

b)

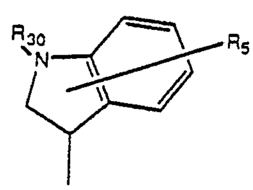

or

c)

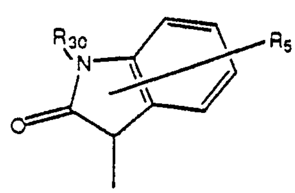

wherein $R_{30}$ is hydrogen, loweralkyl, arylalkyl, (substituted aryl)alkyl, or an N-protecting group; and $R_5$ is independently as defined above.

The subscripts are independently selected at each occurrence from the following values: n is 1 to 3; m is 0 to 4; p is 0 to 2; q is o to 1; r is 0 to 1; and t is 0 to 1.

In particular the compounds of the invention are cholecystokinin antagonists of the formula:

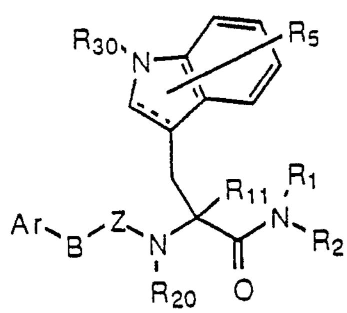

or

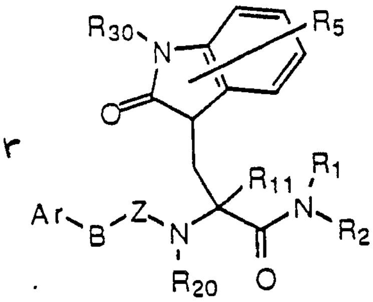

Formula I

wherein

a) $R_1$ and $R_2$ are independently selected from

i) hydrogen,

ii) $C_1$-$C_6$ loweralkyl,

iii) cycloalkyl,

iv) loweralkenyl,

v) $-(CH_2)_m$cycloalkyl,

vi) $-(CH_2)_mCN$,

vii) alkoxyalkyl,

viii) adamantyl,

ix) $-(CH_2)_m(C=O)_rNR_6R_7$ wherein $R_6$ and $R_7$, are independently selected from hydrogen, loweralkyl, cycloalkyl, loweralkenyl, $-(CH_2)_m$aryl, $-(CH_2)_m$(substituted aryl) wherein the aryl group is substituted with one, two or three substituents independently selected from loweralkyl, alkoxy, thioalkoxy, carboxy, carboalkoxy, cyano, haloalkyl, $-N_3$ $-NHP_4$ wherein $P_4$ is an N-protecting group, $-OP_5$ wherein $P_5$ is an O-protecting group, nitro, halogen, hydroxy, amino, alkylamino and dialkylamino;

x) cyclic group wherein $R_1$ and $R_2$ taken together with the adjacent nitrogen atom form a heterocyclic group represented by the formula

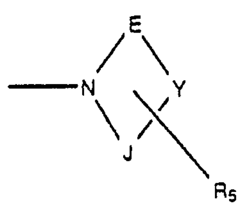

wherein E and J are independently selected from $-(CH_2)_p-$, $-(CH=CH)_r-$, $-YCH_2-$, $-CH_2Y-$, $-C(O)Y-$ and $-YC(O)-$

wherein Y is S, O, $CH_2$, or $-N(R_8)-$, wherein $R_8$ is selected from hydrogen, $-(C=O)_r(C_1$-$C_6$loweralkyl), $--(C=O)_r$-cycloalkyl, $-(C=O)_r$loweralkenyl, $-(C=O)_r(CH_2)_m$aryl, $-(C=O)_r(CH_2)_m$(substituted aryl) wherein substituted aryl is as defined above, $-(CH_2)_mSR_4$ and $-(CH_2)_mOR_4$ wherein $R_4$ is hydrogen, $-(C=O)_r$loweralkyl, $-(O=O)_r$loweralkenyl, $-(C=O)_r$cycloalkyl, $-(C=O)_r(CH_2)_m$aryl or $-(C=O)_r(CH_2)_m$(substituted aryl) wherein substituted aryl is as defined above, and wherein $R_5$ is hydrogen or $R_5$ is one, two or three of the substituents independently selected from the group $-(C_1$-$C_6$loweralkyl), loweralkenyl, $-(O)_t(CH_2)_m$carboxyl, $--(O)_t(CH_2)_m$carboalkoxy, $-(O)_t(CH_2)_m$carboaryloxy, haloalkyl, nitro, cyano, $-N_3$, $-NHP_4$, $-OP_5$, alkylaminocarbonyl, dialkylaminocarbonyl, alkoxy, aryloxy, thioalkoxy, thioaryloxy, halogen, CN, -OH, $-NH_2$, $-NR_6R_7$ wherein $R_6$ and $R_7$ are independently as defined above, $-(CH_2)_mOR_4$ wherein $R_4$ is independently as defined above, and $(CH_2)_mSR_4$ wherein $R_4$ is independently as defined above;

xi) $-(C(R_{21})(R_{18}))_m$aryl wherein $R_{21}$ and $R_{18}$ are independently selected from the group hydrogen, $-(C_1$-$C_6$loweralkyl), loweralkenyl, $-(O)_t(CH_2)_m$carboxyl, $-(O)_t(CH_2)_m$carboalkoxy, $-(O)_t(CH_2)_m$carboaryloxy, haloalkyl, nitro, alkoxy, aryloxy, thioalkoxy, thioaryloxy, halogen, CN, -OH, $-NH_2$, $-NR_6R_7$

wherein $R_6$ and $R_7$ are independently as defined above, $-(CH_2)_mOR_4$ wherein $R_4$ is independently as defined above, and $-(CH_2)_mSR_4$ wherein $R_4$ is independently as defined above,

xii) $-(C(R_{21})(R_{18}))_m$(substituted aryl) wherein $R_{21}$ and $R_{18}$ are independently defined as above, and substituted aryl is as defined above,

xiii) $-(C(R_{21})(R_{18}))_mC(O)R_3$, wherein $R_{21}$ and $R_{18}$ are independently as defined above and wherein $R_3$ is -OH, $-OR_4$ wherein $R_4$ is independently as defined above, $-NR_6R_7$ wherein $R_6$ and $R_7$ are independently as defined above, or $-NHR_4$ wherein $R_4$ is independently as defined above, with the proviso that when $R_3$ is $NR_6R_7$ then $R_{21}$ and $R_{18}$ are not both hydrogen and

xiv) $-(C(R_{21})(R_{18}))_mR_{12}$ wherein $R_{21}$ and $R_{18}$ are independently as defined above and $R_{12}$ is a heterocyclic group; with the proviso that $R_1$ and $R_2$ are not both hydrogen;

b) $R_{11}$ is
i) hydrogen,
ii) loweralkyl, or
iii) loweralkenyl;

c) $R_{20}$ is
i) hydrogen,
ii) loweralkyl, or
iii) loweralkenyl;

d) B is
i) $-(CH_2)_m-$,
ii) $-(CR_{21}=CR_{18})_q-$ wherein $R_{21}$ and $R_{18}$ are independently as defined above,
iii) $-QCH_2-$ wherein Q is O, S, or $-N(R_8)-$ wherein $R_8$ is independently as defined above,
iv) $CH_2Q-$ wherein Q is as defined above,
or
v) NH;

e) Z is
i) C=O,
ii) $S(O)_2$ ,
or
iii) C=S;

f) Ar is a heterocyclic group, aryl or substituted aryl wherein substituted aryl is as defined above;
the subscripts n, m, p, q, r and t are independently selected at each occurrence from the values n is 1 to 3; m is 0 to 4; p is 0 to 2; q is 0 or 1; r is 0 to 1; and t is 0 to 1; or a pharmaceutically acceptable salt thereof.

European Patent Application No. 0230151, published July 29, 1987, discloses the following compounds as CCK antagonists:

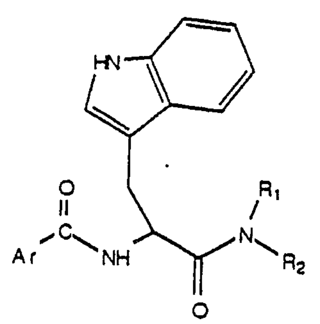

wherein Ar is phenyl or phenyl substituted with one or two substituents independently selected from hydrogen, loweralkyl, alkoxy, hydroxy substituted alkyl, alkoxy substituted alkyl, halogen, amino, hydroxy, nitro, cyano, carboxy, ethoxycarbonyl and trihalomethyl; and $R_1$ is $-(CH_2)_a$phenyl wherein a is 1 to 4; and $R_2$ is loweralkyl, cycloalkyl, $-(CH_2)_b$aryl wherein b is 0 to 4, $-(CH_2)_b$(substituted aryl) wherein b is 0 to 4 and

substituted aryl is as defined above, or $-(CH_2)_c(CO)R_3$ wherein c is 1 to 4 and $R_3$ is hydroxy, alkoxy, $-O-(CH_2)_c$aryl, $-O(CH_2)_c$(substituted aryl) wherein c is 1 to 4, or $NR_6R_7$ wherein $R_6$ and $R_7$ are independently selected from hydrogen and loweralkyl.

The "dashed" bond in the compound of Formula I indicates that the bond is a single bond or a double bond.

The term "loweralkyl" as used herein refers to straight or branched chain alkyl radicals containing from 1 to 6 carbon atoms including but not limited to methyl, ethyl, n-propyl, iso-propyl, n-butyl, iso-butyl, sec-butyl, n-pentyl, 2-methylbutyl, 2,2-dimethylpropyl, n-hexyl, 2-methylpentyl, 2,2-dimethylbutyl, and the like.

The term "cycloalkyl" as used herein refers to an alicyclic ring having 3 to 7 carbon atoms including, but not limited to, cyclopropyl, cyclobutyl, cyclopentyl, cyclohexyl and the like.

The term "loweralkenyl" as used herein refers to a lower alkyl radical which contains at least one carbon-carbon double bond.

The term "loweralkynyl" as used herein refers to a lower alkyl radical which contains at least one carbon-carbon triple bond.

The term "alkylene group" as used herein refers to a $(CH_2)_y$ radical where y is 1 to 5, such as methylene, ethylene, propylene, tetramethylene and the like.

The term "alkenylene group" as used herein refers to a $C_2$-$C_5$ chain of carbon atoms which contains at least one carbon-carbon double bond, such as vinylene, propenylene, butenylene and the like.

The term "halogen" or "halo" as used herein refers to F, Cl, Br, I.

The terms "alkoxy" and "thioalkoxy" as used herein refer to $R_9O$- and $R_9S$- respectively, wherein $R_9$ is a loweralkyl group.

The term "alkoxyalkyl" as used herein refers to an alkoxy group appended to a loweralkyl radical including, but not limited to, methoxymethyl, ethoxypropyl and the like.

The term "alkylamino" as used herein refers to $-NHR_{13}$ wherein $R_{13}$ is a loweralkyl group.

The term "dialkylamino" as used herein refers to $-NR_{14}R_{15}$ wherein $R_{14}$ and $R_{15}$ are independently selected from loweralkyl.

The term "aminoalkyl" as used herein refers to an amino group ($-NH_2$) appended to a loweralkyl radical including, but not limited to, aminomethyl, aminoethyl and the like.

The term "aminoalkenyl" as used herein refers to an amino group appended to a loweralkenyl radical, with the proviso that the amino group is not bonded directly to the double bond of the alkenyl group. Examples include, but are not limited to, 4-amino-but-2-enyl and the like.

The term "aminocarbonyl" as used herein refers to $-C(O)NH_2$.

The term "alkylaminocarbonyl" as used herein refers to $-C(O)NHR_{19}$ wherein $R_{19}$ is a loweralkyl group.

The term "dialkylaminocarbonyl" as used herein refers to $-C(O)NR_{19}R_{20}$ wherein $R_{19}$ and $R_{20}$ are independently selected from loweralkyl.

The terms "alkenyloxy" and "thioalkenyloxy" as used herein refer to $-OR_{17}$ and $-SR_{17}$ respectively, wherein $R_{17}$ is a loweralkenyl group, with the the proviso that the oxygen or sulfur atom is not bonded to the double bond of the loweralkenyl group. Examples include, but are not limited to $CH_2=CHCH_2O$-, $CH_2=CHCH_2S$- and the like.

The term "alkoxycarbonyl" as used herein refers to $-C(O)R_{16}$ wherein $R_{16}$ is an alkoxy group.

The term "carboaryloxy" as used herein refers to $-C(O)R_{17}$ wherein $R_{17}$ is an aryloxy group.

The term "haloalkyl" as used herein refers to a loweralkyl radical substituted by one or more halogens including, but not limited to, chloromethyl, 1,2-dichloroethyl, trifluoromethyl and the like.

The term "aryl" or "aryl group" as used herein refers to phenyl, naphthyl, indanyl, fluorenyl, (1,2,3,4)-tetrahydronaphthyl, indenyl or isoindenyl.

The term "substituted aryl" as used herein refers to an aryl group substituted with one, two or three substituents independently selected from the group including but not limited to loweralkyl, alkoxy, halogen, thioalkoxy, carboxy, carboalkoxy, cyano, haloalkyl, $-N_3$, $-NHP_4$ wherein $P_4$ is an N-protecting group, $-OP_5$ wherein $P_5$ is an O-protecting group, nitro, hydroxy, amino, alkylamino and dialkylamino.

The term "arylalkyl" as used herein refers to an aryl group appended to a loweralkyl radical including, but not limited to, benzyl and the like.

The term "(substituted aryl)alkyl" as used herein refers to a substituted aryl group appended to a loweralkyl radical including, but not limited to, 4-methoxybenzyl and the like.

The term "heterocyclic" or "heterocyclic group" as used herein refers to any 5-, 6- or 7-membered ring containing from one to three heteroatoms independently selected from the group consisting of nitrogen, oxygen and sulfur; wherein the 5-membered rings has 0-2 double bonds, 6 membered ring has 0-3 double bonds and the 7 membered ring has 0-3 double bonds; wherein the nitrogen and sulfur heteroatoms can be oxidized; wherein the nitrogen heteroatom can be quaternized; and including any bicyclic or tricyclic group

in which the above-mentioned heterocyclic ring is fused to one or two benzene rings or one or two 5-membered or 6-membered heterocyclic rings as defined above. Exemplary heterocycles include, but are not limited to, thienyl, furyl, pyrrolyl, imidazolyl, pyrazolyl, thiazolyl, isothiazolyl, oxazolyl, isoxazolyl, oxadiazolyl, oxatriazolyl, pyrrolidinyl, pyrrolinyl, imidazolidinyl, imidazolinyl, pyrazolidinyl, triazolyl, tetrahydrofuryl, pyranyl, pyronyl, pyridyl, pyrazinyl, pyridazinyl, piperidyl, piperazinyl, morpholinyl, thiazinyl, oxadiazinyl, azepinyl, thiapinyl, thionaphthyl, benzofuryl, isobenzofuryl, indolyl, oxyindolyl, isoindolyl, indazolyl, indolinyl, 7-azaindolyl, isoindazolyl, anthranyl, benzopyranyl, coumarinyl, isocoumarinyl, quinolyl, isoquinolyl, naphthyridinyl, cinnolinyl, quinazolinyl, pyridopyridyl, benzoxazinyl, diazepinyl, quinoxadinyl, chromenyl, chromanyl, isochromanyl, carbolinyl, xanthenyl, and acridinyl.

The heterocyclic groups can be unsubstituted or substituted with one, two or three substituents independently selected from hydroxy, oxo, amino, alkylamino, dialkylamino, alkoxy, thioalkoxy, carboxyl, alkoxycarbonyl, halo, haloalkyl, loweralkyl, cyano, aminoalkyl, aminoalkenyl, azide, aminocarbonyl, alkylaminocarbonyl, dialkylaminocarbonyl, nitro, thiocyanate, alkenyloxy, thioalkenyloxy, aryloxy, thioaryloxy, $-OP_1$ and $-NHP_2$ wherein $P_1$ is an O-protecting group and $P_2$ is an N-protecting group.

The term "aryloxy" or "thioaryloxy" as used herein refers to $R_{10}O$- or $R_{10}S$-, respectively, wherein $R_{10}$ is an aryl or substituted aryl group.

The term "N-protecting group" or "N-protected" as used herein refers to those groups intended to protect the N-terminus of an amino acid or peptide or to protect an amino group against undersirable reactions during synthetic procedures or to prevent the attack of exopeptidases on the compounds or to increase the solubility of the compounds and includes but is not limited to sulfonyl, acyl, acetyl, pivaloyl, t-butyloxycarbonyl (Boc), carbonylbenzyloxy (Cbz), benzoyl or an L- or D-aminoacyl residue, which may itself be N-protected similarly.

The term "O-protecting group" as used herein refers to a substituent which protects hydroxyl groups against undesirable reactions during synthetic procedures and includes but is not limited to substituted methyl ethers, for example methoxymethyl, benzylozymethyl, 2-methoxyethoxymethyl, 2-(trimethylsilyl)-ethoxymethyl, benzyl and triphenylmethyl; tetrahydropyranyl ethers; substituted ethyl ethers, for example, 2,2,2-trichloroethyl and t-butyl; and silyl ethers, for example, trimethylsilyl, t-butyldimethylsilyl and t-butyldiphenylsilyl.

The term "Trp" as used herein refers to tryptophan.

Exemplary compounds of the invention include, but are not limited to:

N-(2'-Indolylcarbonyl)-R-Tryptophan-di-n-pentylamide,
N-(3'-Quinolylcarbonyl)-R-Tryptophan-benzylamide,
N-(2'-Indolylcarbonyl)-Tryptophan-(1',R-phenylethyl)amide,
N-(3'-Quinolylcarbonyl)-R-Tryptophan-di-n-pentylamide,
Methyl N-(2'-Indolylcarbonyl)-R-Tryptophyl-S-Phenylglycinate,
Ethyl N-(2'-Indolylcarbonyl)-R-Tryptophyl-S-Phenylglycinate,
Ethyl N-(3'-Quinolylcarbonyl)-R-Tryptophyl-(N-benzyl)Glycinate,
Methyl N-(3 -Quinolylcarbonyl)-Tryptophyl-S-Phenylglycinate,
Ethyl N-(3'-Quinolylcarbonyl)-Tryptophyl-S-Phenylglycinate,
N-(3'-Quinolylcarbonyl)-R-Tryptophan-(1',R-phenylethyl)amide,
Methyl N-(3'-Quinolylcarbonyl)-R-Tryptophyl-S-(p-methoxyphenyl)glycinate,
N-(3'-Quinolylcarbonyl)-R-5-Benzyloxytryptophan-di-n-pentylamide,
N-(3'-Quinolylcarbonyl)-R-5-Fluorotryptophan-di-n-pentylamide,
N-(2'-Indolylcarbonyl)-R-5-Fluorotryptophan-di-n-pentylamide,
N-(3'-Quinolylcarbonyl)-R-(beta-Oxindolyl)Alanine-di-n-pentylamide,
N(alpha)-(3'-Quinolylcarbonyl)-R-5-Hydroxytryptophan-di-n-pentylamide,
N(alpha)-(3'-Quinolylcarbonyl)-R-Tryptophan-bis-(ethoxyethyl)amide,
N(alpha)-(3'-Quinolylcarbonyl)-R-Tryptophan-(N-3-Methoxy-n-propyl, N-n-pentyl)amide,
N-(4',8'-Dihydroxy-2'-quinolylcarbonyl)-R-Tryptophan-di-n-pentylamide,
N-(6'-Quinolylcarbonyl)-R-Tryptophan-di-n-pentylamide,
N-(4-Hydroxy-2'-quinolylcarbonyl)-R-Tryptophan-di-n-pentylamide,
N-(5'-Fluoro 2'-indolylcarbonyl)-R-Tryptophan-di-n-pentylamide,
N-(3'-Quinolylcarbonyl)-R-Tryptophan-di-n-pentylamide mesylate salt
N-4',8'-Dihydroxy-2'-quinolylcarbonyl)-R-Tryptophan-(1',R-phenylethyl)amide,
Methyl N-(4',8'-Dihydroxy-2'-quinolylcarbonyl)-R-Tryptophyl-S-(p-Methoxyphenyl)glycinate,
N-(4',8'-Dihydroxy-2'-quinolylcarbonyl)-R-Benzyloxytryptophan-di-n-pentylamide,
N-(4',8'-Dihydroxy-2'-quinolylcarbonyl)-R-5-Fluorotryptophan-di-n-pentylamide,
N-(4',8'-Dihydroxy-2'-quinolylcarbonyl)-R-(beta-Oxindolyl)Alanine-di-n-pentylamide,

N(alpha)-(4',8'-Dihydroxy-2'-quinolylcarbonyl)-R-5-Hydroxytryptophan-di-n-pentylamide,
N(alpha)-(4',8'-Dihydroxy-2'-quinolylcarbonyl)-R-Tryptophan-bis-(ethoxyethyl)amide,
Methyl N-(4',8'-Dihydroxy-2'-quinolylcarbonyl)-R-Tryptophyl-S-Phenylglycinate,
Ethyl N-(4',8'-Dihydroxy-2'-quinolylcarbonyl)-R-Tryptophyl-S-Phenylglycinate,
Ethyl N-(4',8'-Dihydroxy-2'-quinolylcarbonyl)-R-Tryptophyl-(N-benzyl)Glycinate, and
N(alpha)-(4',8'-Dihydroxy-2'-quinolylcarbonyl)-R-Tryptophan-(N-3-methoxy-n-propyl, N-n-pentyl)amide

Preferred compounds of the invention include
N-(2'-indolylcarbonyl)-R-Tryptophan-di-n-pentylamide,
N-(3'-Quinolylcarbonyl)-R-Tryptophan-di-n-pentylamide,
N-(3'-Quinolylcarbonyl)-R-(beta-Oxindolyl)Alanine-di-n-pentylamide
N(alpha)-(3'-Quinolylcarbonyl)-R-5-Hydroxytryptophan-di-n-pentylamide,
N-(4',8'-Dihydroxy-2'-quinolylcarbonyl)-R-Tryptophan-di-n-pentylamide, and
N(alpha)-(4',8'-Dihydroxy-2'-quinolylcarbonyl)-R-5-Hydroxytryptophan-di-n-pentylamide.

The compounds of the invention may be made as shown in Scheme I. Compounds with DL (R,S), L, or D configuration may be used in the synthetic schemes.

N-protected derivatives of tryptophan 1 are coupled with primary and secondary amines of the type $HNR_1R_2$ (preferably dialkyl, diaryl, or arylalkyl amines or amino acid esters) using bis(2-oxo-3-oxazolidinyl)-phosphinic chloride (BOPCl) or other standard coupling techniques (i.e., isobutylchloroformate (IBCF), phosphorus pentachloride ($PCl_5$), dicyclohexylcarbodiimide (DCC), or 1-ethyl-3-(3-dimethylaminopropyl)-carbodiimide (EDCI) with hydroxybenzotriazole (HOBt)). The product amide 2 is deprotected with hydrochloric acid in dioxane to provide the hydrochloride salt 3 in the case where P = Boc (t-butoxycarbonyl) and with hydrogenolysis over palladium on carbon or other solid supports to provide amine 4 in the case where P = Cbz (carbobenzyloxy). Both the salt 3 and the free base 4 are coupled to an appropriate arylcarboxylic acid or heteroarylcarboxylic acid (preferably quinoline-3-carboxylic acid or indole-2-carboxylic acid) using standard techniques to provide product 5. Alternatively, 5 can be obtained from the coupling of 3 or 4 with the appropriate acid chloride or activated ester form of the aryl- or heteroarylcarboxylic acids. Further substitution of the nitrogem atom can be achieved via prior alkylation. In these cases the amine 4 or its hydrochloride 3 is either directly alkylated with $R_{11}X$ (where X is chloride, bromide, iodide), an activated form of the alcohol $R_{11}OH$ or reductively alkylated with an appropriate aldehyde in the presence of a reducing agent such as sodium cyanoborohydride or sodium borohydride, to provide the same product 6. Product 6 can then be coupled in a fashion analogous to 3 and 4 to provide the key compound 5. Compound 5 can be treated directly with aryl- or alkylsulfenyl chlorides ($R_{10}SCl$ or $R_9SCl$) in a variety of solvents to provide the thioaryl or thioalkyl substituted tryptophans 7.

Alternatively, the tricyclic intermediate 17 can be produced from 5 via the action of t-butylhypochlorite and an amine base and the tricyclic 17 reacted with thiols $R_{10}SH$ or $R_9SH$ to provide the substituted analogs 7.

The product 5 can also be treated with base (sodium hydride, sodium hydroxide under phase transfer conditions, or lithium hexamethyldisilazide in dimethylformamide (DMF)) followed by an alkylating or acylating agent represented by $R_{30}X$, to provide the derivatized products 8. The amine hydrochloride 3 or it substituted analog 6 can likewise be coupled with aryl-or heteroarylsulfonylchlorides ($ArSO_2Cl$) to provide the products 9. Alternatively, one familiar with the art could use the sulfonic acids and standard coupling methodology to provide 9. In addition, the amines 3 and 6 may also be coupled in standard fashion to the beta aryl- or beta heteroaryl substituted acrylic acids ( or their corresponding acid chlorides) to yield the unsaturated derivatives 10. Compound 11 is obtained through direct oxidation of the parent 5 with hydrochloric acid in dimethylsulfoxide (DMSO). Alternatively, controlled hydrolysis of intermediate 17 yields compound 11. Indoline derivatives of the type 12 are obtained by the catalytic hydrogenation of 5 followed by an acylation or an alkylation. Compounds of the type 1 are obtained by an alkylation sequence starting from compound 13. Compound 13 is first reacted with benzaldehyde under dehydrating conditions to provide 14 (Q=H) or with benzophenone imine to provide 14 (Q=phenyl). Compound 14 is alkylated directly with $R_{11}X$ utilizing phase transfer conditions (methylene chloride, or other suitable organic solvent, and 10-50% sodium or potassium hydroxide solutions or potassium carbonate) with a tetraalkylammonium salt present, preferably tetrabutylammonium hydrogensulfate ($NBu_4HSO_4$). The product 15 is deprotected with aqueous acid and subsequently the free amine or its salt is protected with CbzCl or di-t-butyldicarbonate ($Boc_2O$) to yield the ester 16. Ester 16 is saponified in a standard fashion (NaOH) to provide 1 ($R_{11}$ not H) for subsequent reaction including conversion to products of types 5, 7, 8, 9 and 10.

Scheme I

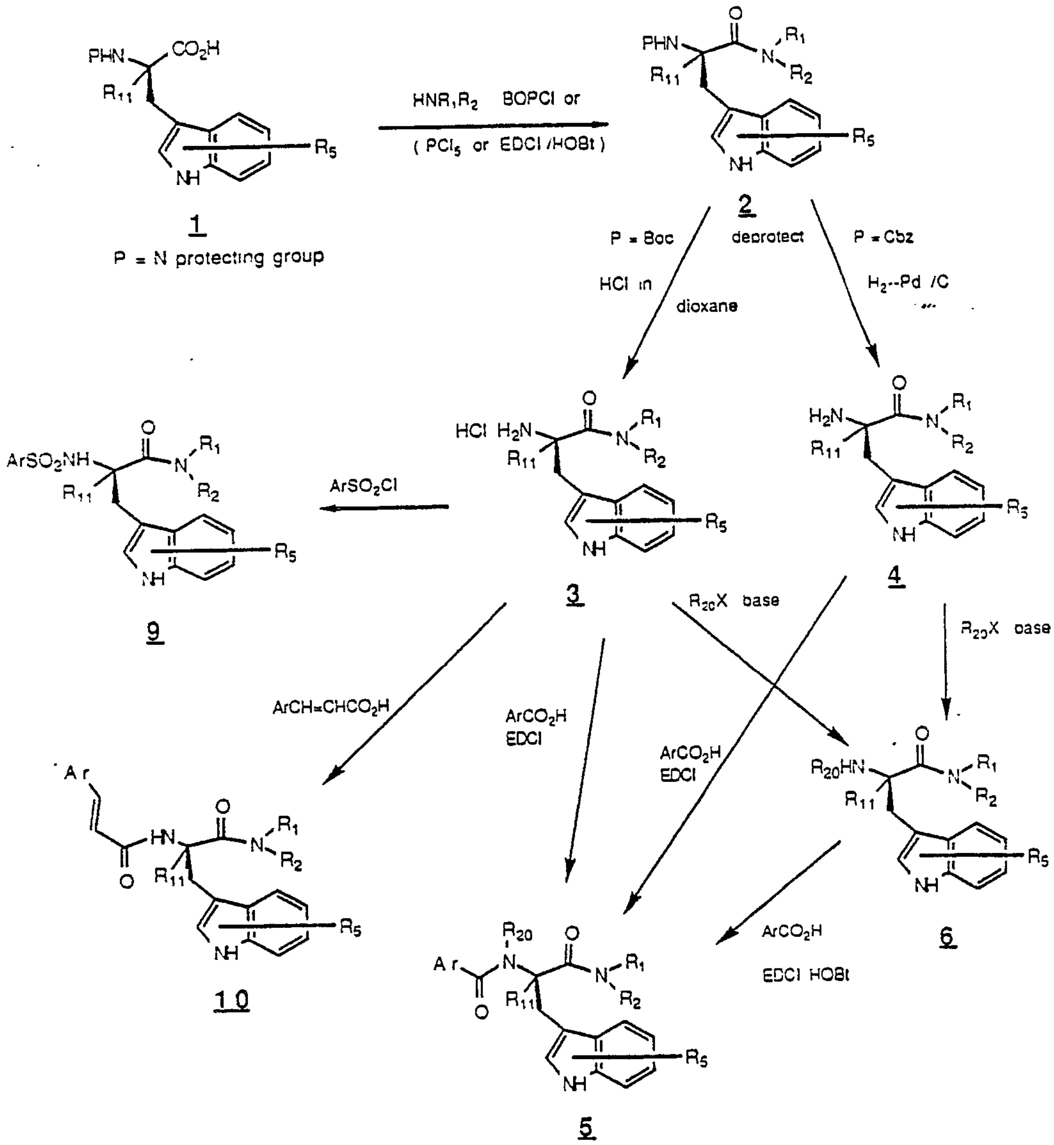
P = N protecting group
HNR1R2 BOPCl or
( PCl5 or EDCI /HOBt )
P = Boc
HCl in
dioxane
deprotect
P = Cbz
H2--Pd /C
ArSO2Cl
R20X base
R20X base
ArCH=CHCO2H
ArCO2H
EDCI
ArCO2H
EDCI
ArCO2H
EDCI HOBt

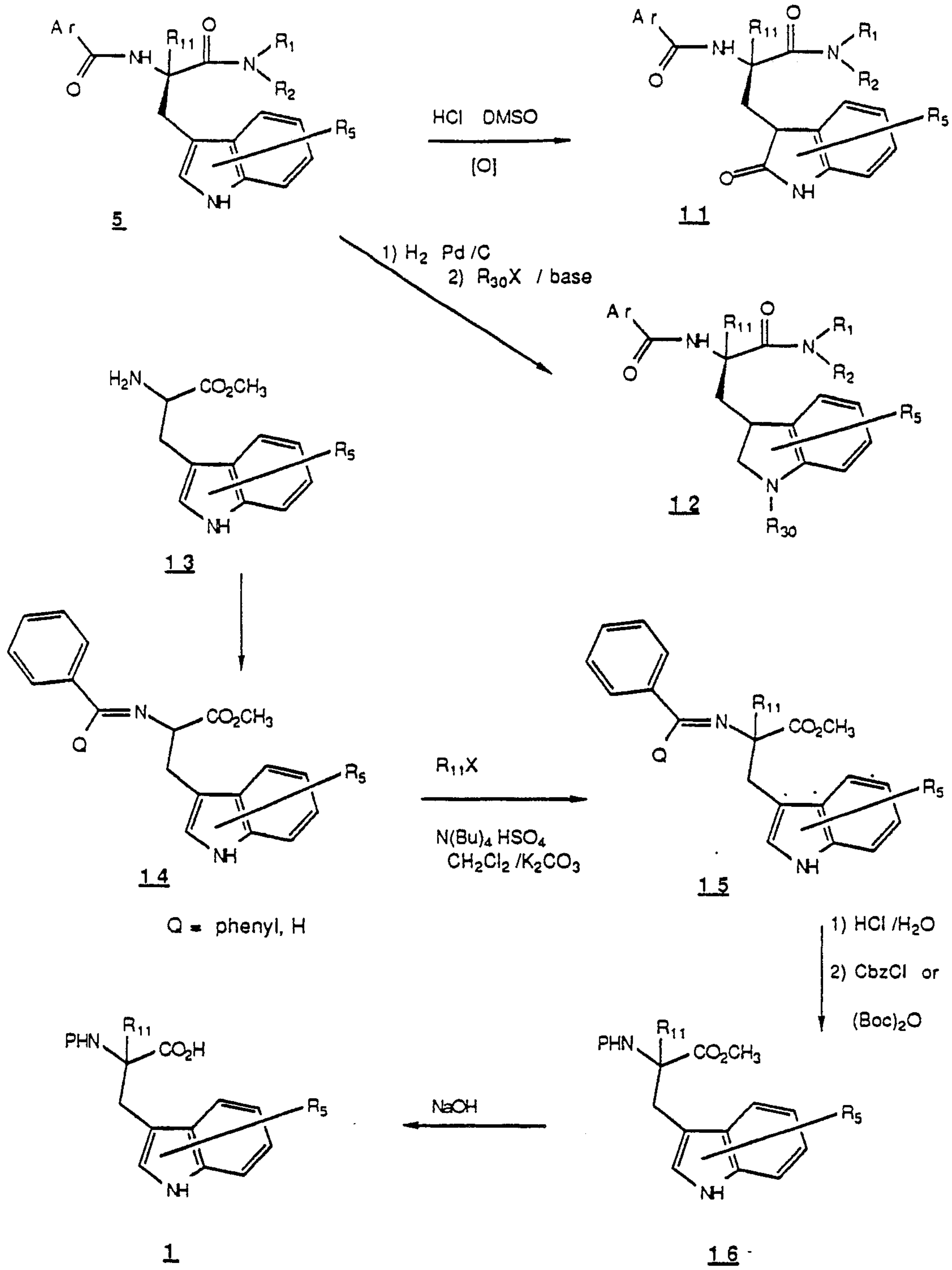


5

11

12

13

14

Q = phenyl, H

15

16

1

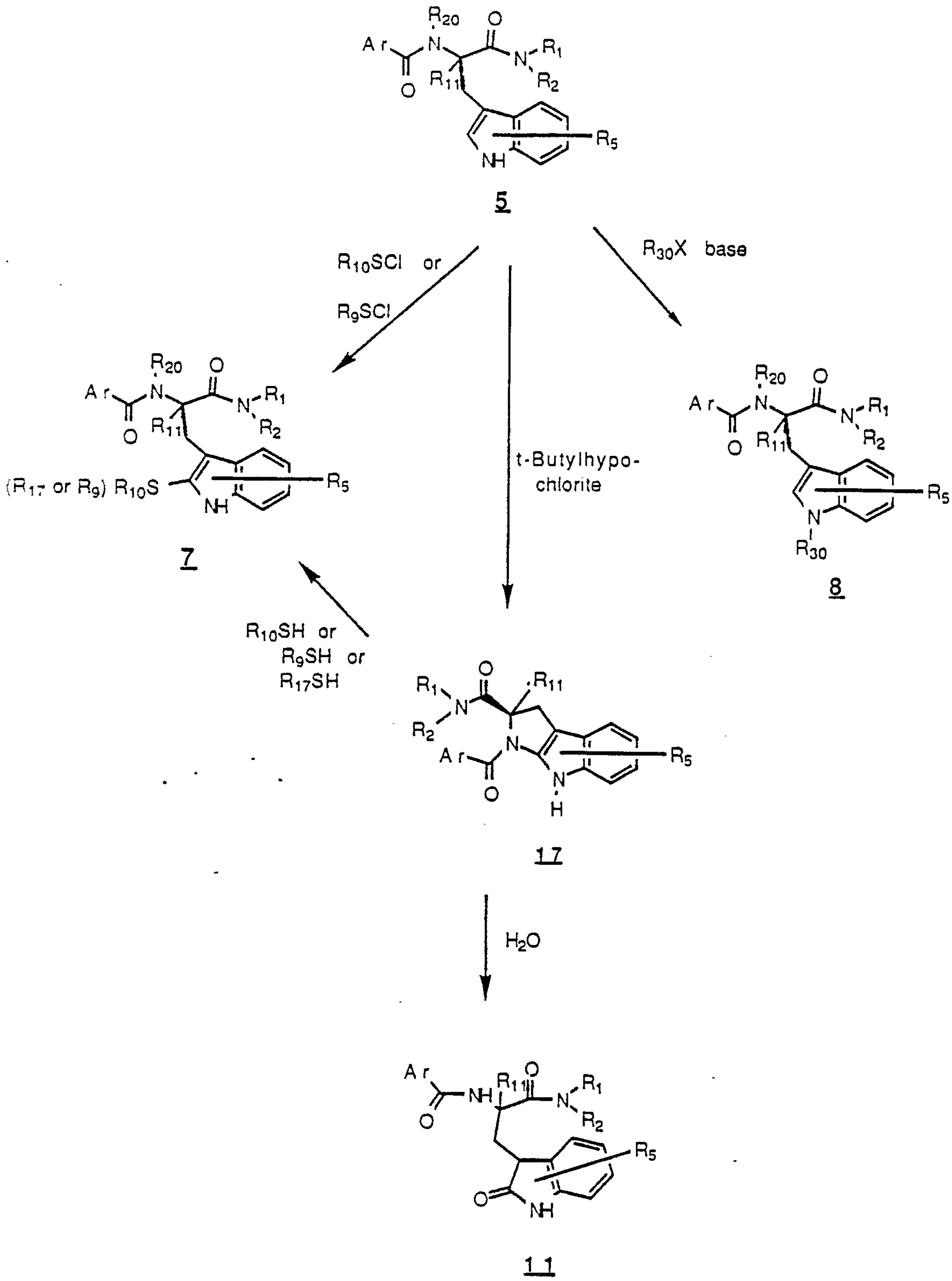


5

7

8

17

11

Intermediates for the preparation of compounds of the formula I include compounds of the formula:

a)

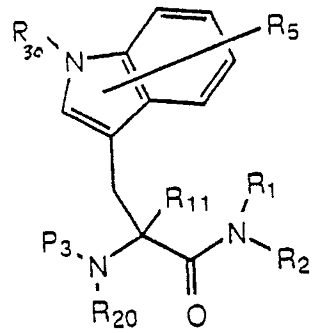

b)

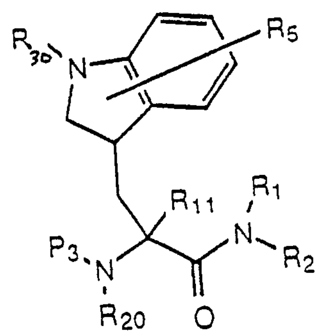

c)

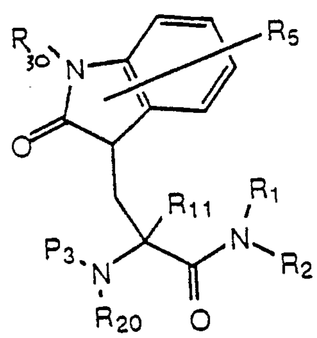

or

d)

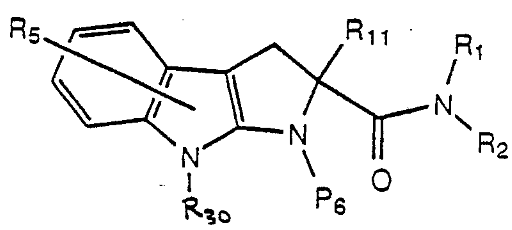

wherein $R_1$ and $R_2$ are independently selected from

i) hydrogen,

ii) $C_1$-$C_6$ loweralkyl,

iii) cycloalkyl,

iv) loweralkenyl,

v) $-(CH_2)_m$cycloalkyl,

vi) $-(CH_2)_mCN$,

vii) alkoxyalkyl,

viii) adamantyl,

ix) $-(CH_2)_m(C=O)_rNR_6R_7$ wherein $R_6$ and $R_7$ are independently selected from hydrogen, loweralkyl, cycloalkyl, loweralkenyl, $-(CH_2)_m$aryl, $-(CH_2)_m$(substituted aryl)

wherein the aryl group is substituted with one, two or three substituents independently selected from

loweralkyl, alkoxy, thioalkoxy, carboxy, carboalkoxy, cyano, haloalkyl, $-N_3$, $-NHP_4$ wherein $P_4$ is an N-protecting group, $-OP_5$ wherein $P_5$ is an O-protecting group, nitro, trihalomethyl, halogen, hydroxy, amino, alkylamino and dialkylamino;

x) cyclic groups wherein $R_1$ and $R_2$ taken together with the adjacent nitrogen atom form a heterocyclic group represented by the formula

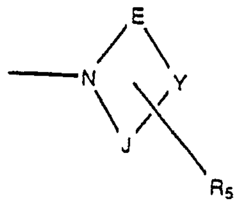

wherein E and J are independently selected from $-(CH_2)_p-$, $-(CH=CH)_r-$, $-YCH_2-$, $-CH_2Y-$, $-C(O)Y-$ and $-YC(O)-$wherein Y is S, O, $CH_2$ or $-N(R_8)-$, wherein $R_8$ is selected from hydrogen, $-(C=O)_r(C_1-C_6\text{loweralkyl})$, $--(C=O)_r$-cycloalkyl, $-(C=O)_r$loweralkenyl, $-(C=O)_r(CH_2)_m$aryl, $-(C=O)_r(CH_2)_m$(substituted aryl) wherein substituted aryl is as defined above, $-(CH_2)_mSR_4$ and $-(CH_2)_mOR_4$ wherein $R_4$ is hydrogen, $-(C=O)_r$loweralkyl, $-(C=O)_r$loweralkenyl, $-(C=O)_r$cycloalkyl, $-(C=O)_r(CH_2)_m$aryl or $-(C=O)_r(CH_2)_m$(substituted aryl) wherein substituted aryl is as defined above, and wherein $R_5$ is hydrogen or $R_5$ is one, two or three of the substituents independently selected from the group $-(C_1-C_6\text{loweralkyl})$, loweralkenyl, $-(O)_t(CH_2)_m$carboxyl, $--(O)_t(CH_2)_m$carboalkoxy, $-(O)_t(CH_2)_m$carboaryloxy, haloalkyl, nitro, cyano, $-N_3$, $-NHP_4$ , $-OP_5$ alkylaminocarbonyl, dialkylaminocarbonyl, alkoxy, aryloxy, thioalkoxy, thioaryloxy, halogen, CN, -OH, $-NH_2$, $-NR_6R_7$ wherein $R_6$ and $R_7$ are independently as defined above, $-(CH_2)_mOR_4$ wherein $R_4$ is independently as defined above, and $-(CH_2)_mSR_4$ wherein $R_4$ is independently as defined above;

xi) $-(C(R_{21})(R_{18}))_m$aryl wherein $R_{21}$ and $R_{18}$ are independently selected from the group hydrogen, $--(C_1-C_6\text{loweralkyl})$, loweralkenyl, $-(O)_t(CH_2)_m$carboxyl, $-(O)_t(CH_2)_m$carboalkoxy, $-(O)_t(CH_2)_m$carboaryloxy, haloalkyl, nitro, alkoxy, aryloxy, thioalkoxy, thioaryloxy, halogen, CN, -OH, $-NH_2$ $-NR_6R_7$ wherein $R_6$ and $R_7$ are independently as defined above, $-(CH_2)_mOR_4$ wherein $R_4$ is independently as defined above, and $--(CH_2)_mSR_4$ wherein $R_4$ is independently as defined above,

xii) $-(C(R_{21})(R_{18}))_m$(substituted aryl) wherein $R_{21}$ and $R_{18}$ are independently defined as above, and substituted aryl is as defined above,

xiii) $-(C(R_{21})(R_{18}))_mC(O)R_3$

wherein $R_{21}$ and $R_{18}$ are independently as defined above and wherein $R_3$ is -OH, $-OR_4$ wherein $R_4$ is independently as defined above, $-NR_6R_7$ wherein $R_6$ and $R_7$ are independently as defined above, or $-NHR_4$ wherein $R_4$ is independently as defined above, with the proviso that when $R_3$ is $NR_6R_7$ then $R_{21}$ and $R_{18}$ are not both hydrogen and

xiv) $-(C(R_{21})(R_{18}))_mR_{12}$ wherein

$R_{21}$ and $R_{18}$ are independently as defined above and

$R_{12}$ is a heterocyclic group;

with the proviso that $R_1$ and $R_2$ are not both hydrogen;

$R_{11}$ is

i) hydrogen,

ii) loweralkyl, or

iii) loweralkenyl;

$R_{20}$ is

i) hydrogen,

ii) loweralkyl, or

iii) loweralkenyl;

the subscripts are independently selected at each occurrence from the values n is 1 to 3; m is 0 to 4; p is 0 to 2; q is 0 or 1; r is 0 to 1; and t is 0 to 1; and

$P_3$ and $P_6$ are independently selected from hydrogen and an N-protecting group.

Other intermediates for the preparation of compounds of the formula I incude compounds of the formula:

Ar-B-Z-Z′

wherein B is

i) $-(CH_2)_m-$,
ii) $-(CR_{21}=CR_{18})_q-$ wherein $R_{21}$ and $R_{18}$ are independently as defined above,
iii) $-QCH_2-$ wherein Q is O, S, or $-N(R_8)-$ wherein $R_8$ is independently as defined above, or
iv) $-CH_2Q-$ wherein Q is as defined above,

Z is
i) C=O,
ii) $S(O)_2$ , or
iii) C=S;

$Z'$ is an activating group; or
B-Z-$Z'$ taken together represent -N=C=O, -N=C=S, $-CH_2N=C=O$ or $-CH_2-N=C=S$;
Ar is a heterocyclic group, aryl or substituted aryl wherein substituted aryl is as defined above; m is 0 to 4; and q is 0 or 1.

Activating groups are those functional groups which activate a carboxylic acid or sulfonic acid group toward coupling with an amine to form an amide or sulfonamide bond. Activating groups $Z'$ include, but are not limited to -OH, -SH, alkoxy, thioalkoxy, halogen, formic and acetic acid derived anhydrides, anhydrides derived from alkoxycarbonyl halides such as isobutyloxycarbonylchloride and the like, N-hydroxysuccinimide derived esters, N-hydroxyphthalimide derived esters, N-hydroxybenzotriazole derived esters, N-hydroxy-5-norbornene-2,3-dicarboxamide derived esters, 4-nitrophenol derived esters, 2,4,5-trichlorophenol derived esters and the like.

The following examples will serve to further illustrate preparation of the novel compounds of the invention.

## Example 1

### N-(t-Butyloxycarbonyl)-R-Tryptophan-di-n-pentylamide

N-t-Butyloxycarbonyl-R-Tryptophan (10 g, 33 mmol) was stirred at 0°C in 160 mL of methylene chloride ($CH_2Cl_2$) with bis(2-oxo-3-oxazolidnyl)phosphinic chloride (BOPCl, 8.6 g, 34 mmol), 4.5mL (32 mmol) of triethylamine (TEA). To this reaction mixture was added di-n-pentylamine (18 mL, 89 mmol). The mixture was stirred overnight and allowed to warm to room temperature. An additional equivalent of BOPCl was added after 18 hrs and the reaction stirred an additional day at ambient temperature. The solvents were evaporated in vacuo and the residue taken up in ethylacetate (EtOAc) and washed with water, 1 N hydrochloric acid (HCl) solution, saturated sodium bicarbonate solution ($NaHCO_3$), water and then the organic solution was dried over magnesium sulfate ($MgSO_4$). After filtration and concentration of the filtrate in vacuo, the residue was purified by chromatography using ethylacetate-hexane as the solvent system in the ratio (1:4). The product was isolated as an oil in 57% yield (8.2 g). MS(Cl) m/e 444$(m+H)^+$, 326. $^1H$ NMR($CDCl_3$,300MHz) $\delta$ 0.78(t,J=7Hz,3H), 0.88(t,J=6Hz,3H), 0.9-1.2(m,8H), 1.25(m,4H), 1.45(s,9H), 2.7-3.08(m,3H), 3.1(d,J=9Hz,2H), 3.33(m,1H), 4.9(q,J=12Hz,1H), 5.4(d,J=9Hz,1H), 7.05(s,1H), 7.15(m,2H), 7.34(d,J=9Hz,1H), 7.7(d,J=9Hz,1H), 8.1(s,1H).

## Example 2

### R-Tryptophan-di-n-pentylamide hydrochloride

The product of example 1 (2.0 g, 4.4 mmol) was dissolved in 4 N HCl in dioxane (12 mL) and stirred under inert atmosphere ($N_2$) for an hour. When the reaction was complete by tlc the solvents were evaporated in vacuo and hexane and diethylether added. The residue was triturated with these two solvents and the solvents again removed in vacuo. This procedure was repeated several times until the product was obtained as a glassy solid in quantitative yield.

Example 3

N-(2′-Indolylcarbonyl)-R-Tryptophan-di-n-pentylamide

The hydrochloride of example 2 (135 mg, 0.36 mmol), 1-ethyl-3-(3-dimethylaminopropyl)carbodiimide (EDCI, 100 mg), HOBt (48 mg) and indole-2-carboxylic acid (110mg) were stirred at 0°C under nitrogen in 5 mL of anhydrous dimethylfomamide (DMF). To this mixture was added 250 µL of TEA and the mixture was stirred overnight with warming to ambient temperature. The reaction mixture was poured into ethylacetate and water and the organic extract was washed successively with water, 10% citric acid solution, and saturated aqueous sodium bicarbonate. The solution was dried over magnesium sulfate, filtered and concentrated. The residue was purified by chromatography using ethylacetate and hexane as the elutant mixture to provide 130 mg of product (75% yield) after removal of the volatiles. mp 75-8°C. $[\alpha]_D$ = -16.2° (c=0.105, MeOH). MS(CI) m/e 487(m+H)$^+$, 358, 329, 217. $^1$H NMR($CDCl_3$,300MHz) δ 9.2-(bs,1H), 8.0(bs,1H), 7.77(d,J=7.5Hz,1H), 7.66(d,J=7.5Hz,1H), 7.13-7.44(m,7H), 7.05(d,J=2Hz,1H), 6.93-(m,1H), 5.43(apparent q,J=7.5Hz,1H), 3.35-3.45(m,1H), 3.32(d,J=7Hz,2H), 2.95-3.08(m,2H), 2.75-2.85-(m,1H), 0.95-1.45(m,12H), 0.88(t,J=7Hz,3H), 0.78(t,J=7Hz,3H). C,H,N analysis calculated for $C_{30}H_{38}N_4O_2$, 0.5 $H_2O$: C 72.68, H 7.94, N 11.31; found: C 72.62, H 7.82, N 11.21.

Example 4

N-(p-Chlorobenzoyl)-R-Tryptophan-di-n-pentylamide

To a mixture of the hydrochloride of example 2 (41 mg) and triethylamine (30 µL) stirred in 3 mL of methylene chloride at 0°C was added 20 µL of p-chlorobenzoylchloride. When tlc analysis indicated the consumption of starting material, the mixture was taken up in ethylacetate and washed three times with portions of water. The organic extract was dried over $MgSO_4$, then filtered. Concentration of the filtrate and chromatography of the residue using ethylacetate and hexane as the elutant mixture provided 30 mg of product. mp 63-5°C. MS(CI) m/e 482(m+H)$^+$, 448, 262, 173, 158. $^1$H NMR($CDCl_3$,300MHz) δ 8.02(bs,1H), 7.77(bd,J=7.5Hz,1H), 7.72(d,J=7Hz,1H), 7.38(d,J=7Hz,1H), 7.34(bd,J=7.5Hz,1H), 7.17(dt,J=1,7Hz,1H), 7.10-7.15(m,4H), 7.05(d,J=2Hz,1H), 5.41(m,1H), 3.35-3.45(m,1H), 3.28(m,2H), 2.93-3.05(m,2H), 2.80(m,1H), 0.95-1.45(m,12H), 0.88(t,J=7Hz,3H), 0.79(t,J=7Hz,3H). C,H,N analysis calculated for $C_{28}H_{36}ClN_3O_2$ C 69.74, H 7.53, N 8.71; found: C 69.28, H 7.57, N 8.60.

Example 5

N-(t-Butyloxycarbonyl)-R-Tryptophan-benzylamide

To a solution of N-t-Butyloxycarbonyl-R-Tryptophan (365 mg), HOBt (155 mg), and EDCI (269 mg) stirred at 0°C in $CH_2Cl_2$ (10 mL) was added benzylamine (140 µL) and TEA (140 µL). The reaction mixture was allowed to warm to ambient temperature overnight. The mixture was treated in a fashion similar to that in example 1 and provided 290 mg (61% yield) of product after chromatography. mp= 142.5-143.5°C. $[\alpha]_D$= +6.0° (c=0.25, 1:1 DMF-MeOH). MS(CI) m/e 394(m+H)$^+$, 338, 294, 276. $^1$H NMR($CDCl_3$,300MHz) δ 8.03(bs,1H), 7.68(d,J=7.5Hz,1H), 7.37(dd,J=0.5,8.5Hz,1H), 7.1-7.25(m,5H), 6.97(bm,3H), 5.96(bs,1H), 5.16-(bs,1H), 4.47(m,1H), 4.25-4.35(m,2H), 3.35(dd,J=5.5,14.5Hz,1H), 3.16(dd,J=7.5,14.5Hz,1H), 1.41(bs,9H). C,H,N analysis calculated for $C_{23}H_{27}N_3O_3$: C 70.19, H 6.92, N 10.68; found: C 69.97, H 7.04, N 10.60.

## Example 6

### R-Tryptophan-benzylamide hydrochloride

The product of example 5 (290 mg) was stirred under inert atmosphere at 0 °C in 4 mL of a 4.5 M solution of hydrogen chloride in dioxane. After completion of reaction by tlc analysis the volatiles were removed in vacuo and the product was triturated with anhydrous diethylether and hexane. Product was collected by filtration providing 232 mg (95% yield). mp = 189-91 °C. MS(Cl) m/e 294$(m+H)^+$. $^1H$ NMR-($DMSO_{d6}$,300MHz) $\delta$ 11.07(s,1H), 9.01(bt,J=6Hz,1H), 8.26(bs,2H), 7.67(d,J=7.5Hz,1H), 7.38(d,J=7.5Hz,1H), 7.2-7.3(m,4H), 7.08-7.15(m,3H), 7.01(t,J=7Hz,1H), 4.28(m,2H), 4.03(bt,J=7.5Hz,1H), 3.26(dd,J=7,14Hz,1H), 3.15(dd,J=7,14Hz,1H).

## Example 7

### N-(3′-Quinolylcarbonyl)-R-Tryptophan-benzylamide

The hydrochloride salt of example 6 (70 mg), quinoline-3-carboxylic acid (63 mg), EDCI (110 mg), and hydroxybenzotriazole (HOBt, 110 mg) were stirred in 3 mL of anhydrous DMF at 0 °C under nitrogen. To this mixture was added TEA (110 µL) and the mixture was stirred overnight with warming to ambient temperature. The reaction mixture was taken up in ethylacetate and washed several times with water. The organic extract was dried over $MgSO_4$, filtered, and concentrated in vacuo. Chromatography of the residue using ethylacetate and hexane as the elutant mixture provided 67 mg (70% yield) of product. mp = 211-12 °C. $[\alpha]_D$ = +45.5° (c=0.09, 1:1 DMF-MeOH). MS(Cl) m/e 449$(m+H)^+$, 342, 314. $^1H$ NMR-($DMSO_{d6}$,300MHz) $\delta$ 10.82(bs,1H), 9.23(d,J=2Hz,1H), 9.0(bd,J=8.5Hz,1H), 8.81(d,J=2Hz,1H), 8.73-(bt,J=7Hz,1H), 8.09(s,1H), 8.06(s,1H), 7.87(dt,J=1,7Hz,1H), 7.72(m,2H), 7.2-7.35(m,7H), 7.06(bt,J=7Hz,1H), 6.99(bt,J=7Hz,1H), 4.85(m,1H), 4.34(d,J=7Hz,2H). C,H,N analysis calculated for $C_{28}H_{24}N_4O_2$: C 74.97, H 5.40, N 12.50; found: C 74.94, H 5.50, N 12.50.

## Example 8

### N-(2′-Indolylcarbonyl)-R-Tryptophan-benzylamide

The reaction sequence was performed similarly to that in example 7 utilizing 90 mg of the hydrochloride salt of example 6, indole-2-carboxylic acid (80 mg), EDCI(120 mg), HOBt (55 mg), and TEA (125 µL). Product was isolated in an identical manner. mp = 207-8 °C. $[\alpha]_D$ = +55.7° (c=0.07, 1:1 DMF-MeOH). MS-(Cl) m/e 437$(m+H)^+$, 330, 302, 276. $^1H$ NMR($DMSO_{d6}$,300MHz) $\delta$ 11.52(bs,1H), 10.8(bs,1H), 8.71-(t,J=7Hz,1H), 8.58(d,J=9Hz,1H), 7.72(d,J=7.5Hz,1H), 7.62(d,J=7.5Hz,1H), 7.40(d,J=7.5Hz,1H), 7.15-7.32-(m,9H), 6.95-7.08(m,3H), 4.80(m,1H), 4.32(d,J=6Hz,2H), 3.15-3.25(m,2H). C,H,N analysis calculated for $C_{27}H_{24}N_4O_2$: C 74.28, H 5.55, N 12.84; found: C 73.95, H 5.72, N 12.47.

## Example 9

### N-(2′-Pyrrolylcarbonyl)-R-Tryptophan-di-n-pentylamide

The hydrochloride salt of example 2 (0.38 g, 1.0 mmol) was stirred in 5 mL of methylene chloride with N-methylmorpholine (NMM, 0.22 mL, 2 mmol) under nitrogen at 0 °C. EDCI (0.2 g, 1.0 mmol) and HOBt (0.27 g, 2 mmol) were added followed by the addition of pyrrole-2-carboxylic acid (0.112 g, 1.0 mmol). The reaction mixture was allowed to stir overnight with warming to ambient temperature. The solvents were evaporated in vacuo and the residue taken up in ethylacetate and washed successively with water, saturated $NaHCO_3$, a saturated solution of citric acid, water, and brine. The organic solution was dried over $MgSO_4$ and then filtered. Solvents were evaporated in vacuo and the crude product subjected to flash chromatography using ethylacetate and hexane as the elutant mixture. The product was crystallized from ethylacetate and hexane to provide 0.36 g (81%). mp= 108-110 °C. $[\alpha]_D$= -2.4° (c=1.0, MeOH). MS(CI) m/e 437(m+H)$^+$. $^1$H NMR($CDCl_3$,300MHz) $\delta$ 0.75(t,J=7Hz,3H), 0.88(t,J=7Hz,3H), 0.9-1.22(m,6H), 1.25-1.45-(m,6H), 2.7-2.85(m,1H), 2.9-3.05(m,2H), 3.28(d,J=6Hz,2H), 3.3-3.4(m,1H), 5.4(apparent q,J=9Hz,1H), 6.22-(m,1H), 6.65(m,1H), 6.(m,2H), 7.03(d,J=3Hz,1H), 7.1-7.2(m,2H), 7.35(d,J=9Hz,1H), 7.8(d,J=9Hz,1H), 8.03-(s,1H), 9.6(bs,1H). C,H,N analysis calculated for $C_{26}H_{36}N_4O_2$, 0.25 $H_2O$: C 70.79, H 8.34, N 12.70; found: C 70.51, H 8.26, N 12.71.

## Example 10

### N-(1′-Isoquinolylcarbonyl)-R-Tryptophan-di-n-pentylamide

The hydrochloride salt of example 2 (0.38 g, 1.0 mmol) was stirred in 10 mL of methylene chloride with N-methylmorpholine (NMM, 0.2 mL, 2.0 mmol) under $N_2$ at 0 °C. EDCI (0.2 g, 1.1 mmol) and HOBt (0.27 g, 2.0 mmol) was added to the mixture, followed by the addition of isoquinoline-1-carboxylic acid (0.17 g, 1.0 mmol). The reaction mixture was treated as in example 9 and a crystalline product resulted in 55% yield (0.28 g). mp= 87.5-89 °C. $[\alpha]_D$= +16.7° (c=0.5, MeOH). MS(CI) m/e 499(m+H)$^+$, 342. $^1$H NMR-($CDCl_3$,300MHz) $\delta$ 0.78(t,J=7Hz,3H), 0.85(t,J=7Hz,3H), 0.95-1.2(m,8H), 1.25-1.45(m,4H), 2.85-3.1(m,3H), 3.3-3.45(m,3H), 5.5(apparent q,J=7Hz,1H), 7.1-7.2(m,3H), 7.35(d,J=9Hz,1H), 7.65(m,2H), 7.78-(d,J=6Hz,1H), 7.85(d,J=9Hz,2H), 8.05(s,1H), 8.5(d,J=6Hz;1H), 8.85(d,J=9Hz,1H), 9.55(d,J=9Hz,1H). C,H,N analysis calculated for $C_{31}H_{38}N_4O_2$: C 74.66, H 7.68, N 11.24; found: C 75.05, H 7.74, N 11.19.

## Example 11

### N-(3′-(2′-Chloropyridylcarbonyl))-R-Tryptophan-di-n-pentylamide

The hydrochloride of example 2 (0.38 g, 1.0 mmol) was stirred in tetrahydrofuran (THF, 10 mL) with NMM (0.2 mL, 2.0 mmol) under nitrogen atmosphere at 0 °C. EDCI (0.2 g, 1.0 mmol), HOBt (0.27 g, 2.0 mmol), and 2-chloronicotinic acid (0.16 g, 1.0 mmol) were added. The reaction mixture was allowed to stir at ambient temperature overnight. The solvents were removed in vacuo and ethylacetate and water added to the residue. The organic extract was separated and washed with citric acid, $NaHCO_3$ solution, water, brine and then dried over $MgSO_4$. The solution was filtered and concentrated in vacuo and the residue subjected to chromatography using ethylacetate and hexane as the elutant mixture to provide 0.31 g (64%) of oily product which crystallized under vacuum. mp= 81-2 °C. $[\alpha]_D$= -54.5° (c=1.1, MeOH). MS(CI) m/e 483(m+H)$^+$, 326. $^1$H NMR($CDCl_3$,300MHz) $\delta$ 0.8(t,J=7Hz,3H), 0.9(t,J=7Hz,3H), 0.95-1.2(m,8H), 1.25-1.45-(m,4H), 2.7-2.85(m,1H), 2.9-3.1(m,2H), 3.25-3.4(m,3H), 5.4(q,J=6Hz,1H), 7.1(d,J=3Hz,1H), 7.15(m,2H), 7.25-7.4(m,3H), 7.78(d,J=9Hz,1H), 7.95(dd,J=3,7Hz,1H), 8.05(bs,1H), 8.45(dd,J=3,6Hz,1H). C,H,N analysis calculated for $C_{27}H_{35}ClN_4O_2$, 0.5 $H_2O$: C 65.90, H 7.38, N 11.39; found: C 66.11, H 7.09, N 11.26.

## Example 12

N-(4′-Cinnolinylcarbonyl)-R-Tryptophan-di-n-pentylamide

The hydrochloride salt of example 2 (0.38 g, 1.0 mmol) was stirred in THF (8 mL) with NMM (0.2 mL, 2 mmol) under $N_2$ atmosphere at 0 °C. To the mixture was added EDCI (0.2 g, 1.1 mmol), HOBt (0.27 g, 2 mmol), and cinnolinic acid (0.18 g, 1.0 mmol). The reaction mixture was allowed to stir overnight reaching ambient temperature. The solvents were evaporated in vacuo. The residue was processed in a similar fashion as in example 11 to provide 0.41 g (83%) of pure product. mp= 63.5-65 °C. $[\alpha]_D$= -2.2° (c=1.1, MeOH). MS(Cl) m/e 500(m+H)$^+$, 326. $^1$H NMR($CDCl_3$,300MHz) δ 0.86(m,6H), 1.0-1.35(m,8H), 1.4-1.58-(m,4H), 2.95(m,1H), 3.0-3.2(m,2H), 3.35-3.5(m,3H), 5.5(m,1H), 7.15(m,2H), 7.2(t,J=7Hz,2H), 7.4-(d,J=9Hz,1H), 7.75(t,J=7Hz,1H), 7.78(d,J=9Hz,1H), 7.85(t,J=7Hz,1H), 8.15(d,J=9Hz,2H), 8.6-(d,J=9Hz,1H), 9.2(s,1H). C,H,N analysis calculated for $C_{30}H_{37}N_5O_2$, 0.66 $H_2O$: C 70.41, H 7.29, N 13.69; found: C 70.44, H 7.39, N 13.37.

## Example 13

Methyl N-(3′-Quinolylcarbonyl)-R-Tryptophanate

EDCI (1.91 g, 10 mmol) was added to a cooled (4 °C) solution containing quinoline-3-carboxylic acid (1.73 g, 10 mmol), tryptophan methyl ester hydrochloride (2.55 g, 10 mmol), and TEA (2.8 mL, 20 mmol) in 50 mL of methylene chloride. After 4 hrs, the solvent was evaporated and the residue dissolved in ethylacetate and extracted three times with 1 M $H_3PO_4$ (phosphoric acid), three times with 1 M $Na_2CO_3$ - (sodium carbonate), three times with brine and then dried over $MgSO_4$. The solution was filtered and concentrated. The solid residue was recrystallized from EtOAc to yield 2.62 g (73%). $R_f$= 0.13 (1:1 hexanes-EtOAc); 0.80 (80:20:1 $CHCl_3$-MeOH-$NH_4OH$). mp= 205-6 °C. $[\alpha]_D$= +87.5° (c=0.16, 1:1 DMF-MeOH). MS(FAB) m/e 374(m+H)$^+$, 277, 201. $^1$H NMR($CDCl_3$,300MHz) δ 9.17(bs,1H), 8.42(d,J=1.4Hz,1H), 8.13(d,J=9Hz,1H), 7.81(m,2H), 7.62(m,2H), 7.40(d,J=8.5Hz,1H), 7.22(bt,J=7.5Hz,1H), 7.11(t,J=7Hz,1H), 7.06(s,1H), 7.0(bs,1H), 5.21(m,1H), 3.79(s,3H), 3.52(m,2H). C,H,N analysis calculated for $C_{22}H_{19}N_3O_3$: C 70.75, H 5.13, N 11.26; found: C 70.59, H 5.25, N 11.19.

## Example 14

N-(3′-Quinolylcarbonyl)-R-Tryptophan

The product of example 13 (2.5 g, 6.7 mmol) and 1 N NaOH solution (6.7 mL) were dissolved in 100 mL of MeOH and 50 mL of dioxane. An additional 0.67 mL of 1 N NaOH solution was added at 2 and again at 4 hrs and the reaction stirred overnight at ambient temperature. The solvents were evaporated and the residue partitioned between EtOAc and water. The aqueous fraction was separated and acidified and then extracted with EtOAc. These organic washings were combined and washed until neutral (pH~7) then dried over $MgSO_4$. The solution was filtered and concentrated. The resulting solid was dissolved in hot EtOAc-ethanol and this solution was cooled, an equal volume of hexanes was added and the resulting precipitate collected to provide 0.79 g (33%) of product. The filtrate was concentrated to provide 0.77 g (32%) of product. The original EtOAc solution containing residual ester was resubjected to NaOH in the same fashion to provide an additional 0.84 g (35%) of product. mp= 42-3 °C. $[\alpha]_D$= +100° (c=0.08, 1:1 DMF-MeOH). MS(Cl) m/e 360(m+H)$^+$, 342, 314, 296, 213. $^1$H NMR($DMSO_{d6}$,300MHz) δ 3.21-3.4(m,2H), 4.72-4.78(m,1H), 6.98-7.09(m,2H), 7.26(d,J=2Hz,1H), 7.32(d,J=8Hz,1H), 7.64(d,J=7Hz,1H), 7.70(dt,J=1,8Hz,1H), 7.87-(dt,J=1,7Hz,1H), 8.08(bs,1H), 8.11(bs,1H), 8.80(d,J=3Hz,1H), 9.08(d,J=8Hz,1H), 9.22(d,J=2Hz,1H), 10.85-(bs,1H), 12.85(bs,1H).

## Example 15

### N-(3′-Quinolylcarbonyl)-R-Tryptophanamide

Ester from example 13 (68 mg) was treated with a saturated solution of ammonia in methanol (5 mL) at -78 °C. The reaction vessel was sealed and allowed to warm to ambient temperature overnight. Product (37 mg) was isolated via chromatography of the residue after removal of the volatiles in vacuo. mp = 228-30 °C. $[\alpha]_D$ = +90° (c = 0.16, 1:1 DMF-MeOH). MS(CI) m/e 359$(m+H)^+$, 314, 230, 212, 173. $^1H$ NMR-$(DMSO_{d6}$,300MHz) δ 10.8(s,1H), 9.21(d,J = 2Hz,1H), 8.86(d,J = 8Hz,1H), 8.78(d,J = 2Hz,1H), 8.06-(bd,J = 8Hz,2H), 7.86(m,1H), 7.71(m,3H), 7.30(d,J = 7.5Hz,1H), 7.24(s,1H), 7.17(s,1H), 7.05(dt,J = 1,7Hz,1H), 6.98(bt,J = 7Hz,1H), 4.76(m,1H), 3.35(bs,$H_2O$), 3.1-3.3(m,2H). C,H,N analysis calculated for $C_{21}H_{18}N_4O_2$ 0.5, $H_2O$: C 68.64, H 5.22, N 15.26; found: C 68.23, H 5.16, N 14.65.

## Example 16

### Methyl N-(2′-Indolylcarbonyl)-R-Tryptophanate

EDCI (10 mmol) was added to a cooled solution (4 °C) containing indole-2-carboxylic acid (1.61 g, 10mmol), R-tryptophan methyl ester hydrochloride (2.55 g, 10 mmol), HOBt (10 mmol) and TEA (2.8 mL) in $CH_2Cl_2$ (50 mL). Reaction conditions and purification of product proceeded as in example 13 to provide 1.59 g (44%). mp = 220-221 °C. $[\alpha]_D$ = +96.4° (c = 1.08, 1:1 DMF-MeOH). MS(CI) m/e 362$(m+H)^+$. $^1H$ NMR$(DMSO_{d6}$,300MHz) δ 3.15(s,3H), 3.19-3.25(m,2H), 4.20-4.27(m,1H), 6.97-7.08(m,3H), 7.15-(dt,J = 1,7Hz,1H), 7.22(d,J = 2Hz,2H), 7.33(d,J = 7Hz,1H), 7.40(dd,J = 1,8Hz,1H), 7.58(d,J = 7Hz,1H), 7.62-(d,J = 8Hz,1H), 8.81(d,J = 8Hz,1H), 10.84(bd,J = 1Hz,1H), 11.55(s,1H). C,H,N analysis calculated for $C_{21}H_{19}N_3O_3$: C 69.79, H 5.30, N 11.63; found: C 69.76, H 5.39, N 11.51.

## Example 17

### N-(2′-Indolylcarbonyl)-R-Tryptophan

The product of example 16 (1.5 g) and 4.2 mL of 1.0 N NaOH solution were dissolved in 100 mL of methanol. Reaction and processing continued as in example 14. A precipitate was collected (0.50 g) 34% and the filtrate provided an additional 0.44 g (30% yield) of product. $R_f$ = 0.24 (70:30:1 $CHCl_3$-MeOH-$NH_4OH$). MS(FAB) m/e 348$(m+H)^+$. $^1H$ NMR$(DMSO_{d6}$,300MHz) δ 3.18-3.4(m,2H), 4.66-4.72(m,1H), 6.96-7.08(m,3H), 7.14-7.22(m,3H), 7.32(d,J = 8Hz,1H), 7.40(dd,J = 1,8Hz,1H), 7.62(d,J = 8Hz,2H), 8.66-(d,J = 8Hz,1H), 10.83(d,J = 2Hz,1H), 11.54(d,J = 1Hz,1H), 12.8(bs,1H).

## Example 18

### N-(2′-Indolylcarbonyl)-Tryptophan-(1′,R-phenylethyl)amide

EDCI (191 mg, 1.0 mmol) was added to a cooled solution (4 °C) containing the product of example 17 (374 mg, 1.0 mmol), R-α-methylbenzyl amine (0.129 mL, 1.0 mmol), HOBt (135 mg), and TEA (0.139 mL) in

15 mL of $CH_2Cl_2$. The reaction was stirred overnight and reached ambient temperature. After 2 days the solvent had evaporated. The residue was dissolved in EtOAc and extracted with 0.1 M $H_3PO_4$, 0.1 M $Na_2CO_3$, $H_2O$ then dried over $MgSO_4$ and filtered. The crude product obtained from concentration of the filtrate was recrystallized from 80% aqueous ethanol. HPLC (high pressure liquid chromatography) indicated two components in an apparently equal ratio. $R_f$ = 0.31; 0.36 (1:1 hexanes-EtOAc). mp = 215-19° C. $[\alpha]_D$ = +5.6° (c = 0.78, 1:1 DMF-MeOH). MS(CI) m/e 451(m + H)$^+$, 330. $^1$H NMR(DMSO$_{d6}$,300MHz) δ 1.29-(d,J = 7Hz,1.5H), 1.39(d,J = 7Hz,1.5H), 3.04-3.26(m,2H), 4.77-4.89(m,1H), 4.91-5.03(m,1H), 6.95-7.08(m,3H), 7.12-7.42(m,10H), 7.61(dd,J = 3,8Hz,1H), 7.71-7.76(m,1H), 8.51(dd,J = 3,8Hz,1H), 8.57(d,J = 8Hz,0.5H), 8.66-(d,J = 8Hz,0.5H), 10.79(dd,J = 2,5Hz,1H), 11.54(bs,1H). C,H,N analysis calculated for $C_{28}H_{26}N_4O_2$: C 74.64, H 5.82, N 12.44; found: C 74.75, H 5.92, N 12.42.

## Example 19

### N-(2′-Indolylcarbonyl)-Tryptophan-(1′,S-phenylethyl)amide

BOPCl (255 mg, 1.0 mmol) was added to a cooled (4° C) solution of N-(2′-Indolylcarbonyl)-R-Tryptophan (347 mg, 1.0 mmol), S-α-methylbenzylamine (129µL, 1.0 mmol), HOBt (203 mg, 1.5 mmol), and TEA (139 µL, 1.0 mmol) in THF (20 mL). The reaction was allowed to attain room temperature overnight. The solvent was evaporated and the residue was dissolved in EtOAc and extracted with 1 M $H_3PO_4$ (3x), 1 M $Na_2CO_3$ (3x), brine (3x) the dried over $MgSO_4$, filtered and the solvent evaporated in vacuo. The crude product was recrystallized from aqueous EtOH to provide 205 mg, 0.46 mmol (46%). Tlc indicated two components, (1:1 hexanes-EtOAc). The product was purified further by chromatography to yield 12 mg of isomer a (mp = 216-18° C), 32 mg of isomer b (mp = 245-7° C) and 107 mg of mixed fractions (mp = 212-14° C). Isomer a: MS(CI) m/e 451(m + H)$^+$, 373, 330. $^1$H NMR(DMSO$_{d6}$,300MHz) δ 1.38 (d,J = 5Hz,3H), 3.10-(dd,J = 6,8Hz,1H), 3.18(dd,J = 3,8Hz,1H), 4.84-4.87(m,1H), 4.98(m,1H), 6.97-7.06(m,3H), 7.15-7.22(m,4H), 7.24-7.31(m,5H), 7.40(dd,J = <1,5Hz,1H), 7.62(d,J = 5Hz,1H), 7.73(d,J = 5Hz,1H), 8.48(dd,J = 1,5Hz,1H), 8.63-(d,J = 5Hz,1H), 10.77(d,J = 1Hz,1H), 11.53(bs,1H). Isomer b: MS(CI) m/e 451(m + H)$^+$. $^1$H NMR-(DMSO$_{d6}$,300MHz) δ 1.28(d,J = 4Hz,3H), 3.13-3.27(m,2H), 3.34(s,$H_2O$), 4.77-4.83(m,1H), 4.92-4.97(m,1H), 6.98-7.07(m,3H), 7.16(t,J = 4Hz,1H), 7.19-7.22(m,2H), 7.26(s,1H), 7.30(t,J = 4Hz,3H), 7.35(d,J = 4Hz,2H), 7.38-(dd,J = 1,5Hz,1H), 7.60(d,J = 5Hz,1H), 7.73(d,J = 5Hz,1H), 8.49(bd,J = 5Hz,0.5H), 8.55(d,J = 5Hz,0.5H), 10.78-(bs,0.5H), 11.52(s,0.5H). Mixture: $^1$H NMR(DMSO$_{d6}$,300MHz) δ 1.28(d,J = 7Hz,1.65H), 1.39(d,J = 7Hz,1.35H), 3.06-3.28(m,2H), 3.28(s,$H_2O$), 4.87(m,1H), 4.89-5.03(m,1H), 6.95-7.07(m,3H), 7.13-7.37(m,9H), 7.40-(dd,J = 1,4Hz,1H), 7.61(dd,J = 2,7Hz,1H), 7.72(dd,J = 3,8Hz,1H), 8.40-8.47(m,1.5H), 8.55(d,J = 8Hz,0.5H), 10.74(dd,J = 1,5Hz,1H), 11.48(s,1H). C,H,N analysis calculated for $C_{28}H_{26}N_4O_2$, 0.5 $H_2O$ (mixture of isomers): C 73.18, H 5.92, N 12.19; found: C 73.23, H 5.88, N 12.15.

## Example 20

### N-(3′-Quinolylcarbonyl)-Tryptophyl-3′-methyl-5′-phenylpyrazylide

EDCl (190 mg, 1.0 mmol) was added to a cooled (4° C) solution of N-(3′-Quinolylcarbonyl)-R-Tryptophan (360 mg, 1.0 mmol), 3-methyl-5-phenylpyrazole (200 mg), HOBt (130 mg, 1.0 mmol), and TEA (140 µL, 1.0 mmol) in $CH_2Cl_2$ (20 mL). The reaction was allowed to attain room temperature overnight. The solvents were evaporated and the residue was dissolved in EtOAc and extracted with 1 M $H_3PO_4$ (3x), $H_2O$ (3x) then dried over $MgSO_4$, filtered and concentrated in vacuo. The residue was purified by chromatography on silica eluted with a gradient from $CHCl_3$ to 18:1 $CHCl_3$-EtOH. The pure fractions were combined, concentrated and the product crystallized from aqueous EtOH to provide 94 mg, 0.17 mmol (17%). $[\alpha]_D$ = +0.7° (c = 0.54, DMF). MS(FAB) m/e 500(m + H)$^+$, 342, 314. $^1$H NMR(DMSO$_{d6}$,300MHz) δ 2.62(s,3H), 3.29-3.38(m,1H), 3.38(s,$H_2O$), 3.54(dd,J = 4,14Hz,1H), 6.12-6.19(m,1H), 7.02-7.06(m,2H), 7.12(dt,J = 1,8Hz,1H), 7.36(d,J = 8Hz,1H), 7.42(d,J = 2Hz,1H), 7.48-7.51(m,3H), 7.72 (dt,J = 1,7Hz,1H), 7.90(dt,J = 1,7Hz,1H), 7.95-

(d,J = 7Hz,2H), 8.02(dd,J = 1,8Hz,2H), 8.12(dt,J = 1,7Hz,2H), 8.87(d,J = 2Hz,1H), 9.26(d,J = 2Hz,1H), 9.36-(d,J = 8Hz,1H), 10.89(d,J = 2Hz,1H). C,H,N analysis calculated for $C_{31}H_{25}N_5O_2$, 0.33 $H_2O$: C 73.63, H 5.12, N 13.85; found: C 73.71, H 5.24, N 13.77.

## Example 21

### N-(3′-Quinolylcarbonyl)-R-Tryptophan-di-n-pentylamide

EDCI (440 mg, 2.3 mmol) was added to a cooled (4°C) solution of Quinoline-3-carboxylic acid (398 mg, 2.3 mmol), R-Tryptophan-dipentylamide hydrochloride (840 mg, 2.2 mmol), HOBt (68 mg, 0.50 mmol), and TEA (641 µL, 4.6 mmol) in $CH_2Cl_2$ (50 mL). The reaction was allowed to attain room temperature overnight. The solvent was evaporated and the residue was dissolved in EtOAc and extracted with 0.1 M $H_3PO_4$ (3x), 0.1 M $Na_2CO_3$ (3x), $H_2O$ (3x), brine (1x) then dried over $MgSO_4$, filtered and the filtrate concentrated in vacuo. The residue was purified by chromatography on silica and eluted with a step gradient from $CHCl_3$ to 1% EtOH in $CHCl_3$. Yield: 873 mg, 1.75 mmol (76%). $R_f$ = 0.39 (30:1 $CHCl_3$-EtOH). mp = 67-72°C. $[\alpha]_D$ = -21.9° (c = 0.57, $CHCl_3$). MS(CI) m/e 499(m + H)$^+$, 386, 369, 342, 326. $^1$H NMR($CDCl_3$,300MHz) δ 0.82-(t,J = 7Hz,3H), 0.89(t,J = 7Hz,3H), 1.0-1.48(m,12H), 2.79-2.89(m,1H), 2.99-3.12(m,2H), 3.37(d,J = 7Hz,2H), 3.42-3.52(m,1H), 5.45-5.53(m,1H), 7.08(d,J = 2Hz,1H), 7.12-7.23(m,2H), 7.34(bt,J = 7Hz,2H), 7.62-(dt,J = 1,7Hz,1H), 7.78-7.88(m,3H), 8.05(bs,1H), 8.16(d,J = 8Hz,1H), 8.50(d,J = 2Hz,1H), 9.32(d,J = 2Hz,1H). C,H,N analysis calculated for $C_{31}H_{38}N_4O_2$, 0.25 $H_2O$: C 74.00, H 7.71, N 11.14; found: C 74.08, H 7.76, N 11.16.

## Example 22

### Methyl S-Phenylglycinate hydrochloride

S-Phenylglycine (5 g, 33mmol) was refluxed for 4 hours in methanol saturated with HCl gas (50 mL). The solvent was then evaporated and the residue triturated with ether to yield 6.0 g, 30 mmol (91%) of product. mp 224-6°C(dec). $[\alpha]_D$ = -137° (c = 1.0, MeOH). $^1$H NMR($DMSO_{d6}$,300MHz) δ 3.72(s,3H), 5.27-(s,1H), 7.45-7.48(m,3H), 7.50-7.54(m,3H), 9.14(s,3H).

## Example 23

### Methyl N-(t-Butyloxycarbonyl)-R-Tryptophyl-S-Phenylglycinate

EDCI (191 mg, 1.0 mmol) was added to a cooled solution (4°C) of Boc-R-Tryptophan (304 mg, 1.0 mmol), S-Phenylglycine methyl ester (202 mg, 1.0 mmol), HOBt (135 mg, 1.0 mmol) and TEA (279 µL, 2.0 mmol) in dry $CH_2Cl_2$ (10 mL). The reaction was allowed to reach ambient temperature overnight. The solvents were evaporated in vacuo and the residue was dissolved in EtOAc and extracted successively with 1 M $H_3PO_4$ (3x), 1 M $Na_2CO_3$ (3x), brine (3x) then dried over $MgSO_4$, filtered and the filtrate concentrated in vacuo. Yield: 385 mg, 0.85 mmol (85%). $R_f$ = 0.41 (1:1 hexanes-EtOAc); 0.35 (18:1 $CHCl_3$-EtOH). MS(CI) m/e 452(m + H)$^+$, 469(m + $NH_4$)$^+$, 413, 396, 352, 334, 304. $^1$H NMR($CDCl_3$,300MHz) δ 1.42(bs,9H), 3.17-(dd,J = 8,15Hz,1H), 3.30(bdd,J = 5,15Hz,1H), 3.66(s,3H), 4.52(bs,1H), 5.12(bs,1H), 5.46(bd,J = 5Hz,1H), 6.72-(d,J = 7Hz,1H), 6.90(s,1H), 7.10-7.14(m,3H), 7.21(dt,J = 1,7Hz,1H), 7.28-7.31(m,4H), 7.35(d,J = 8Hz,1H), 7.63-(d,J = 7Hz,1H), 7.97(bs,1H).

Example 24

Methyl R-Tryptophyl-S-Phenylglycinate hydrochloride

Methyl Boc-R-Tryptophyl-S-Phenylglycinate (380 mg, 0.84 mmol) was mixed with HCl-Dioxane (4 mL, 16 mmol, pre-cooled to 4° C) under an $N_2$ atmosphere at ambient temperature. After 60 minutes, the reaction was quenched with ether and the resulting solid collected and dried in vacuo. Yield: 299 mg, 0.77 mmol (92%).

Example 25

Methyl N-(2,indolylcarbonyl)-R-Tryptophyl-S-Phenylglycinate

EDCI (75 mg, 0.39 mmol) was added to a cooled (4° C) solution of indole-2-carboxylic acid (63 mg, 0.39 mmol), methyl R-Tryptophyl-S-Phenyglycinate hydrochloride (150 mg, 0.39 mmol), HOBt (53 mg, 0.39 mmol), and TEA (112 µL, 0.8 mmol) in $CH_2Cl_2$ (10 mL). The reaction was allowed to attain ambient temperature overnight. Additional indole-2-carboxylic acid (6 mg), EDCI (8 mg) and TEA (12 µL) were added and the reaction was continued 6 hours. The solvent was evaporated and the residue was dissolved in EtOAc and extracted with 1 M $H_3PO_4$ (3x), 1 M $Na_2CO_3$ (3x), brine (3x). The organic extracts were dried over $MgSO_4$, filtered, and then concentrated. The crude residue was purified by chromatography on silica eluted with 2:1 hexane/EtOAc. The combined product fractions were evaporated and crystallized from aqueous EtOH to provide the product, 65 mg, 0.13 mmol (34%). $R_f$ = 0.30 (18:1 $CHCl_3$-EtOH). mp= 190-1° C. $[\alpha]_D$= +76.7° (c=1.0, 1:1 DMF-MeOH). MS(FAB+) m/e 495(m+H)$^+$, 334, 330. $^1$H NMR-($DMSO_{d6}$,300MHz) δ 3.06-3.22(m,2H), 3.64(s,3H), 4.93-5.02(m,1H), 5.51(dd,J=3,3Hz,1H), 6.96-(dt,J=1,8Hz,1H), 7.0-7.06(m,2H), 7.13-7.22(m,3H), 7.28(d,J=7Hz,1H), 7.35-7.41(m,6H), 7.62(d,J=8Hz,1H), 7.72(d,J=7Hz,1H), 8.52(d,J=8Hz,1H), 9.14(d,J=7Hz,1H), 10.77(d,J=1Hz,1H), 11.55(bs,1H). C,H,N analysis calculated for $C_{29}H_{26}N_4O_4$: C 70.43, H 5.30, N 11.33; found: C 70.16, H 5.42, N 11.23.

Example 26

Ethyl N-(Butyloxycarbonyl)-R-Tryptophyl-(N-benzyl)Glycinate

Boc-R-Tryptophan (1.43 g, 4.7 mmol), ethyl N-benzylglycinate (1.04 g, 5.4 mmol), EDCI (1.05 g, 5.48 mmol), and HOBt (500 mg, 3.7 mmol) were stirred in 10 mL of DMF at 0° C and TEA (700 µL) was added. The reaction was allowed to stir overnight and warm to ambient temperature. The reaction mixture was treated in an analogous fashion to that in example 23 and chromatography with EtOAc/hexane as the elutant mixture provided 1.43 g of an oily product after evaporation of the solvents in vacuo. MS(FAB) m/e 480-(m+H)$^+$, 380, 362, 347, 294. $^1$H NMR($DMSO_{d6}$,300MHz) δ 10.82(bd,J=5.5Hz,1H), 7.5(d,J=7.5Hz,0.5H), 6.85-7.35(m,10.5H), 4.35-4.85(m,4H), 4.08(m,2H), 3.91(m,1H), 2.90-3.05(m,2H), 1.33(s,4.5H), 1.28(s,4.5H), 1.15(m,3H).

Example 27

Ethyl R-Tryptophyl-(N-benzyl)Glycinate hydrochloride

Ethyl Boc-R-Tryptophan-(N-benzyl)glycinate (353 mg, 0.76 mmol) was mixed with HCl-Dioxane (5 mL, 20 mmol, precooled to 4° C) under an $N_2$ atmosphere and allowed to attain ambient temperature. After 1 hour, the solvent was evaporated and the residue was triturated with ether and filtered to give 257 mg, 0.76 mmol (84%) of product. MS(FAB) m/e 380(m+H)$^+$, 347. $^1$H NMR(DMSO$_{d6}$,300MHz) δ 1.13(t,J=7Hz,1.5H), 1.18(t,J=7Hz,1.5H), 3.15-3.22(m,2H), 3.79-4.62(m,7H), 6.96-7.03(m,1H), 7.08-7.19(m,3H), 7.24-7.66(m,6H), 8.34(bs,3H), 11.11(d,J=2Hz,0.5H), 11.15(d,J=2Hz,0.5H).

## Example 28

### Ethyl N-(2′-Indolylcarbonyl)-R-Tryptophyl-(N-benzyl)Glycinate

EDCI (82 mg, 0.43 mmol) was added to a cooled (4° C) solution of indole-2-carboxylic acid (69 mg, 0.43 mmol) ethyl R-Tryptophan-(N-benzyl)glycinate hydrochloride (104 mg, 0.43 mmol), HOBt (58 mg, 0.43 mmol), and TEA (120 μL, 0.86 mmol) in $CH_2Cl_2$ (10 mL). The reaction was allowed to attain ambient temperature overnight. Additional EDCI (8 mg) and TEA (12 μL) were added after 1 day. After 4 days, the solvents were evaporated and the residue was dissolved in EtOAc and extracted with 1 M $H_3PO_4$ (3x), 1 M $Na_2CO_3$ (3x), brine (3x) then dried over $MgSO_4$, filtered and the filtrate concentrated. The residue (which indicated two products by tlc) was purified by chromatography on silica gel eluted with 30:1 $CHCl_3$-EtOH. Early fractions gave 102 mg, 0.2 mmol (45%) of desired product. $R_f$= 0.44 (18:1 $CHCl_3$). $[\alpha]_D$= +17.8° (c=1.0, MeOH). MS(FAB) m/e 523(m+H)$^+$, 507, 436, 393, 362, 330, 302, 285. $^1$H NMR($CDCl_3$,300MHz) 2 amide bond isomers in a 65:35 ratio: δ 1.11(t,J=7Hz,1.1H), 1.23(t,J=7Hz,1.9H), 3.31-3.50(m,2H), 3.73-3.92-(m,1.3H), 4.0(q,J=7Hz,0.7H), 4.12-4.20(m,2H), 4.38(d,J=16Hz,0.6H), 4.53-4.62(m,1H), 4.70-(d,J=15Hz,0.4H), 5.31-5.38(m,0.4H), 5.57-5.65(m,0.6H), 6.84-6.86(m,0.6H), 6.91-6.92(m,0.4H), 6.98-7.29-(m,11H), 7.3-7.40(m,2H), 7.62-7.66(m,1.3H), 7.72(d,J=8Hz,0.7H), 8.01(s,0.4H), 8.08(s,0.6H), 9.18(s,0.4H), 9.21(s,0.6H). C,H,N analysis calculated for $C_{31}H_{30}N_4O_4$, 0.25 $H_2O$: C 70.64, H 5.83, N 10.63; found: C 70.46, H 5.67, N 10.55.

Later fractions provided 36 mg, 0.11 mmol, 25% of undesired diketopiperazine. $R_f$= 0.18 (18:1 $CHCl_3$-EtOH). $[\alpha]_D$= -39.3° (c=0.60, DMF). mp= 204-6°C. MS(CI) m/e 334(m+H)$^+$. $^1$H NMR($CDCl_3$,300MHz) δ 3.08(dd,J=1,18Hz,1H), 3.31-3.45(m,2H), 3.53(dd,J=1,18Hz,1H), 4.14(d,J=15Hz,1H), 4.37-4.41(m,1H), 4.62-(d,J=15Hz,1H), 6.0(bs,1H), 7.03(d,J=2Hz,1H), 7.05-7.08(m,2H), 7.13-7.22(m,2H), 7.25-7.30(m,3H), 7.39-(dt,J=8,1Hz,1H), 7.65(bd,J=8Hz,1H), 8.12(bs,1H). C,H,N analysis calculated for $C_{20}H_{19}N_3O$, 0.25 $CHCl_3$: C 67.23, H 5.34, N 11.57; found: C 66.87, H 5.49, N 11.44.

## Example 29

### Ethyl N-(3′-Quinolylcarbonyl)-R-Tryptophyl-(N-benzyl)Glycinate

EDCI (104 mg, 0.54 mmol) was added to a cooled (4° C) solution of quinoline-3-carboxylic acid (94 mg, 0.54 mmol), ethyl R-Tryptophan-(N-benzyl)glycinate hydrochloride (217 mg, 0.54 mmol), and TEA (151 μL, 1.08 mmol) in $CH_2Cl_2$ (10 mL). The reaction was allowed to attain ambient temperature overnight. After 5 hours, the solvent was evaporated and the residue was dissolved in EtOAc and extracted with 1 M $H_3PO_4$ -(3x), 1 M $Na_2CO_3$ (3x), brine (3x) the dried over $MgSO_4$, filtered and the filtrate concentrated. The residue (which indicated two products by tlc) was purified by chromatography on silica gel eluted with a solvent gradient of 50:1 to 30:1 $CHCl_3$-EtOH. Early fractions provided 64 mg, 0.12 mmol, 22% yield of desired product. $R_f$= 0.15 (30:1 $CHCl_3$-EtOH). mp= 82-85°C. $[\alpha]_D$= +37.0° (c=0.50, DMF). MS(FAB) m/e 535-(m+H)$^+$, 391, 362, 342. $^1$H NMR($CDCl_3$,300MHz), 2 amide bond isomers in a 2:1 ratio: δ 1.17(t,J=7Hz,1H), 1.26(t,J=7Hz,2H), 3.38-3.45(m,0.67H), 3.54(dd,J=7,14Hz,0.33H), 3.76(d,J=17Hz,0.67H), 3.86-(d,J=12Hz,0.33H), 4.03-4.28(m,2H), 4.43(d,J=16Hz,0.67H), 4.54(d,J=15Hz,0.33H), 4.71(d,J=7Hz,0.67H),

4.76(d,J=7Hz,0.33H), 5.34-5.42(m,0.33H), 5.63-5.71(m,0.67H), 7.04-7.12(m,3H), 7.18(dt,J=1,7Hz,2H), 7.23-7.30(m,4H), 7.38(bd,J=8Hz,1H), 7.57-7.68(m,2H), 7.73-7.88(m,2H), 8.07(bs,0.33H), 8.12-8.18(m,1.67H), 8.36-(d,J=2Hz,0.67H), 8.43(d,J=2Hz,0.33H), 9.22(d,J=2Hz,0.67H), 9.28(d,J=2Hz,0.33H). Analysis calculated for $C_{32}H_{30}N_4O_4$, 1.1 $CHCl_3$: C 59.70, H 4.71, N 8.41; found: C 60.07, H 4.91, N 8.41.
Later fractions provided 116 mg, 0.35 mmol, 65% of undesired diketopiperazine identical to that isolated in example 28. $R_f$= 0.06 (30:1 $CHCl_3$-EtOH).

## Example 30

### Methyl R-Phenylglycinate hydrochloride

R-Phenylglycine (5 g, 33 mmol) was refluxed for 2 hours in methanol (50 mL) saturated with HCl gas. The solvent was then evaporated and the residue triturated with ether to give 6.3 g, 31 mmol (94%) of product. mp= 243-5° C. $[\alpha]_D$ = +140° (c=1.0, MeOH).

## Example 31

### Methyl N-(3′-Quinolylcarbonyl)-Tryptophyl-R-Phenylglycinate

EDCI (27 mg, 0.14 mmol) was added to a cooled (4° C) solution of N-(3′-Quinolylcarbonyl)-R-Tryptophan (50 mg, 0.14 mmol), methyl R-Phenylglycinate hydrochloride (28 mg, 0.14 mmol), HOBt (19 mg, 0.14 mmol), and TEA (39 µL, 0.28 mmol) in $CH_2Cl_2$ (5 mL). After 6 hours, the solvents were evaporated and the residue was dissolved in EtOAc and extracted with 1 M $H_3PO_4$ (3x), $H_2O$ (3x) then the solution was concentrated without drying and the residue was recrystallized from aqueous EtOH to give 17.4 mg, 0.034 mmol (24%). The mother liquor was evaporated to give 28 mg, 0.06 mmol (39%). Tlc $R_f$= 0.25 (2:1 hexanes-EtOAc). HPLC: crop 1 shows two peaks in a 46/54 ratio, crop 2 shows a 92/8 ratio (normal phase silica, solvents: hexane, EtOAc in a gradient from 5% to 70% over 10 minutes). Crop 1 (1/1 mixture of diastereomers): mp= 235-7° C. MS(FAB) m/e 507$(m+H)^+$, 342, 334, 314. $^1H$ NMR-($DMSO_{d6}$,300MHz) δ 3.10-3.28(m,2H), 3.63(s,1.5H), 3.66(s,1.5H), 4.98-5.08(m,1H), 5.50(m,1H), 6.95-7.08-(m,2H), 7.24-7.32(m,2H), 7.36-7.46(m,5H), 7.66-7.89(m,4H), 8.04-8.08(m,2H), 8.73(d,J=2Hz,0.5H), 8.78-(d,J=2Hz,0.5H), 8.92-8.96(m,1H), 9.13-9.21(m,2H), 10.79-10.82(m,1H). C,H,N analysis calculated for $C_{30}H_{26}N_4O_4$, 0.5 $H_2O$: C 69.89, H 5.28, N 10.87; found: C 70.18, H 5.01, N 10.83. Crop 2 (predominately one diastereomer): mp= 214-16° C. $[\alpha]_D$= -42.6° (c=0.5, DMF). $^1H$ NMR($DMSO_{d6}$,300MHz) δ 3.22-(dd,J=10,14Hz,1H), 3.33(dd,J=5,14Hz,1H), 3.64(s,3H), 4.97-5.05(m,1H), 5.49(d,J=7Hz,1H), 6.97-7.08-(m,2H), 7.28-7.46(m,8H), 7.68(dt,J=1,7Hz,1H), 7.78(d,J=7Hz,1H), 7.86(dt,J=1,7Hz,1H), 8.06-(dt,J=1,7Hz,1H), 8.74(d,J=2Hz,1H), 8.87(d,J=8Hz,1H), 9.03(d,J=7Hz,1H), 9.17(d,J=2Hz,1H), 10.78-(bd,J=1Hz,1H).

## Example 32

### Methyl N-(3′-Quinolylcarbonyl)-Tryptophyl-S-Phenylglycinate

EDCI (27 mg, 0.14 mmol) was added to a cooled (4° C) solution of N-(3′-Quinolylcarbonyl)-R-Tryptophan (50 mg, 0.14 mmol), methyl S-Phenylglycinate hydrochloride (28 mg, 0.14 mmol), HOBt (19 mg, 0.14 mmol), and TEA (39 µL, 0.28 mmol) in $CH_2Cl_2$ (5 mL). The reaction was allowed to attain room temperature overnight. The reaction mixture was not homogeneous and an additional quantity of TEA (4 µL)

was added. After 2 hours, the solvent was evaporated and the residue was dissolved in EtOAc and extracted with 1 M $H_3PO_4$ (3x, some solid was present and was filtered and dried in vacuo, crop 1), 1 M $Na_2CO_3$ (3x), brine (3x), then dried over $MgSO_4$, filtered and the solvent evaporated to give crop 2. Crop 1: 12 mg, 0.024 mmol, 17%. $R_f$ = 0.24 (18:1 $CHCl_3$-EtOH); 0.28 (2:1 hexanes-EtOAc). mp = 252-4° C. Crop 2: $[\alpha]_D$ = +75.5° (c=0.47, DMF). MS(CI) m/e 507(m+H)$^+$. $^1$H NMR(DMSO$_{d6}$,300MHz) δ 3.06-3.23(m,2H), 3.64(s,3H), 4.95-5.05(m,1H), 5.47-5.53(m,1H), 6.93-7.06(m,3H), 7.13-7.21(m,3H), 7.28(d,J=8Hz,1H), 7.34-7.42(m,6H), 7.62(d,J=8Hz,1H), 7.72(d,J=7Hz,1H), 8.50(d,J=8Hz,1H), 9.10(d,J=7Hz,1H), 10.75-(d,J=1Hz,1H), 11.54(d,J=1Hz,1H) C,H,N analysis calculated for $C_{30}H_{26}N_4O_4$ 0.33 $H_2O$: C 70.29, H 5.24, N 10.93; found: C 70.08, H 5.23, N 10.74.

## Example 33

### N-(3′-Quinolylcarbonyl)-Tryptophan-(N-methyl,N-benzyl)amide

BOPCl (128 mg, 0.50 mmol) was added to a cooled (4° C) solution of N-(3,-Quinolylcarbonyl)-R-Tryptophan (180 mg, 0.50 mmol), N-methylbenzylamine (130 µL, 1.0 mmol), and HOBt (68 mg, 0.50 mmol) in $CH_2Cl_2$ (10 mL). The reaction was allowed to attain room temperature overnight. An additional quantity of BOPCl (13 mg) was added and the reaction was continued. After 3 days, the solvents were evaporated and the residue was dissolved in EtOAc and extracted with 1 M $H_3PO_4$ (3x), 1 M $Na_2CO_3$ (3x), brine (3x) the dried over $MgSO_4$, filtered and the filtrate concentrated in vacuo to provide 148 mg, 0.33 mmol (66% crude yield). The product was purified by chromatography on silica eluted with 2:1 hexanes-EtOAc to pure EtOAc. Purified yield: 64 mg. $[\alpha]_D$ = -5.6° (c=0.55, DMF). MS(FAB) m/e 463(m+H)$^+$, 342, 314, 307. $^1$H NMR-($CDCl_3$,300MHz) δ 2.67(s,2H), 2.86(s,1H), 3.38-3.45(m,2H), 4.47(d,J=14Hz,1H), 4.56(d,J=14Hz,1H), 5.52-5.62(m,1H), 6.95(d,J=7Hz,0.33H), 7.01(dd,J=2,7Hz,0.67H), 7.09-7.43(m,9H), 7.57-7.65(m,1H), 7.74-7.90-(m,3H), 8.01(bs,0.67H), 8.10(bs,0.33H), 8.16(bd,J=8Hz,1H), 8.48(d,J=2Hz,0.33H), 8.51(d,J=2Hz,0.67H), 9.28(d,J=3Hz,0.33H), 9.32(d,J=2Hz,0.67H); (2 amide bond isomers coalesced upon heating in DMSO$_{d6}$ at 125° C). C,H,N analysis calculated for $C_{29}H_{26}N_4O_2$, 0.5 EtOAc 0.5 $H_2O$: C 72.21, H 6.06, N 10.87; found: C 72.06, H 5.87, N 11.09.

## Example 34

### N-(t-Butyloxycarbonyl)-R-Tryptophan-(1′,S-phenylethyl)amide

BOPCl (255 mg, 1.0 mmol) was added to a cooled solution (4° C) of Boc-R-Tryptophan (304 mg, 1.0 mmol), S-α-methylbenzylamine (129 µL, 1.0 mmol), HOBt (135 mg, 1.0 mmol) and TEA (140 µL, 1.0 mmol) in dry THF (15 mL). After 2 hours, the solvents were evaporated in vacuo and the residue was dissolved in EtOAc and extracted successively with 1 M $H_3PO_4$ (3x), 1 M $Na_2CO_3$ (3x), brine (3x) then dried over $MgSO_4$, filtered and the filtrate concentrated in vacuo. The residue was purified by chromatography on silica gel eluted with a 10:1 to 1:1 hexanes-EtOAc gradient. Yield: 199 mg, 0.49 mmol, 49%. $R_f$ = 0.25 (2:1 hexanes-EtOAc); 0.59 (1:1 hexanes-EtOAc). mp = 80-83° C. MS(CI) m/e 408(m+H)$^+$, 425(m+$NH_4$)$^+$, 352, 308, 290. $^1$H NMR($CDCl_3$,300MHz) δ 1.10-1.18(bs,3H), 1.42(s,9H), 3.14(dd,J=8,14Hz,1H), 3.34-(dd,J=5,14Hz,1H), 4.4-4.48(bd,J=5Hz,1H), 4.88-4.97(m,1H), 5.18(bs,1H), 5.86(bd,J=7Hz,1H), 6.98(s,1H), 7.03(d,J=2Hz,1H), 7.07(d,J=2Hz,1H), 7.12-7.25(m,6H), 7.38(dt,J=8,<1Hz,1H), 7.69(d,J=7Hz,1H), 8.08-(s,1H).

## Example 35

R-Tryptophan-(1′,S-phenylethyl)amide hydrochloride

Boc-R-Tryptophan-(1,S-phenylethyl)amide (144 mg, 0.36 mmol) was mixed with HCl-Dioxane (2 mL, 8 mmol, pre-cooled to 4° C) under an $N_2$ atmosphere at ambient temperature. After 1 hour, the reaction was evaporated in vacuo and placed under high vacuum overnight. $R_f$ = 0.59 (80:20:1 $CHCl_3$-MeOH-$NH_4OH$).

Example 36

N-(3′-Quinolylcarbonyl)-R-Tryptophan-(1′,S-phenylethyl)amide

EDCI (76 mg, 0.40 mmol) was added to a cooled (4° C) solution of quinoline-3-carboxylic acid (69 mg, 0.40 mmol), hydrochloride of example 35 ( 0.36 mmol assumed), and TEA (112 µL, 0.8 mmol) in $CH_2Cl_2$ - (10 mL). After 5 hours, an additional 10% equivalent amount of the acid, EDCI and TEA were added. After 1 day, the solvents were evaporated and the mixture treated as in example 19. The concentrated residue was purified by chromatography on silica eluted with 2:1 to 1:2 hexanes-EtOAC to provide 60 mg, 0.13 mmol, 36% of product. $R_f$ = 0.27 (2:1 hexanes-EtOAc). mp = 246-9° C. $[\alpha]_D$ = +15.3° (c = 0.59, 1:1 DMF-MeOH). MS(CI) m/e 463(m + H)$^+$, 342, 290. $^1$H NMR(DMSO$_{d6}$,300MHz) δ 1.28(d,J = 7Hz,3H), 3.18-3.35(m,2H), 4.83-4.99(m,2H), 6.97-7.19(m,2H), 7.19-7.37(m,5H), 7.68(dt,J = 1,7Hz,1H), 7.76(d,J = 8Hz,1H), 7.86-(dt,J = 1,7Hz,1H), 8.04(s,1H), 8.08(s,1H), 8.59(d,J = 8Hz,1H), 8.79(d,J = 2Hz,1H), 8.92(d,J = 7Hz,1H), 9.21-(d,J = 2Hz,1H), 10.83(s,1H). C,H,N analysis calculated for $C_{29}H_{26}N_4O_2$, 0.25 $H_2O$: C 74.58, H 5.72, N 12.00; found: C 74.25, H 5.82, N 11.69.

Example 37

N-(t-Butyloxycarbonyl)-R-Tryptophan-(1′,R-phenylethyl)amide

BOPCl (255 mg, 1.0 mmol) was added to a cooled solution (4° C) of Boc-R-Tryptophan (304 mg, 1.0 mmol), R-α-methylbenzylamine (129 µL, 1.0 mmol), HOBt (135 mg, 1.0 mmol) and TEA (140 µL, 1.0 mmol) in dry THF (15 mL). The reaction was allowed to reach ambient temperature, overnight. The solvents were evaporated in vacuo and the residue was dissolved in EtOAc and extracted successively with 1 M $H_3PO_4$ - (3x), 1 M $Na_2CO_3$ (3x), brine (3x) then dried over $MgSO_4$, filtered and the filtrate concentrated in vacuo. The residue was purified by chromatography on silica gel eluted with a 10:1 to 1:1 hexanes-EtOAc gradient. Yield: 225 mg, 0.56 mmol, 56%. $R_f$ = 0.20 (2:1 hexanes-EtOAc); 0.59 (1:1 hexanes-EtOAc). mp = 80-84° C. MS(CI) m/e 408(m + H)$^+$, 425(m + $NH_4$)$^+$, 352, 308, 290. $^1$H NMR(CDCl$_3$,300MHz) δ 0.82(d,J = 7Hz,3H), 0.93-(s,9H), 3.11(bdd,J = 8,14Hz,1H), 3.31(ddd,J<1;5,14Hz,1H), 4.42(bs,1H), 4.92-5.08(m,1H), 5.2(bs,1H), 5.86-(bd,J = 6Hz,1H), 6.83(s,1H), 7.02(bs,2H), 7.11-7.27(m,6H), 7.69(d,J = 8Hz,1H), 7.84(d,J = 8Hz,1H), 7.90(s,1H).

Example 38

R-Tryptophan-(1′,R-phenylethyl)amide hydrochloride

Boc-R-Tryptophan-(1′,R-phenylethyl)amide (200 mg, 0.49 mmol) was mixed with HCl-Dioxane (1.2 mL, 4.9 mmol, pre-cooled to 4° C) under an $N_2$ atmosphere at ambient temperature. After 2 hours, the volatiles were evaporated in vacuo and placed under high vacuum overnight to provide the product.

## Example 39

### N-(3′-Quinolylcarbonyl)-R-Tryptophan-(1′,R-phenylethyl)amide

EDCI (86 mg, 0.45 mmol) was added to a cooled (4° C) solution of quinoline-3-carboxylic acid (78 mg,.0.45 mmol), hydrochloride of example 38 (140 mg, 0.41 mmol), HOBt (13 mg, 0.10 mmol), and TEA (125 µL, 0.9 mmol) in $CH_2Cl_2$ (15 mL). The reaction was allowed to attain ambient temperature overnight. The solvents were evaporated and the residue treated as in example 19. The crude yield was 174 mg, 0.38 mmol, (92%). Product was recrystallized from 80% aqueous ethanol to provide 67 mg of pure material. $R_f$ = 0.25 (1:2 hexanes-EtOAc). MS(CI) m/e 463$(m+H)^+$, 173. $^1H$ NMR($CDCl_3$,300MHz) δ 1.38(d,J=7Hz,3H), 3.18(dd,J=9,14Hz,1H), 3.50(dd,J=4,14Hz,1H), 4.96-5.10(m,2H), 5.83(d,J=8Hz,1H), 6.72(d,J=2Hz,1H), 7.03-7.06(m,2H), 7.15-7.37(m,7H), 7.62(dt,J=1,7Hz,1H), 7.78-7.89(m,4H), 8.16(d,J=8Hz,1H), 8.50(d,J=2Hz,1H), 9.32(d,J=2Hz,1H). C,H,N analysis calculated for $C_{29}H_{26}N_4O_2$, 0.75 $H_2O$: C 73.17, H 5.82, N 11.77; found: C 73.40, H 5.85, N 11.50.

## Example 40

### N-(3′-Quinolylcarbonyl)-Tryptophan-perhydroisoquinolylamide

BOPCl (152 mg, 0.60 mmol) was added to a cooled (4° C) solution of N-(3′-quinolylcarbonyl)-R-Tryptophan (200 mg, 0.56 mmol), perhydroisoquinoline (192 mg, 1.4 mmol, prepared by catalytic reduction of isoquinoline (cf. Witkop J Am Chem Soc, 70, 2617-19, **1948**), TEA (84 µL, 0.6 mmol) and HOBt (81 mg, 0.60 mmol) in THF (10 mL). The reaction was allowed to attain room temperature overnight. The solvents were evaporated and the residue treated as in example 18. The crude product was purified by chromatography on silica eluted with $CHCl_3$ to 18:1 $CHCl_3$-EtOH to provide 153 mg, 0.32 mmol (57% yield). mp = 124-35° C. MS(CI) m/e 481$(m+H)^+$, 385, 352, 314, 296. $^1H$ NMR($DMSO_{d6}$,300Hz,120° C) δ 1.2-1.8(m,12H), 3.05-3.24(m,3H), 3.28-3.36(m,1H), 3.65-3.82(m,2H), 5.29-5.45(m,1H), 6.96-7.09(m,2H), 7.17(t,J=2Hz,1H), 7.32-7.37(m,1H), 7.62-7.70(m,2H), 7.84(dt,J=1,7Hz,1H), 8.02-8.09(m,2H), 8.50-8.61(m,1H), 8.75-8.79(m,1H), 9.23-9.27(m,1H), 10.48(vbs,1H). C,H,N analysis calculated for $C_{30}H_{32}N_4O_2$, 0.83 $H_2O$: C 72.65, H 6.90, N 11.30; found: C 72.66, H 6.80, N 10.96.

## Example 41

### N-(3′-Quinolylcarbonyl)-Tryptophan-dibenzylamide

BOPCl (152 mg, 0.60 mmol) was added to a cooled (4° C) solution of N-(3′-quinolylcarbonyl)-R-Tryptophan (200 mg, 0.56 mmol), dibenzylamine (268 µL, 1.4 mmol), TEA (84 µL, 0.6 mmol) and HOBt (81 mg, 0.60 mmol) in THF (10 mL). The reaction was allowed to attain room temperature overnight. The solvent was evaporated and the residue was treated as in example 18. The product was purified by chromatography on silica eluted with 2:1 to 1:1 hexanes-EtOAc to provide 100 mg of product, 0.186 mmol (33%). MS(FAB) m/e 539$(m+H)^+$, 366, 342, 314. $^1H$ NMR($CDCl_3$,300MHz) δ 3.38(s,1H), 3.41(s,1H), 4.20-4.23(m,1H), 4.26(bs,2H), 4.46(d,J=16Hz,1H), 4.89(d,J=14Hz,1H), 5.61(apparent q,J=7Hz,1H), 6.86-(d,J=2Hz,1H), 7.02-7.22(m,6H), 7.23-7.36(m,7H), 7.58-7.64(m,2H), 7.78-7.86(m,2H), 7.99(s,1H), 8.15-(d,J=8Hz,1H), 8.43(d,J=2Hz,1H), 9.26(d,J=2Hz,1H). C,H,N analysis calculated for $C_{35}H_{30}N_4O_2$, 0.5 $H_2O$: C 76.76, H 5.71, N 10.23; found: C 76.88, H 5.66, N 10.09.

## Example 42

### N-(t-Butyloxycarbonyl)-S-Tryptophan-N-(3′-quinolyl)amide

BOPCl (255 mg, 1.0 mmol) was added to a cooled solution (4° C) of Boc-S-Tryptophan (304 mg, 1.0 mmol), 3-aminoquinoline (144 mg, 1.0 mmol), HOBt (13 mg, 0.1 mmol) and TEA (140 µL, 1.0 mmol) in dry $CH_2Cl_2$ (10 mL). The reaction was allowed to reach ambient temperature overnight. The solvents were evaporated in vacuo and the residue was treated as in example 18. The residue was purified by chromatography on silica gel eluted with a 2:1 to 1:1 hexanes-EtOAc gradient. Yield: 195 mg, 0.45 mmol, (45%). $R_f$ = 0.25 (1:1 hexanes-EtOAc). MS(Cl) m/e 431(m+H)$^+$, 331. $^1$H NMR($CDCl_3$,300MHz) δ 1.48(s,9H), 3.28(dd,J=8,15Hz,1H), 3.46(dd,J=6,15Hz,1H), 4.69(m,1H), 5.28(bs,1H), 7.10-7.15(m,2H), 7.22-(dt,J=1,7Hz,1H), 7.38(d,J=8Hz,1H), 7.52(dt,J<1,8Hz,1H), 7.62(dt,J=1,8Hz,1H), 7.71(d,J=7Hz,1H), 7.78-(d,J=8Hz,1H), 7.98(s,1H), 8.03(s,1H), 8.20(s,1H), 8.31(d,J=2Hz,1H), 8.58(d,J=1Hz,1H).

## Example 43

### S-Tryptophan-N-(3′-quinolyl)amide hydrochloride

Boc-S-Tryptophan-N-(3′-quinolyl)amide (164 mg, 0.38 mmol) was mixed with HCl-Dioxane (3 mL, 12 mmol, pre-cooled to 4° C) under an $N_2$ atmosphere at ambient temperature. After 2 hours, the volatiles were evaporated in vacuo and placed under high vacuum overnight to provide the product. MS(Cl) m/e 331-(m+H)$^+$. $^1$H NMR($CD_3OD$,300MHz) δ 3.42-3.58(m,2H), 4.4(t,J=7Hz,1H), 6.85(dt,J=1,7Hz,1H), 7.02-(dt,J=1,7Hz,1H), 7.32(s,1H), 7.35(d,J=7Hz,1H), 7.58(d,J=8Hz,1H), 7.94(dt,J=1,7Hz,1H), 8.08-(dt,J=1,7Hz,1H), 8.20(m,J=7Hz,2H), 8.41(d,J=2Hz,1H), 9.26(d,J=2Hz,1H).

## Example 44

### N-Diphenylacetyl-S-Tryptophan-N-(3′-quinolyl)amide

EDCl (67 mg, 0.35 mmol) was added to a cooled (4° C) solution of diphenylacetic acid (74 mg, 0.35 mmol), S-Tryptophan-N-(3′-quinolyl)amide hydrochloride ( 0.32 mmol) and TEA (89 µL, 0.64 mmol) in $CH_2Cl_2$ (10 mL). The reaction was allowed to attain ambient temperature overnight. The solvents were evaporated and the residue was treated as in example 18. The crud eproduct was purified by chromatography on silica eluted with 1:1 hexanes-EtOAc. mp= 233-35° C. [α[$_D$= +17.7° .(c=1.03, 1:1 DMF-MeOH). MS(Cl) m/e 525(m+H)$^+$, 381, 353, 331, 313, 277, 257. $^1$H NMR($CDCl_3$,300MHz) δ 3.26(dd,J=7,14Hz,1H), 3.38(dd,J=7,14Hz,1H), 4.93(s,1H), 5.06(apparent q,J=7Hz,1H), 6.41(d,J=7Hz,1H), 6.87(d,J=2Hz,1H), 7.06-7.16(m,5H), 7.2-7.31(m,7H), 7.38(d,J=8Hz,1H), 7.49(dt,J=1,7Hz,1H), 7.58-7.64(m,2H), 7.72-(dd,J=1,8Hz,1H), 7.99(d,J=8Hz,1H), 8.12(s,1H), 8.36(d,J=2Hz,1H), 8.49(d,J=2Hz,1H), 8.56(s,1H). Analysis calculated for $C_{34}H_{28}N_4O_2$, $H_2O$: C 75.25, H 5.57, N 10.33; found: C 75.13, H 5.14, N 10.07.

## Example 45

### N-(3′-Quinolylcarbonyl)-2-(2′-nitrophenylsulfenyl)-R-Tryptophan-di-n-pentylamide

2-Nitrophenylsulfenylchloride (3.4 mg, 0.02 mmol) was added to N-(3′-quinolylcarbonyl)-R-Tryptophan-dipentylamide (10 mg, 0.02 mmol) in $CHCl_3$. After 3 hours, the reaction mixture was purified by chromatography on silica eluted with $CHCl_3$ to 1% EtOH in $CHCl_3$ to provide 13 mg, 0.02 mmol (100%) of yellow product. MS(CI) m/e 652(m+H)$^+$. $^1$H NMR($CDCl_3$,300MHz) δ 0.81-0.90(m,6H), 1.04-1.37(m,9H), 1.43-1.58(m,4H), 3.12-3.25(m,2H), 3.31-3.48(m,4H), 5.38-5.45(m,1H), 6.33(dd,J=1,7Hz,1H), 7.08-7.18(m,2H), 7.21-7.36(m,4H), 7.58(dt,J=1,7Hz,1H), 7.75-7.83(m,3H), 8.10(d,J=8Hz,1H), 8.13(dd,J=1,8Hz,1H), 8.33-(d,J=2Hz,1H), 8.38(s,1H), 9.10(d,J=2Hz,1H). C,H,N analysis calculated for $C_{37}H_{41}N_5O_4S$: C 68.18, H 6.34, N 10.75; found: C 67.88, H 6.41, N 10.65.

## Example 46

### N-(3′-Quinolylcarbonyl)-Tryptophan-N-(3′-quinolyl)amide

BOPCl (152 mg, 0.60 mmol) was added to a cooled (4°C) solution of N-(3′-quinolylcarbonyl)-R-Tryptophan (200 mg, 0.56 mmol), 3-aminoquinoline (173 mg, 1.2 mmol, recrystallized from hexanes), TEA (167 µL, 1.2 mmol) and HOBt (81 mg, 0.60 mmol) in $CH_2Cl_2$ (15 mL). The reaction was allowed to attain room temperature. After 5 hours, the solvents were evaporated and the residue was treated as in example 18. The crude product was crystallized from aqueous ethanol to provide 60 mg, 0.12 mmol (21% yield). $R_f$= 0.17 (EtOAc). mp= 268-70°C. MS(CI) m/e 486(m+H)$^+$, 385, 342. $^1$H NMR($CDCl_3$-$CD_3OD$,500MHz) δ 3.50(dd,J=4,8Hz,1H), 3.58(dd,J=4,8Hz,1H), 5.28(t,J=4Hz,1H), 7.03(dt,J=<1,4Hz,1H), 7.13-(dt,J=<1,8Hz,1H), 7.21(s,1H), 7.39(d,J=5Hz,1H), 7.56-7.59(m,1H), 7.65-7.72(m,3H), 7.82(d,J=5Hz,1H), 7.85-7.88(dt,J=1,5Hz,1H), 7.98(m,2H), 8.11(d,J=5Hz,1H), 8.59(d,J=1Hz,1H), 8.68(d,J=1Hz,1H), 8.71-(d,J=2Hz,1H), 9.25(d,J=2Hz,1H). Analysis calculated for $C_{30}H_{23}N_5O_2$, $H_2O$: C 71.55, H 5.00, N 13.91; found: C 71.56, H 5.01, N 13.51.

## Example 47

### Methyl S-(p-Hydroxyphenyl)glycinate hydrochloride

S-(p-Hydroxyphenyl)glycine (3 g, 18 mmol) was heated to reflux with saturated HCl in MeOH (50 mL) for 3 hours. After evaporation of the solvents, the residue was triturated with ether to give 3.6 g (14 mmol, 77% yield) of product. mp= 192-3°C(dec). $[\alpha]_D$= +147° (c=1.0, MeOH).

## Example 48

### Methyl N-(t-Butyloxycarbonyl)-R-Tryptophyl-S-(p-hydroxyphenyl)glycinate

BOPCl (254 mg, 1.0 mmol) was added to a cooled solution (4°C) of Boc-R-Tryptophan (304 mg, 1.0 mmol), methyl S-(p-hydroxyphenyl)glycinate (129 µl, 1.0 mmol), HOBt (135 mg, 1.0 mmol) and TEA (279 µl, 2.0 mmol) in dry THF (10 mL). The reaction was allowed to reach ambient temperature overnight. After one day, additional BOPCl (25 mg) and TEA (28µL) were added. After two days, another 109 mg of methyl S-(p-hydroxyphenyl)glycinate, BOPCl (127 mg) and TEA (140µL) were added. After three days, the solvents were evaporated in vacuo and the residue was dissolved in EtOAc and extracted successively with 0.1 M $H_3PO_4$ (3x), brine (3x) then dried over $MgSO_4$, filtered and the filtrate concentrated in vacuo to provide 323 mg of product, 0.69 mmol (69%). $R_f$= 0.14 (1:1 hexanes-EtOAc). mp= 83-7°C. MS(CI) m/e 468(m+H)$^+$, 412, 368, 350. $^1$H NMR($DMSO_{d6}$,500MHz) δ 1.31(s,9H), 2.90(dd,J=5,8Hz,1H), 3.03(dd,J=3,8Hz,1H), 3.62-

(s,3H), 4.30-4.34(m,1H), 5.24(d,J=4Hz,1H), 6.72-6.75(m,3H), 6.96(t,J=5Hz,1H), 7.05(t,J=5Hz,1H), 7.09-7.12(m,3H), 7.31(d,J=5Hz,1H), 7.54(d,J=5Hz,1H), 8.51(d,J=4Hz,1H), 9.53(s,1H).

## Example 49

### Methyl R-Tryptophyl-S-(p-hydroxyphenyl)glycinate hydrochloride

Methyl Boc-R-Tryptophan-S-(p-hydroxyphenyl)glycinate (222 mg, 0.47 mmol) was mixed with HCl-Dioxane (3 mL, 12 mmol, pre-cooled to 4° C) under an $N_2$ atmosphere at ambient temperature. After 2 hours, the reaction mixture was concentrated in vacuo and placed under high vacuum overnight to provide product.

## Example 50

### Methyl N-(3′-Quinolylcarbonyl)-R-Tryptophyl-S-(p-hydroxyphenyl)glycinate

EDCI (80 mg, 0.42 mmol) was added slowly over ~3 hours to a cooled (4° C) solution of quinoline-3-carboxylic acid (73 mg, 0.42 mmol), S-(p-hydroxyphenyl)glycine methyl ester hydrochloride (170 mg, 0.42 mmol), HOBt (6 mg, 0.04 mmol), and TEA (117 µL, 0.84 mmol) in $CH_2Cl_2$ (15 mL). After 2 days, an additional 10% equivalent amount of EDCI and TEA were added and the reaction was continued for one day. The solvents were evaporated and the residue was dissolved in EtOAc and extracted with 0.1 M $H_3PO_4$ (3x), $H_2O$ (3x), and dried over $MgSO_4$. The filtrate was concentrated and the residue purified by chromatography using 1% EtOH in $CH_2Cl_2$ as the elutant mixture to provide product 28 mg (0.048 mmol, 11% yield). MS(Cl) m/e 523$(m+H)^+$, 540$(m+NH_4)^+$. $^1H$ NMR($CD_3OD$,300MHz) δ 3.24-3.43(m,2H), 3.68-(s,3H), 5.07(t,J=7Hz,1H), 5.25(s,1H), 6.71(d,J=8Hz,1H), 6.94-7.02(m,3H), 7.08-7.12(m,2H), 7.33-(d,J=7Hz,1H), 7.62(d,J=8Hz,1H), 7.68(dt,J=1,7Hz,1H), 7.87(dt,J=1,7Hz,1H), 7.99(d,J=8Hz,1H), 8.07-(d,J=8Hz,1H), 8.63(d,J=2Hz,1H), 9.13(d,J=2Hz,1H). C,H,N analysis calculated for $C_{30}H_{26}N_4O_5$, 0.2 $H_2O$, 0.2 $CH_2Cl_2$: C 66.78, H 4.97, N 10.32; found: C 66.83, H 4.73, N 10.14.

## Example 51

### N-(t-Butyloxycarbonyl)-R,S-5-benzyloxytryptophan-di-n-pentylamide

R,S-5-benzyloxytryptophan (2.0 g) was treated with di-t-butyldicarbonate (1.7 g) in a 50% solution of saturated sodium bicarbonate in dioxane at 0° C. The reaction was allowed to warm to ambient temperature and stir overnight. The solvents were evaporated in vacuo and residue partioned between NaOH (1.0 N) and EtOAc. The aqueous portion was then acidified to pH 2.0 using concentrated HCl and the solution extracted with ethylacetate. The EtOAc was dried over $MgSO_4$ and filtered. Evaporation of the volatiles gave 1.12 g which was stirred with dipentylamine (2.5 mL) and BOPCl (1.0 g) in 20 mL of $CH_2Cl_2$ at 0°. After stirring overnight with warming to ambient temperature the reaction mixture was treated as in example 1. The product was purified by crystallization from EtOAc/hexane to provide 997 mg of a white powder. MS(FAB) m/e 550$(m+H)^+$, 450, 433, 360, 265, 236. $^1H$ NMR($CDCl_3$,300MHz) δ 7.88(bs,1H), 7.52(bd,J=7.5Hz,2H), 7.2-7.4(m,5H), 7.02(d,J=2Hz,1H), 6.93(dd,J=2,9Hz,1H), 5.40(bd,J=7.5Hz,1H), 5.13(s,2H), 4.87(m,1H), 3.32-(m,1H), 2.82-3.12(m,5H), 1.42(s,9H), 0.95-1.4(m,12H), 0.87(t,J=7Hz,3H), 0.78(t,J=7Hz,3H).

Example 52

R,S-5-Benzyloxytryptophan-di-n-pentylamide hydrochloride

The product of example 51 (800 mg, 1.5 mmol) was mixed with 4.0 N HCl-Dioxane (7 mL, 28 mmol, pre-cooled to 4° C) under an $N_2$ atmosphere at ambient temperature. After 1 hour, the reaction mixture was evaporated in vacuo and placed under high vacuum overnight to provide the product. MS(FAB) m/e 450-$(m+H)^+$, 433, 265, 236, 213. $^1H$ NMR($DMSO_{d6}$,300MHz) δ 0.73(t,J=7Hz,3H), 0.84(t,J=7Hz,3H), 0.96-1.3-(m,12H), 2.56-2.66(m,1H), 2.72-2.81(m,1H), 2.87-2.96(m,1H), 3.07(dd,J=8,14Hz,1H), 3.17-3.23(m,1H), 3.36-(s,$H_2O$), 4.32(bs,1H), 5.09(s,2H), 6.83(dd,J=2,8Hz,1H), 7.12(d,J=2Hz,1H), 7.25-7.28(m,2H), 7.32-7.42-(m,3H), 7.46-7.49(m,2H), 8.33(bs,2H), 10.91(d,J=2Hz,1H).

Example 53

N-(3′-Quinolylcarbonyl)-R,S-5-benzyloxytryptophan-di-n-pentylamide

EDCI (154 mg, 0.80 mmol) was added to a cooled (4° C) solution of quinoline-3-carboxylic acid (139 mg, 0.80 mmol), hydrochloride salt of example 52 (0.78 mmol) and TEA (223 μL, 1.6 mmol) in $CH_2Cl_2$ (5 mL). The reaction was allowed to attain ambient temperature overnight. The solvents were evaporated and the residue was treated as in example 18. Chromatography of the residue utilizing 1% EtOH in CHCl3 as the elutant mixture provided product 239 mg (0.4 mmol, 51%). $R_f$= 0.19 (30:1 $CHCl_3$-EtOH). MS(FAB) m/e 605$(m+H)^+$, 448, 432, 420. $^1H$ NMR($CDCl_3$,300MHz) δ 0.82(t,J=7Hz,3H), 0.89(t,J=7Hz,3H), 1.02-1.35-(m,10H), 1.40-1.50(m,2H), 2.82-2.92(m,1H), 3.02-3.13(m,2H), 3.32(s,1H), 3.34(s,1H), 3.41-3.51(m,1H), 5.01-(d,J=14Hz,1H), 5.07(d,J=14Hz,1H), 5.43-5.52(m,1H), 6.93(dd,J=2,12Hz,1H), 7.06(d,J=2Hz,1H), 7.23-(s,0.5H), 7.27-7.45(m,7.5H), 7.61(dt,J=1,8Hz,1H), 7.78-7.82(m,1H), 7.87(bd,J=8Hz,1H), 7.96(s,1H), 8.16-(bd,J=8Hz,1H), 8.49(d,J=2Hz,1H), 9.32(s,J=2Hz,1H). Analysis calculated for $C_{38}H_{44}N_4O_3$, 1.5 $H_2O$: C 72.24, H 7.50, N 8.87; found: C 71.91, H 7.12, N 8.63.

Example 54

N-(3′-(3″-Pyridyl)prop-2-enoyl)-R-Tryptophan-di-n-pentylamide

To a solution of the hydrochloride of example 2 (0.2 g, 0.53 mmol) and N-methylmorpholine (0.11 mL) in $CH_2Cl_2$ (10 mL) at 0° C was added HOBt (0.15 g, 1.06 mmol) and 3-(3′-pyridyl)acrylic acid (0.08 g, 0.53 mmol) followed by EDCI (0.11 g, 0.57 mmol). The reaction mixture was stirred overnight at ambient temperature. The solvents were evaporated in vacuo and after the addition of water the mixture was extracted with ethylacetate. The combined ethylacetate extracts were washed with a saturated solution of citric acid, water, a saturated solution of sodium bicarbonate, and brine. The solution was dried over $MgSO_4$ and filtered. Concentration of the solution and chromatography of the residue using ethylacetate and hexane as the elutants provided 0.09 g (37%) of product. $[\alpha]_D$= +5.1° (c=0.75, MeOH). MS(CI) m/e 475$(m+H)^+$, 344, 280. $^1H$ NMR($CDCl_3$,300MHz) δ 0.8(t,J=7Hz,3H), 0.9(t,J=7Hz,3H), 1.08-1.3(m,8H), 1.32(m,4H), 2.8-(m,1H), 2.9(m,1H), 3.05(m,1H), 3.25(d,J=6Hz,2H), 3.35(m,1H), 5.4(apparent q,J=6Hz,1H), 6.5-(d,J=15Hz,1H), 6.8(d,J=9Hz,1H), 7.05(d,J=3Hz,1H), 7.15(m,2H), 7.3(m,2H), 7.63(d,J=15Hz,1H), 7.75-(m,2H), 8.12(s,1H), 8.55(d,J=5Hz,1H), 8.7(s,1H). C,H,N analysis calculated for $C_{29}H_{38}N_4O_2$, 0.33 $H_2O$: C 72.47, H 8.11, N 11.66; found: C 72.12, H 8.14, N 11.55.

## Example 55

### N-(3′-Pyridylcarbonyl)-R-Tryptophan-di-n-pentylamide

The hydrochloride of example 2 (0.15g, 0.4 mmol) was stirred in methylene chloride ($CH_2Cl_2$, 8 mL) with NMM (0.05 mL, 0.4 mmol) under nitrogen atmosphere at 0° C. EDCI (0.095g, 0.5 mmol), HOBt (0.1g, 0.8 mmol) and nicotinic acid (0.05g, 0.4 mmol) were added. The reaction mixture was allowed to stir at ambient temperature overnight. The solvents were removed in vacuo and ethylacetate and water added to the residue. The organic extract was separated and washed with citric acid, $NaHCO_3$ solution, water, brine and then dried over $MgSO_4$. The solution was filtered and concentrated in vacuo and residue subjected to chromatography using ethylacetate and hexane as the eluant mixture to provide 0.085 g (46%) of pure oily product. $[\alpha]_D$= -1.1° (c=0.9, MeOH). MS(Cl) m/e 449(m+H)$^+$, 158. $^1$H NMR($CDCl_3$,300MHz) δ 0.8-(t,J=7Hz,3H), 0.9(t,J=7Hz,3H), 0.95-1.4(m,12H), 2.75-2.8(m,1H), 2.95-3.1(m,2H), 3.3(d,J=6Hz,1H), 3.4-(m,2H), 5.45(apparent q,J=6Hz,1H), 7.1(d,J=3Hz,1H), 7.15(m,2H), 7.3-7.4(m,3H), 7.8(d,J=9Hz,1H), 8.05-(dt,J=3,6Hz,1H), 8.25(bs,1H), 8.7(dd,J=3,6Hz,1H), 9.0(d,J=2Hz,1H). C,H,N analysis calculated for $C_{27}H_{36}N_4O_2$, 0.75 $H_2O$: C 70.17, H 8.18, N 12.13; found: C 70.04, H 8.16, N 12.53.

## Example 56

### N-(9′-Fluorenylidenylacetyl)-R-Tryptophan-di-n-pentylamide

EDCI (50 mg, 0.26 mmol) was added to a cooled (4° C) solution of fluorenylidene acetic acid (66 mg, 0.30 mmol), hydrochloride of example 2 (100 mg, 0.30 mmol), HOBt (7 mg, 0.05 mmol), and TEA (73 µL, 0.52 mmol) in $CH_2Cl_2$ (5 mL). The reaction was allowed to attain ambient temperature overnight. The solvents were evaporated and the residue was treated as in example 18. to provide the product. mp= 68-74° C. $[\alpha]_D$= +29.9° (c=1.08, MeOH). MS(Cl) m/e 548(m+H)$^+$. $^1$H NMR($CDCl_3$,300MHz) δ 0.82-(t,J=7Hz,3H), 0.88(t,J=7Hz,3H), 1.01-1.45(m,12H), 2.82-2.92(m,1H), 3.00-3.12(m,2H), 3.29-3.41(m,3H), 5.43-5.50(m,1H), 6.72(s,1H), 6.86(d,J=8Hz,1H), 7.09-7.28(m,6H), 7.33-7.40(m,3H), 7.62(bt,J=7Hz,2H), 7.78-(dd,J=1,7Hz,1H), 8.02(s,1H), 8.14(d,J=8Hz,1H). C,H,N analysis calculated for $C_{36}H_{41}N_3O_2$: C 78.94, H 7.55, N 7.67; found: C 78.94, H 7.73, N 7.58.

## Example 57

### N-(2′-(3′-Methylindenyl)carbonyl)-R-Tryptophan-di-n-pentylamide

EDCI (50 mg, 0.26 mmol) was added to a cooled (4° C) solution of 3-methylindene-2-carboxylic acid (45 mg, 0.26 mmol), hydrochloride of example 2 (100 mg, 0.30 mmol), HOBt (7 mg, 0.05 mmol), and TEA (73µL, 0.53 mmol) in $CH_2Cl_2$ (5 mL). The reaction was allowed to attain ambient temperature overnight. The solvents were evaporated and the residue was treated as in example 18. The crude product was purified by chromatography to provide 86.5 mg, 0.17 mmol (67%). mp= 60-64° C. $[\alpha]_D$= -12.9° (c=1.11, MeOH). MS-(Cl) m/e 500(m+H)$^+$, 326. $^1$H NMR($CDCl_3$,300MHz) δ 0.79(t,J=7Hz,3H), 0.88(t,J=7Hz,3H), 0.92-1.03(m,1H), 1.06-1.44(m,11H), 2.53(t,J=2Hz,3H), 2.76-2.86(m,1H), 2.95-3.06(m,2H), 3.28-3.31(m,2H), 3.33-3.45(m,1H), 3.59(t,J=2Hz,2H), 5.38-5.44(m,1H), 6.72(d,J=8Hz,1H), 7.06(d,J=2Hz,1H), 7.09-7.21(m,2H), 7.28-7.37-(m,3H), 7.43-7.47(m,2H), 7.78(d,J=8Hz,1H), 8.06(s,1H). C,H,N analysis calulated for $C_{32}H_{41}N_3O_2$, 0.25 $H_2O$: C 76.23, H 8.30, N 8.33; found: C 76.38, H 8.23, N 8.30.

## Example 58

### N-(2′-(5′-Methoxy)indolylcarbonyl)-R-Tryptophan-di-n-pentylamide

EDCI (57 mg, 0.3 mmol) was added to a cooled (4° C) solution of 5-methoxyindole-2-carboxylic acid (57 mg, 0.3 mmol), hydrochloride of example 2 (100 mg, 0.30 mmol), HOBt (7 mg, 0.05 mmol), and TEA (84µL, 0.6 mmol) in $CH_2Cl_2$ (5 mL). The reaction was allowed to attain ambient temperature overnight. The solvents were evaporated and the residue was treated as in example 18. The crude product was purified by chromatography with 2:1 hexanes-EtOAc as the elutant ($R_f$ = 0.54). Chloroform was used to transfer and consolidate fractions. mp = 73-82° C. $[\alpha]_D$ = -18.0° (c = 0.50, MeOH). MS(CI) m/e 517(m + H)$^+$, 388, 329. $^1$H NMR($CDCl_3$,300MHz) δ 0.78(t,J = 7Hz,3H), 0.88(t,J = 7Hz,3H), 0.95-1.43(m,12H), 2.28-2.37(m,1H), 2.93-3.08-(m,2H), 3.30(s,1H), 3.33(s,1H), 3.35-3.45(m,1H), 3.86(s,3H), 5.4-5.47(m,1H), 6.85(d,J = 2Hz,1H), 6.94-(dd,J = 2,8Hz,1H), 7.05(t,J = 3Hz,2H), 7.11-7.22(m,3H), 7.28(m,2H), 7.77(dd,J<1,7Hz,1H), 8.01(bs,1H), 9.18-(bs,1H). C,H,N analysis calulated for $C_{31}H_{40}N_4O_3$, $CHCl_3$: C 60.42, H 6.50, N 8.81; found: C 60.33, H 6.57, N 8.88.

## Example 59

### N-(4′-Hydroxy-3′-iodobenzoyl)-R-Tryptophan-di-n-pentylamide

EDCI (57 mg, 0.3 mmol) was added to a cooled (4° C) solution of 4-hydroxy-3-iodobenzoic acid (79 mg, 0.30 mmol), hydrochloride of example 2 (100 mg, 0.30 mmol), HOBt (13 mg, 0.1 mmol), and TEA (84µL, 0.6 mmol) in $CH_2Cl_2$ (5 mL). The reaction was allowed to attain ambient temperature overnight. The solvents were evaporated and the residue was treated as in example 18. The crude product was purified by chromatography on silica eluted with 2:1 to 1:1 hexanes-EtOAc. Chloroform was used to transfer the fractions. mp = 71-80° C. $[\alpha]_D$ = -2.8° (c = 0.78, 1:1 DMF-MeOH). MS(CI) m/e 590(m + H)$^+$, 464, 335. $^1$H NMR($CDCl_3$,300MHz) δ 0.81(t,J = 8Hz,3H), 0.88(t,J = 8Hz,3H), 1.01-1.08(m,2H), 1.10-1.52(m,10H), 2.93-3.18-(m,3H), 3.28(s,1H), 3.31(s,1H), 3.38-3.46(m,1H), 5.32(apparent q,J = 6Hz,1H), 6.84(d,J = 7Hz,2H), 7.07-(d,J = 2Hz,1H), 7.13-7.22(m,2H), 7.34(d,J = 6Hz,1H), 7.47(dd,J = 2,7Hz,1H), 7.75(d,J = 6Hz,1H), 7.98-(d,J = 2Hz,1H), 8.10(bs,1H). C,H,N analysis calculated for $C_{28}H_{36}IN_3O_3$, 0.5 $CHCl_3$: C 52.00, H 5.74, N 6.38; found: C 52.13, H 5.72, N 6.27.

## Example 60

### N-(t-Butyloxycarbonyl)-R,S-5-fluorotryptophan

In a manner similar to that in example 51 R,S-5-fluorotryptophan (2.0 g) was treated with 2.3 g of di-t-butyldicarbonate and after the standard workup provided 2.45 g (89%) of the desired product. MS(FAB) m/e 323(m + H)$^+$, 295, 279, 267, 223. $^1$H NMR($CD_3OD$,300MHz) δ 7.27(dd,J = 4,9.5Hz,1H), 7.22-(dd,J = 2.5,9.5Hz,1H), 7.14(s,1H), 6.85(dt,J = 2.5,9Hz,1H), 4.88(bs,3H), 4.39(m,1H), 3.25(dd,J = 5,14Hz,1H), 3.06(dd,J = 7.5,14Hz,1H), 1.46(bs,9H).

## Example 61

N-(t-Butyloxycarbonyl)-R,S-5-fluorotryptophan-di-n-pentylamide

In a manner similar to that in example 51, using the product of example 60 (879 mg), dipentyl amine (2 mL), and BOPCl (1.0 g), the product was obtained after standard purification (919 mg, 73%). mp = 134-5° C. MS(Cl) m/e 462(m + H)$^+$, 406, 362, 344. $^1$H NMR($CDCl_3$,300MHz) δ 8.03(bs,1H), 7.32-(bdd,J = 2,9.5Hz,1H), 7.2-7.25(m,1H), 7.07(d,J = 2Hz,1H), 6.94(dt,J = 2,9Hz,1H), 5.39(bd,J = 9Hz,1H), 4.83-(m,1H), 3.34(m,1H), 2.85-3.15(m,5H), 1.42(s,9H), 1.05-1.4(m,12H), 0.87(t,J = 7Hz,3H), 0.80(t,J = 7Hz,3H). C,H,N analysis calculated for $C_{26}H_{40}FN_3O_3$: C 67.63, H 8.74, N 9.10; found: C 67.63, H 8.79, N 8.96.

Example 62

R,S-5-Fluorotryptophan-di-n-pentylamide hydrochloride

Employing similar procedures as in example 2 and using the product of example 61 (491 mg) and 8.0 mL of 4.5 M HCl in dioxane, the product hydrochloride salt was obtained in quantitative yield.

Example 63

N-(3′-Quinolylcarbonyl)-R,S-5-fluorotryptophan-di-n-pentylamide

In a fashion analogous to that in example 3, using the hydrochloride salt of example 62 (200 mg), EDCI (300 mg), HOBt (50 mg), quinoline-3-carboxylic acid (180 mg), and TEA (280 µL) in a DMF solution, the desired product (153 mg) was isolated in 59% yield after standard chromatography with EtOAc/hexane as the elutant mixture. mp = 62-5° C. MS(FAB) m/e 517(m + H)$^+$, 362, 344, 332, 229. $^1$H NMR($CDCl_3$,300MHz) δ 9.30(d,J = 2Hz,1H), 8.52(d,J = 2Hz,1H), 8.15(d,J = 8.5Hz,1H), 8.10(bs,1H), 7.88(dd,J = 0.7,8Hz,1H), 7.80-(dt,J = 1.5,7Hz,1H), 7.62(dt,J = 1,7Hz,1H), 7.42(dd,J = 2.5,9Hz,1H), 7.33(bd,J = 8Hz,1H), 7.27(dd,J = 4,9Hz,1H), 7.12(d,J = 2.5Hz,1H), 6.94(dt,J = 2.5,9Hz,1H), 5.45(m,1H), 3.42-3.50(m,1H), 3.32(m,2H), 3.03-3.13(m,2H), 2.85-2.95(m,1H), 1.05-1.5(m,12H), 0.84(t,J = 7.5Hz,3H), 0.89(t,J = 7.5Hz,3H). C,H,N analysis calculated for $C_{31}H_{37}FN_4O_2$: C 72.05, H 7.22, N 10.85; found: C 72.17, H 7.31, N 10.78.

Example 64

N-(2′-Indolylcarbonyl)-R,S-5-fluorotryptophandi-n-pentylamide

In a fashion analogous to that in example 3 using the hydrochloride salt of example 62 (200 mg), EDCI (300 mg), HOBt (50 mg) indole-2-carboxylic acid (150 mg), and TEA (280 µL) in a DMF solution, the desired product (17 mg) was isolated in 62% yield after standard chromatography with EtOAc/hexane as the elutant mixture. mp = 78-82° C. MS(FAB) m/e 505(m + H)$^+$, 362, 344, 320, 217. $^1$H NMR($CDCl_3$,300MHz) δ 9.30(bs,1H), 8.02(bs,1H), 7.65(bd,J = 7.5Hz,1H), 7.41(m,2H), 7.22-7.30(m,3H), 7.14(t,J = 7.5Hz,1H), 7.09-(d,J = 2Hz,1H), 6.92(dt,J = 2.5,9Hz,2H), 5.39(m,1H), 3.37-3.45(m,1H), 3.27(m,2H), 3.0-3.1(m,2H), 2.85-2.95-(m,1H), 1.0-1.45(m,12H), 0.87(t,J = 7Hz,3H), 0.80(t,J = 7Hz,3H). C,H,N anaysis calculated for $C_{30}H_{37}FN_4O_2$: C 71.39, H 7.40, N 11.10; found: C 71.14, H 7.43, N 10.97.

Example 65

(N$^{\alpha}$,N$^{i}$-Dimethyl)-N-(3′-quinolylcarbonyl)-R-Tryptophan-di-n-pentylamide

The product from example 21 (10 mg, 0.02 mmol) was dissolved in DMF (1 mL), cooled to 0° C and treated with lithium bis(trimethylsilyl)amide (60µL, 0.06 mmol, 1 M in THF) in one portion. After five minutes, methyliodide (3.7 µL, 0.06 mmol) was added in one portion. Additional lithium bis(trimethylsilyl)-amide (30 µL) and methyliodide (3.7 µL) were added after 1 hour and the temperature was allowed to reach room temperature overnight. The solvents were evaporated and the residue was purified by chromatography on silica eluted with a step gradient from 4:1 to 2:1 hexanes-ethylacetate to provide the product. MS-(CI) m/e 527(m+H)$^{+}$, 370, 340. $^{1}$H NMR($CDCl_3$,300MHz) δ 0.80(t,J=7Hz,3H), 0.91(t,J=7Hz,3H), 1.13-1.58-(m,12H), 3.07(s,3H), 3.16-3.26(m,2H), 3.31-3.58(m,4H), 3.76(s,3H), 6.04(t,J=8Hz,1H), 7.02(s,1H), 7.17-(dt,J=1,8Hz,1H), 7.24-7.33(m,2H), 7.59(t,J=7Hz,1H), 7.73-7.82(m,2H), 7.87(d,J=1Hz,1H), 8.12-(d,J=9Hz,1H), 8.75(d,J=1Hz,1H).

Example 66

N-(p-Toluenesulfonyl)-R-Tryptophan-di-n-pentylamide

The product of example 2 (50 mg, 0.13 mmol) was dissolved in methylene chloride (2 mL) and treated with p-toluenesulfonyl chloride (25 mg, 0.13 mmol) and TEA (18 µL, 0.13 mmol). After 5 hours, additional TEA (18 µL) was added and the reaction was left overnight. The solvent was evaporated to provide 67 mg of crude product. $R_f$= 0.23 (18:1 $CHCl_3$-EtOH); 0.62 (1:1 hexanes-ethylacetate). MS(CI) m/e 498(m+H)$^{+}$, 515(m+$NH_4$)$^{+}$, 344. $^{1}$H NMR($CDCl_3$,300MHz) δ 0.88(t,J=7Hz,3H), 0.96(t,J=7Hz,3H), 0.98-1.22(m,12H), 2.38(s,3H), 2.52-2.75(m,2H), 2.84-2.96(m,1H), 2.99-3.08(m,1H), 3.10(s,1H), 3.12(s,1H), 4.33-4.42(m,1H), 5.77-(d,J=12Hz,1H), 7.05-7.14(m,2H), 7.18(d,J=7Hz,2H), 7.26(s,2H), 7.32(dt,J=8,1Hz,1H), 7.50(bd,J=7Hz,1H), 7.67(d,J=8Hz,2H), 8.0(s,1H).

Example 67

N-(p-Chlorobenzenesulfonyl)-R-Tryptophan-di-n-pentylamide

The product from example 2 (500 mg, 1.31 mmol) was treated with p-chlorobenzene sulfonylchloride (277 mg, 1.31 mmol) and TEA (362 µL, 2.6 mmol) in methylene chloride (15 mL) at room temperature overnight. The solvents were evaporated and the residue in ethylacetate was extracted with 0.1% citric acid, 0.1 M sodium bicarbonate and water, then dried over magnesium sulfate and the filtrate concentrated. $R_f$ = 0.88 (1:1 hexanes-ethylacetate). The crude residue was crystallized from aqueous ethanol to provide 478 mg, 0.95 mmol (73%). MS(FAB) m/e 518(m+H)$^{+}$, 484, 344, 326. $^{1}$H NMR($DMSO_{d6}$,300MHz) δ 0.71-(t,J=7Hz,3H), 0.82(t,J=7Hz,5H), 0.91-1.27(m,10H), 2.65-3.07(m,6H), 4.21-4.29(m,1H), 6.93(t,J=7Hz,1H), 7.02-7.07(m,3H), 7.25-7.31(m,2H), 7.53(d,J=8Hz,2H), 7.70(d,J=8Hz,1H), 8.50(d,J=9Hz,1H), 10.83(bs,1H). C,H,N analysis calculated for $C_{27}H_{36}ClN_3O_3S$, 0.1 $H_2O$: C 62.38, H 7.02, N 8.08; found: C 62.26, H 6.86, N 8.10.

Example 68

N-(p-Chlorobenzoyl)-N[i]-acetyl-2,3-dihydro-R-Tryptophan-di-n-pentylamide

The product from example 4 (500 mg, 1.0 mmol) was treated with 10% Pd/C (200 mg) in acetic acid (30 ml) under 3 atmospheres of hydrogen gas overnight in a Paar shaker. After filtration of the catalyst, the solution was treated with excess acetic anhydride (283 µL, 3.0 mmol). After completion of the acetylation, the solvent was evaporated and the residue was purified by chromatography on silica gel to provide the product.

## Example 69

N-(3′-Quinolylcarbonyl)-R-(β-oxyindolyl)Ala-di-n-pentylamide

The product of example 78 (121 mg, 0.24 mmol) was dissolved in dioxane (10 mL) and treated with concentrated hydrochloric acid 1 mL). A red colored solution formed immediately which bleached in 10-15 seconds to provide a colorless solution. After several hours, the reaction mixture was poured into EtOAc and washed until neutral then dried over $MgSO_4$. The crude product was purified by chromatography on silica eluted with a step gradient from 1-5% EtOH in $CH_2Cl_2$ to provide product 97 mg (0.19 mmol, 79% yield). MS(Cl) m/e 515$(m+H)^+$, 369, 358. $^1$H NMR($CDCl_3$,300MHz) δ 0.85-0.96(m,6H), 1.24-1.38(m,8H), 1.48-1.61(m,4H), 1.69(s,H2O), 2.16(ddd,J=3,8,14Hz,0.6H), 2.32(ddd,J=3,8,14Hz,0.4H), 2.50-2.62(m,1H), 3.03-3.16(m,0.6H), 3.18-3.29(m,0.4H), 3.34-3.45(m,1H), 3.51-3.64(m,1.6H), 3.92(m,1H), 5.32-(dt,J=2,8Hz,0.4H), 5.53(dt,J=2,10Hz,0.6H), 6.82(d,J=7Hz,0.6H), 6.37(d,J=7Hz,0.4H), 6.98-7.06(m,1H), 7.12(t,J=7Hz,1H), 7.20(t,J=8Hz,1H), 7.45(d,J=7Hz,0.6H), 7.52-7.58(m,0.4H), 7.62(t,J=8Hz,1H), 7.72-7.88-(m,2H), 8.02(d,J=8Hz,0.4H), 8.08(d,J=8Hz,0.6H), 8.12-8.15(m,1H), 8.37(s,0.4H), 8.42(d,J=2Hz,0.6H), 8.62-(d,J=2Hz,0.4H), 9.20(d,J=2Hz,0.6H), 9.34(d,J=2Hz,0.4H). C,H,N analysis calculated for $C_{31}H_{38}N_4O_3$, 0.3 $H_2O$: C 71.59, H 7.48, N 10.77; found: C 71.55, H 7.40, N 10.61.

## Example 70

Methyl N-(Diphenylmethylene)-R-Tryptophanate

Benzophenone imine (1.0 g, 5.5 mmol) and R-Tryptophan methyl ester hydrochloride (5.5 mmol) were stirred in 20 mL of anhydrous $CH_2Cl_2$ overnight. The ammonium hydrochloride was filtered away and the solvents removed in vacuo. The residue was taken up in diethylether and filtered and the filtrate washed with water and dried over $MgSO_4$. The solution was filtered, concentrated and the remainder crystallized from diethylether/hexane. mp= 112-112.5°C. MS(EI) m/e 382$(m)^+$, 323, 253, 193. $^1$H NMR($CDCl_3$,300MHz) δ 7.92(bs,1H), 7.59(m,2H), 7.10-7.40(m,9H), 6.97(bd,J=3Hz,1H), 6.92(m,1H), 6.60(bd,J=7Hz,2H), 4.41-(dd,J=5,8.5Hz,1H), 3.72(s,3H), 3.47(dd,J=5,15Hz,1H), 3.23(dd,J=8.5,15Hz,1H) C,H,N analysis calculated for $C_{25}H_{22}N_2O_2$: C 78.50, H 5.80, N 7.33; found: C 78.22, H 5.89, N 7.05.

## Example 71

Methyl N-(Diphenylmethylene)-α-allyl-Tryptophanate

The product of example 70 (222 mg), potassium carbonate (260 mg), and tetrabutylammonium hydrogensulfate (27 mg) were stirred at ambient temperature in 4 mL of $CH_2Cl_2$. To this mixture was added

allylbromide (60 µL) and the reaction stirred overnight. The mixture was partitioned between water and ethylacetate and the ethylacetate solution was dried over $MgSO_4$ and filtered. Concentration of the filtrate and chromatography of the residue using ethylacetate and hexane as the elutants provided product.

## Example 72

### Methyl N-(t-Butyloxycarbonyl)-α-allyl-Tryptophanate

The product of example 71 was stirred in dioxane (2 mL) and 3.0 N HCl (3.0 mL) was added to the solution. When tlc analysis indicated that the product had fully reacted, the mixture was carefully neutralized with solid $NaHCO_3$ while cooling. The reaction was made slightly basic and an excess of di-t-butyldicarbonate was added. After an hour at ambient temperature the mixture was partioned between water and ethylacetate. The ethylacetate was dried over $MgSO_4$ and filtered. Chromatography of the concentrated residue yielded product. $^1H$ NMR($CDCl_3$,300MHz) δ 7.55(bd,J = 7.5Hz,1H), 7.25(m,1H), 7.20(dt,J = 1,7Hz,1H), 7.10(bt,J = 7Hz,1H), 6.90(bs,1H), 5.97(ddt,J = 5,10.5,16.5Hz,1H), 5.18(bd,J = 10.5Hz,1H), 5.06(m,2H), 4.68-(m,3H), 3.68(s,3H), 3.28(m,2H), 1.42(bs,9H).

## Example 73

### N-(t-Butyloxycarbonyl)-α-allyl-Tryptophan-di-n-pentylamide

The product of example 72 was treated with 1.0 N NaOH in dioxane at ambient temperature. When tlc indicated that the starting material had been consumed, the reaction mixture was concentrated in vacuo and partioned between water and ethylacetate. The aqueous portion was separated and acidified to pH 2.0 and extracted sucessively with EtOAc. The extracts were dried over $MgSO_4$ and the solution filtered and concentrated. The crude product was treated directly as in example 51 to provide product after chromatography using EtOAc/hexanes as the elutant.

## Example 74

### N-(3′-Quinolylcarbonyl)-α-allyl-Tryptophan-di-n-pentylamide

The product of example 73 was treated with 4.5 N HCl in dioxane as in example 2 to provide product which was used directly in a coupling reaction analogous to that described in example 21. The product was purified by chromatography using EtOAc and hexane.

## Example 75

### $N^{\alpha}$-t-Butyloxycarbonyl-$N^{i}$(t-butyloxycarbonylmethyl)-R-Tryptophan-di-n-pentylamide

The product of example 1 (150 mg, 0.34 mmol) was dissolved in 6 mL of DMF and treated with lithium bis(trimethylsilyl) amide (340 µL, 0.34 mmol, 1.0 M in THF) followed by t-butylbromoacetate (65 µL, 0.4

mmol). After two hours, the reaction was quenched with saturated ammonium chloride and then poured into ethylacetate and extracted and purified as in example 67 to provide a quantitative yield of product. $R_f$ = 0.5 (2:1 hexane-ethylacetate). MS(CI) m/e 558$(m+H)^+$, 502, 458, 440. $^1H$ NMR($CDCl_3$,300MHz) $\delta$ 0.78-(t,J=7Hz,3H), 0.88(t,J=7Hz,3H), 1.05-1.44(m,30H), 1.44(s,$H_2O$), 2.7-2.8(m,1H), 2.85-3.08(m,2H), 3.12-3.15-(m,2H), 3.26-3.38(m,1H), 4.65(s,2H), 4.83-4.92(m,1H), 5.42(d,J=8Hz,1H), 6.91(s,1H), 7.08-7.20(m,3H), 7.69-(d,J=7Hz,1H).

## Example 76

### $N^i$-(Carboxymethyl)-R-Tryptophan-di-n-pentylamide hydrochloride

The product of example 75 (225 mg, 0.4 mmol) was dissolved in 20 mL of 4 N HCl in dioxane. After 5 hours, an additional 20 mL of acid was added and the reaction was continued overnight. The excess reagent was evaporated and the residue was used directly in the next step. $R_f$ = 0.16 (10:3 EtOAc-PAW). PAW = (20:11:6 pyridine-$H_2O$-acetic acid).

## Example 77

### $N^\alpha$-Quinolylcarbonyl-$N^i$-(carboxymethyl)-R-Tryptophan-di-n-pentylamide

$N^\alpha$-Quinoline-3-(N-hydroxysuccinimide) ester (95 mg, 0.35), and the product of example 76 (0.3 mmol assumed) were dissolved in 10 mL of methylene chloride and treated with TEA (49 µL, 0.35 mmol). Additional ester (95 mg) and TEA (49 µL) were added after 1 and again at 2 days. The solvents were evaporated and the residue was extracted as in example 67 and then purified by chromatography eluting with 90:10:0.5 methylene chloride-ethanol-ammonium hydroxide to provide 71 mg, 0.13 mmol (37%) as a yellow glass. $R_f$ = 0.3 (80:20:1 chloroform-methanol-ammonium hydroxide). MS(CI) m/e 557$(m+H)^+$, 400, 372, 340. $^1H$ NMR($DMSO_{d6}$,300MHz) $\delta$ 0.76(t,J=7Hz,3H), 0.83(t,J=7Hz,3H), 1.03-1.42(m,12H), 3.05-3.4-(m,6H), 3.4(bs,$H_2O$), 4.73(s,2H), 5.14(m,1H), 7.02(t,J=7Hz,1H), 7.08(t,J=7Hz,1H), 7.18(s,1H), 7.28-(d,J=7Hz,1H), 7.55(d,J=7Hz,1H), 7.71(d,J=8Hz,1H), 7.86(dt,J=1,7Hz,1H), 8.10(t,J=7Hz,1H), 8.88-(d,J=2Hz,1H), 9.20(d,J=8Hz,1H), 9.26(d,2Hz,1H). C,H,N analysis calculated for $C_{33}H_{40}N_4O_4$, 0.5 $NH_3$, 1.9 $H_2O$: C 66.12, H 7.62, N 10.52; found: C 66.18, H 7.24, N 10.58.

## Example 78

### 2,3-Dihydro-1-(3′-quinolylcarbonyl)-pyrrolo[2,3-b]indole-(2R)-dipentylcarboxamide

The product of example 21 (2.5 g, 5.0 mmol) was dissolved in 50 mL of methylene chloride and cooled to 4°C. Then TEA (2.8 mL, 20 mmol) was added in one portion followed by the addition of t-butylhypochlorite (0.57 mL, 5.0 mmol) over 10 minutes (cf. Ohne, Spande and Witkop J Amer Chem Soc, 92(2), 343, **1970**). The reaction was allowed to attain room temperature overnight. The reaction solution was washed with water then dried over $MgSO_4$. The solution was filtered and the filtrate concentrated. The residue was mixed with 4:1 hexane-ethylacetate and the resulting solid was filtered to provide 1.43 g, 2.9 mmol (58%). mp= 145-53°C. $[\alpha]_D$ = +172° (c=1.04, $CH_2Cl_2$). MS(CI) m/e 497$(m+H)^+$. $^1H$ NMR-($CDCl_3$,300MHz) $\delta$ 0.74(t,J=7Hz,3H), 0.78(t,7Hz,3H), 0.85-1.13(m,12H), 1.26-1.34(m,1H), 2.85-2.94(m,3H), 3.05-3.13(m,3H), 5.50(dd,J=3,10Hz,1H), 7.11-7.13(m,2H), 7.32-7.39(m,2H), 7.52(t,J=7Hz,1H), 7.8-(dt,J=1,7Hz,1H), 7.86(d,J=7Hz,1H), 8.13(d,J=8Hz,1H), 8.35(d,J=2Hz,1H), 9.06(d,J=2Hz,1H), 9.38(s,1H).

C,H,N analysis calculated for $C_{31}H_{36}N_4O_2$, 0.4 $H_2O$: C 73.90, H 7.36, N 11.12; found: C 73.92, H 7.40, N 11.21.

## Example 79

### $N^{\alpha}$-(3′-Quinolylcarbonyl)-R-(2-phenylthio)Tryptophan di-n-pentylamide

The product of example 78 (50 mg, 0.1 mmol), $NH_4HCO_3$ (0.5M, 400 µL, 0.2 mmol) and thiophenol (103 µL, 1.0 mmol) were dissolved in 1 mL of dioxane and stirred overnight at 55° C. The reaction was poured into 0.1 M citric acid and extracted with ethylacetate then washed with water, dried over $MgSO_4$, filtered and the filtrate concentrated. The residue was then purified by chromatography on silica gel eluted with a methylene chloride to 20:1 methylene chloride-ethanol step gradient to provide 14.5 mg, 0.024 mmol (24%). MS(CI) m/e 607(m+H)$^+$, 497, 158; MS(FAB) m/e 607(m+H)$^+$, 422, 325. $^1$H NMR($CDCl_3$,300MHz) δ 0.74-0.83(m,6H), 1.0-1.45(m,12H), 2.94-3.11(m,2H), 3.18-3.42(m,4H), 5.4(apparent q,J=8Hz,1H), 6.96-7.27-(m,7H), 7.51(dt,J=1,7Hz,1H), 7.67-7.73(m,3H), 8.04(dd,J=1,8Hz,1H), 8.28(d,J=2Hz,2H), 9.10(d,J=2Hz,1H).

## Example 80

### $N^{\alpha}$-(3′-Quinolylcarbonyl)-R-(2-butylthio)Tryptophan-di-n-pentylamide

The product of example 78 (250 mg, 0.5 mmol), sodium bicarbonate (63 mg, 0.75 mmol) and n-butanethiol (5 mL, 47 mmol) were dissolved in 5 mL DMF and warmed to 60° C for 4 days. The solvent was evaporated and the residue in ethylacetate was extracted as in example 67. The residue was purified by chromatography on silica gel eluted with a 10:1 to 4:1 methylene chloride-ethylacetate step gradient to provide 89 mg, 0.15 mmol (30%) and 121 mg (48%) of recovered starting material. MS(CI) m/e 587(m+H)$^+$, 325. $^1$H NMR($CDCl_3$,300MHz) δ 0.84-0.91(m,9H), 1.1-1.62(m,16H), 2.78(dt,J=1,8Hz,2H), 2.96-3.06(m,1H), 3.09-3.30(m,2H), 3.34-3.45(m,3H), 5.45(apparent q,J=7Hz,1H), 7.12(dt,J=1,8Hz,1H), 7.19(dt,J=1,8Hz,1H), 7.31(t,J=7Hz,2H), 7.59(dt,J=1,8Hz,1H), 7.73-7.85(m,3H), 8.10(s,1H), 8.13(d,J=8Hz,1H), 8.41(d,J=2Hz,1H), 9.23(d,J=2Hz,1H). C,H,N analysis calculated for $C_{35}H_{46}N_4O_2S$: C 71.63, H 7.90, N 9.55; found: C 71.54, H 7.97, N 9.42.

## Example 81

### $N^{\alpha}$-t-Butyloxycarbonyl-R-5-hydroxytryptophan

Di-t-butyldicarbonate (327 mg, 1.5 mmol) was added to 5-hydroxy-R-Tryptophan (250 mg, 1.14 mmol) and TEA (167 µL, 1.2 mmol) in 10 mL THF and 5 mL of water. After 1 day, the solvent was evaporated and the residue in ethylacetate was extracted with 0.1 M citric acid and water then dried over $MgSO_4$ and the filtrate concentrated. The product was used directly in the next step.

## Example 82

### $N^{\alpha}$-t-Butyloxycarbonyl-R-5-hydroxytryptophan-di-n-pentylamide

The product of example 81 (1.5 mmol assumed) and dipentylamine (760 µL, 3.75 mmol) were dissolved in 20 mL methylene chloride and treated with BOPCl (382 mg, 1.5 mmol) overnight. The solvents were evaporated and the residue was extracted as in example 81 followed by chromatography on silica gel eluted with a 4:1 to 1:1 hexane-ethylacetate step gradient to provide 288 mg, 0.63 mmol (42% yield). mp = 60-65 °C. MS(CI) m/e 460(m+H)$^+$, 404, 360, 342. $^1$H NMR($CDCl_3$,300MHz) δ 0.77(t,J = 7Hz,3H), 0.85-(t,J = 7Hz,3H), 0.95-1.38(m,12H), 1.43(s,9H), 2.68-2.78(m,1H), 2.82-2.93(m,1H), 2.98-3.15(m,3H), 3.26-3.36-(m,1H), 4.83-4.92(m,1H), 5.52(d,J = 8Hz,1H), 5.80(s,1H), 6.78(dd,J = 2,8Hz,1H), 6.98(d,J = 2Hz,1H), 7.16-(s,1H), 7.18(d,J = 8Hz,1H), 7.93(s,1H).

## Example 83

### R-5-Hydroxytryptophan-di-n-pentylamide hydrochloride

The product of example 82 (270 mg, 0.59 mmol) was treated with 5 mL of 4 N HCl/dioxane for 1 hour. The excess acid and solvents were evaporated and the residue was placed on high vacuum overnight to provide 243 mg, 0.6 mmol, quantitative yield.

## Example 84

### N$^\alpha$-(3′-Quinolylcarbonyl)-R-5-hydroxytryptophan-di-n-pentylamide

Quinoline-3-carboxylic acid (26 mg, 0.15 mmol), the product of example 83 (50 mg, 0.13 mmol) and TEA (21 µL, 0.15 mmol) were dissolved in 5 mL of 1:1 THF and treated with EDCI (29 µL, 0.15 mmol) overnight. The solvent was evaporated and the residue in ethylacetate was extracted with 0.1 M citric acid and water. After drying over $MgSO_4$, the filtered solution was concentrated and then placed under high vacuum overnight to provide 72 mg, 0.14 mmol (93%) as a glass. mp = 78-90 °C. MS(CI) m/e 515(m+H)$^+$, 358, 342, 330. $^1$H NMR($CDCl_3$,300MHz) δ 0.69(t,J = 7Hz,3H), 0.76(t,J = 7Hz,3H), 0.82-1.28(m,12H), 2.84-3.12-(m,3H), 3.22(dd,J = 4,14Hz,1H), 3.51(d,J = 2Hz,1H), 3.60(dd,J = 10,14Hz,1H), 5.43(dt,J = 4,8Hz,1H), 6.88-(dd,J = 2,8Hz,1H), 7.10(d,J = 2Hz,1H), 7.22(d,J = 8Hz,1H), 7.40(d,J = 2Hz,1H), 7.62(dt,J = 1,7Hz,1H), 7.82-(dt,J = 1,7Hz,1H), 7.90(dd,J = 1,8Hz,1H), 8.01(d,J = 2Hz,1H), 8.23(d,J = 8Hz,1H), 8.49(d,J = 8Hz,1H), 8.62-(s,1H), 8.72(d,J = 1Hz,1H), 9.62(d,J = 2Hz,1H). C,H,N analysis calculated for $C_{31}H_{38}N_4O_3$, 0.5 $H_2O$: C 71.10, H 7.51, N 10.70; found: C 71.23, H 7.50, N 10.45.

## Example 85

### N$^\alpha$-(3′-Quinolylcarbonyl)-R-Tryptophyl-3,5-dimethylpyrazylide

N$^\alpha$-(3′-Quinolylcarbonyl)-R-Tryptophyl (36o mg, 1.0 mmol), 3,5-dimethylpyrazole (96 mg, 1.0 mmol) and HOBt (270 mg, 2.0 mmol) were dissolved in 10 mL of methylene chloride and treated with EDCI (197 mg, 1.0 mmol) at 4 °C and allowed to attain room temperature overnight. TEA (140 µL, 1.0 mmol) was added after 1 day as well as additional pyrazole (10 mg) and EDCI (20 mg). After 2 days, the solvents were evaporated and the residue was extracted and purified as in example 67. MS(FAB) m/e 438(m+H)$^+$, 342, 314, 265. $^1$H NMR($DMSO_{d6}$,300MHz) δ 2.12(s,2H), 2.36(s,3H), 2.52(s,3H), 3.22-3.49(m,2H), 5.96-6.02(m,1H), 6.32(s,1H), 7.02-7.11(m,2H), 7.32-7.36(m,2H), 7.71(dt,J = 1,8Hz,1H), 7.38(dt,J = 1,7Hz,1H), 7.94-

(dd,J=1,7Hz,1H), 8.10(dt,J=<1,7Hz,2H), 8.83(d,J=2Hz,1H), 9.21-9.24(m,2H), 10.85(d,J=1Hz,1H). C,H,N analysis calculated for $C_{26}H_{23}N_5O_2$ 0.5 $H_2O$: C 69.94, H 5.42, N 15.69; found: C 70.11, H 5.47, N 15.23.

## Example 86

### $N^{\alpha}$-(3′-Quinolylcarbonyl)-R-Tryptophan(bis(2′-hydroxyethyl))amide

$N^{\alpha}$-(3′-Quinolylcarbonyl)-R-Tryptophan (150 mg, 0.49 mmol) and diethanolamine (240 µL, 2.5 mmol) were dissolved in 10 mL THF and treated with BOPCl (127 mg, 0.50 mmol) and stirred overnight. The solvents were evaporated and the residue was purified by chromatography on silica gel eluted with 20:1 methylene chloride-ethanol to provide 144 mg, 0.32 mmol (66%). $R_f$= 0.52 (80:20:1 chloroform-methanol-ammonium hydroxide). MS(CI) m/e 447(m+H)$^+$, 429, 342, 274. $^1$H NMR($CDCl_3$-$D_2O$,300MHz) δ 3.20(s,1H), 3.23-3.40(m,3H), 3.44-3.52(m,4H), 3.54-3.62(m,2H), 5.27(dd,J=6,8Hz,1H), 7.02(t,J=7Hz,1H), 7.11-(t,J=7Hz,1H), 7.27(s,1H), 7.37(d,J=8Hz,1H), 7.71(d,J=8Hz,1H), 7.76(d,J=7Hz,1H), 7.93(dt,J=1,7Hz,1H), 8.10(d,J=8Hz,2H), 8.29(d,J=2Hz,1H), 9.17(d,J=2Hz,1H). C,H,N analysis calculated for $C_{25}H_{26}N_4O_4$, 0.7 $H_2O$: C 65.40, H 6.02, N 12.20; found: C 65.58, H 6.03, N 11.89.

## Example 87

### $N^{\alpha}$-(3′-Quinolylcarbonyl)-R-Tryptophan(N-ethoxyethyl, N-2′hydroxyethyl)amide

The product of example 86 (50 mg, 0.112 mmol) was dissolved in 5 mL of DMF and treated with lithium bis(trimethlysilyl)amide (220 µL, 0.110 mmol, 0.5 M in toluene) and ethyliodide (38 µL, 0.48 mmol). The crude reaction mixture was purified by chromatography on silica gel eluted with 1% ethanol in methylene chloride to provide product. mp= 195-8°C. $[\alpha]_D$= +38.4 (c=0.63, DMF). MS(FAB) m/e 475(m+H)$^+$. $^1$H NMR($CDCl_3$,300MHz) δ 1.42(t,J=7Hz,3H), 3.08(dq,J=2,15Hz,1H), 3.35-3.80(m,9H), 4.06(bs,1H), 4.13-(q,J=7Hz,2H), 4.95-5.03(m,1H), 7.13(dt,J=1,7Hz,2H), 7.2-7.27(m,2H), 7.35(d,J=8Hz,1H), 7.56-(dt,J=1,7Hz,1H), 7.72(t,J=7Hz,1H), 7.79(dt,J=1,7Hz,1H), 7.92(d,J=7Hz,1H), 8.11(d,J=8Hz,1H), 8.33-(d,J=2Hz,1H), 9.22(d,J=2Hz,1H). C,H,N analysis calculated for $C_{27}H_{30}N_4O_4$, 1.0 $H_2O$: C 65.84, H 6.55, N 11.37; found: C 65.64, H 6.02, N 11.13.

## Example 88

### $N^{\alpha}$-(3′-Quinolylcarbonyl)-R-Tryptophan-(bis(ethoxyethyl)amide

The product of example 86 (60 mg, 0.134 mmol) was dissolved in 5 mL of DMF and treated with lithium bis(trimethylsilyl)amide (540 µL, 0.27 mmol, 0.5 M in toluene) and ethyliodide (43 µL, 0.54 mmol). After 2 hours, additional base (270 µL) and ethyliodide (43 µL) were added and the reaction was stirred overnight. The solvents were evaporated and the residue was treated as in example 81 to provide 68 mg, 0.14 mmol (quantitative). MS(CI) m/e 503(m+H)$^+$, 485, 457, 398, 330. $^1$H NMR($CDCl_3$,300MHz) δ 1.06(t,J=7Hz,1.5H), 1.15(t,J=7Hz,1.5H), 1.37-1.46(m,3H), 3.2-3.68(m,12H), 4.13(q,J=7Hz,2H), 5.47-5.59(m,1H), 7.05-7.38(m,5H), 7.57-7.87(m,5H), 8.08-8.17(m,1H), 8.43(d,J=2Hz,0.5H), 8.50(d,J=2Hz,0.5H), 9.25(d,J=2Hz,0.5H), 9.32-(d,J=2Hz,0.5H).

## Example 89

N$^{\alpha}$-(3′-Quinolylcarbonyl)-R-Tryptophan-(N-benzyloxyethyl, N-hydroxyethyl)amide (a)

N$^{\alpha}$-(3′-Quinolylcarbonyl)-R-Tryptophan-(bis(benzyloxyethyl)amide (b)

The product of example 86 (50 mg, 0.112 mmol) was alkylated with benzylbromide (13 µL, 0.11 mmol) as in example 87. Chromatography on silica gel eluted with a 1% to 5% ethanol in methylene chloride in a step gradient to provide 8.0 mg of dialkylated (b) and 23.2 mg of monoalkylated (a) product. Monoalkylated (a): MS(FAB) m/e 537(m+H)$^+$, 307, 220, 185. $^1$H NMR(CDCl$_3$,300MHz) δ 3.03(dq,J=3,15Hz,1H), 3.4-3.68-(m,8Hz), 3.76-3.86(m,2H), 4.02(bs,1H), 4.60(bs,1H), 5.23(s,2H), 5.50(apparent q,J=7Hz,1H), 7.01-7.22-(m,7H), 7.30(d,J=8Hz,1H), 7.49-7.56(m,2H), 7.72(d,J=8Hz,1H), 7.74-7.79(m,1H), 8.06(dd,J=<1,8Hz,1H), 8.16(d,J=2Hz,1H), 8.38(d,J=7Hz,1H), 9.16(d,J=2Hz,1H). C,H,N analysis calculated for $C_{32}H_{32}N_4O_4$ 3.5 $H_2O$: C 64.09, H 6.56, N 9.34; found C 64.36, H 5.55, N 8.99. Dialkylated (b): MS(FAB) m/e 627(m+H)$^+$, 460, 404, 307, 220. $^1$H NMR(CDCl$_3$,300MHz) δ 3.30-3.45(m,5H), 3.5-3.66(m,5H), 4.26(d,J=14Hz,0.5H), 4.32-(d,J=14Hz,0.5H), 4.37(s,1H), 5.22(s,1H), 5.23(s,1H), 5.47-5.56(m,1H), 7.03-7.38(m,16H), 7.56-7.63(m,1H), 7.70-7.84(m,3H), 8.12-8.17(m,1H), 8.38(d,J=2Hz,0.5H), 8.42(d,J=2Hz,0.5H), 9.22(d,J=2Hz,0.5H), 9.28-(d,J=2Hz,0.5H).

## Example 90

N′(8′-Hydroxy-2′-quinolylcarbonyl)-R-Tryptophan-di-n-pentylamide

In a manner similar to example 58, 8-Hydroxyquinoline-2-carboxylic acid (50 mg, 0.26 mmol) was coupled to the product of example 2 (100 mg, 0.26 mmol). The concentrated residue after extraction and drying was crystallized from ethylacetate-hexane to provide 86 mg, 0.16 mmol (60% yield). $R_f$= 0.4 (30:1 methylene chloride-ethanol). MS(CI) m/e 515(m+H)$^+$, 189, 158. $^1$H NMR(CDCl$_3$,300MHz) δ 0.80-(t,J=7Hz,3H), 0.85(t,J=7Hz,3H), 1.0-1.32(m,9H), 1.35-1.5(m,3H), 2.85-3.12(m,3H), 3.38(s,1H), 3.41(s,1H), 3.43-3.52(m,1H), 5.45-5.53(m,1H), 7.10-7.23(m,4H), 7.33(t,J=1Hz,1H), 7.37(t,J=1Hz,1H), 7.52(t,J=8Hz,1H), 7.81(d,J=7Hz,1H), 8.02(s,1H), 8.07(s,1H), 8.23(s,2H), 8.85(d,J=8Hz,1H). C,H,N analysis calculated for $C_{31}H_{38}N_4O_3$: C 72.34, H 7.44, N 10.89; found: C 72.38, H 7.61, N 10.66

## Example 91

N-(4′,8′-Dihydroxy-2′-quinolylcarbonyl)-R-Tryptophan-di-n-pentylamide

In a manner similar to example 58, 4,8-dihydroxyquinoline-2-carboxylic acid (205 mg, 1.0 mmol) was coupled to the product of example 2 (380 mg, 1.0 mmol). After 3 days, the reaction mixture was concentrated and the crude product residue was purified by chromatography on silica gel eluted with 1% to 6% ethanol in methylene chloride step gradient to provide 59 mg, 0.11 mmol (11%). $R_f$= 0.41 (18:1 methylene chloride-ethanol). MS(FAB+) m/e 531(m+H)$^+$, 375, 346, 328. $^1$H NMR(CD$_3$OD,300MHz) δ 0.77-(t,J=7Hz,3H), 0.87(t,J=7Hz,3H), 0.94-1.45(m,12H), 2.97-3.13(m,3H), 3.23-3.45(m,3H), 5.33-5.39(m,1H), 7.01-7.15(m,4H), 7.32-7.40(m,3H), 7.65-7.72(m,2H). C,H,N analysis calculated for $C_{31}H_{38}N_4O_4$, 0.5 $H_2O$: C 68.99, H 7.28, N 10.38; found: C 69.06, H 7.24, N 10.30.

Example 92

N-(6′-Acetoxy-2′-naphthoyl)-R-Tryptophan-di-n-pentylamide

In a manner similar to example 58, 6-Acetoxynaphthalene-2-carboxylic acid (230 mg, 1.0 mmol), the product of example 2 (380 mg, 1.0 mmol) were coupled with EDCI. After 2 days, the solvents were evaporated and the residue was extracted with 0.1 M citric, 0.1 M $NaHCO_3$, $H_2O$ and dried over $MgSO_4$. The residue from evaporation of the volatiles was purified by chromatography on silica gel eluted with 20:1 methylene chloride-ethanol solvent system to provide 467 mg, 0.87 mmol (87% yield). MS(Cl) m/e 556-$(m+H)^+$, 514. $^1H$ NMR($CDCl_3$,300MHz) $\delta$ 0.79(t,J=7Hz,3H), 0.89(t,J=7Hz,3H), 0.97-1.48(m,12H), 2.38-(s,3H), 2.78-2.88(m,1H), 2.96-3.08(m,2H), 3.32-3.48(m,3H), 5.43-5.52(m,1H), 7.07(d,J=3Hz,1H), 7.12-(dt,J=1,7Hz,1H), 7.19(dt,J=1,7Hz,1H), 7.26-7.36(m,3H), 7.59(d,J=2Hz,1H), 7.80-7.92(m,4H), 8.06(s,1H), 8.28(s,1H). C,H,N analysis calculated for $C_{34}H_{41}N_3O_4$, $H_2O$: C 73.22, H 7.77, N 7.53; found: C 72.92, H 7.52, N 7.42.

Example 93

N-(6′-Hydroxy-2′-naphthoyl)-R-Tryptophan-di-n-pentylamide

The product of example 92 (200 mg, 0.37 mmol) was dissolved in 10 mL methanol and treated with 1 N NaOH (37 µL, 0.37 mmol). After 3 hours, the solvents were evaporated and the residue was extracted with 0.1% citric acid and water then dried over MgSO4. The cocentrated filtrate was purified by chromatography on silica gel eluted with 4:1 hexanes-ethylacetate to provide a quantitative yield of product. MS(Cl) m/e 514-$(m+H)^+$, 327, 158. $^1H$ NMR($CDCl_3$,300MHz) $\delta$ 0.82(t,J=7Hz,3H), 0.91(t,J=7Hz,3H), 1.03-1.37(m,9H), 1.44-1.62(m,3H), 3.08-3.24(m,3H), 3.38-3.51(m,3H), 5.41(apparent q,J=8Hz,1H), 6.91(d,J=1Hz,1H), 6.92-(d,J=8Hz,1H), 7.03(dd,J=2,9Hz,1H), 7.10(d,J=2Hz,1H), 7.14-7.26(m,3H), 7.29(d,J=9Hz,1H), 7.35-7.44-(m,2H), 7.57(d,J=1Hz,1H), 7.82(dd,J=1,7Hz,1H), 8.16(d,J=2Hz,1H), 9.22(s,1H). C,H,N analysis calculated for $C_{32}H_{39}N_3O_3$: C 74.79, H 7.50, N 8.18; found: C 74.76, H 7.71, N 8.12.

Example 94

N-Benzyloxycarbonyl-1,2,3,4-tetrahydroisoquinoline-3-carboxylic acid

1,2,3,4-Tetrahydro-isoquinoline-3-carboxylic acid (500 mg, 2.3 mmol) and Cbz-OSu (874 mg, 3.5 mmol) were dissolved in 30 mL of 1:1:1 dioxane-water-methanol treated with TEA (641 µL, 4.6 mmol) and left stirring overnight. The mixture was concentrated and the residue was extracted with 0.1 M $H_3PO_4$, $H_2O$ then dried over $MgSO_4$, filtered and the filtrate concentrated. The crude material yield was quantitative and was used directly in the next step. $^1H$ NMR($DMSO_{d6}$,300MHz) $\delta$ 3.17(d,J=5Hz,2H), 4.44-4.73(m,2H), 4.86-4.94(m,1H), 5.08-5.26(m,2H), 7.16-7.24(m,4H), 7.32-7.42(m,6H).

Example 95

N-(N-Benzyloxycarbonyl-1,2,3,4-tetrahydroisoquinolyl-3-carbonyl)-R-Tryptophan-di-n-pentylamide

The product of example 94 (400 mg, 1.29 mmol), the product of example 2 (464 mg, 1.29 mmol), HOBt (176 mg, 1.3 mmol) and TEA (181 µL, 1.3 mmol) were dissolved in 10 mL methylene chloride and treated with EDCI (248 mg, 1.3 mmol) at 4° C and allowed to reach room temperature overnight. The solvents were evaporated and the residue was purified by chromatography to provide product. $R_f$ = 0.32 (18:1 methylene chloride-ethanol). MS(CI) m/e 637(m+H)$^+$, 508, 503, 478, 391, 326. $^1$H NMR(DMSO$_{d6}$,300MHz,145° C) δ 0.84-0.88(m,3H), 1.10-1.40(m,15H), 2.82(s,$H_2O$), 2.85-3.23(m,8H), 4.53(dd,J=2,15Hz,1H), 4.76-(dd,J=8,15Hz,1H), 4.87(t,J=5Hz,1H), 4.93-5.02(m,1H), 5.15-5.27(m,2H), 6.96-7.03(m,2H), 7.08-7.12(m,2H), 7.17-7.22(m,4H), 7.34-7.47(m,6H), 7.55-7.58(m,1H), 10.35(s,1H). C,H,N analysis calculated for $C_{39}H_{48}N_4O_4$: C 73.55, H 7.60, N 8.80; found: C 73.31, H 7.66, N 8.74.

## Example 96

### N-(3′-Isoquinolylcarbonyl)-R-Tryptophan-di-n-pentylamide

The product of example 95 (260 mg, 0.41 mmol) was dissolved in 25 mL of decalin and treated with 10 mg of 10% Pd/C at reflux for 5 hours. The reaction was cooled and the mixture was filtered. After concentration of the filtrate, the residue was purified by chromatography on silica gel eluted with a 4:1 to 2:1 hexane-ethylacetate step gradient to yield 121 mg, 0.24 mmol (59%). $R_f$ = 0.5 (1:1 hexane-ethylacetate). MS(CI) m/e 499 (m+H)$^+$. $^1$H NMR(CDCl$_3$,300 MHz) δ 0.77(t,J=7Hz,3H), 0.86(t,J=7Hz,3H), 0.94-1.44(m,12H), 2.86-3.10(m,3H), 3.33-3.44(m,3H), 5.47-5.54(m,1H), 7.11-7.20(m,3H), 7.32-(dd,J=1,7Hz,1H), 7.67-7.78(m,2H), 7.83(dd,J=1,7Hz,1H), 7.96(d,J=8Hz,1H), 8.03(d,J=7Hz,1H), 8.15(s,1H), 8.59(s,1H), 9.02(d,J=9Hz,1H), 9.18(s,1H). C,H,N analysis calculated for $C_{31}H_{38}N_4O_2$, $H_2O$: C 72.06, H 7.80, N 10.84; found: C 72.09, H 7.44, N 10.74.

## Example 97

### N-(6′-Quinolylcarbonyl)-R-Tryptophan-di-n-pentylamide

Quinoline-6-carboxylic acid (52 mg, 0.30 mmol), the product of example 2 (100 mg, 0.26 mmol) and TEA (37 µL, 0.3 mmol) were dissolved in 5 mL of methylene chloride and treated with EDCI (51 mg, 0.26 mmol) at room temperature. The reaction became homogeneous within 1 hour. After 12 hours, the solvents were evaporated and the residue in EtOAc was extracted with 0.1 M citric acid, 0.1 M $Na_2CO_3$, and water. The solution was dried over $MgSO_4$, filtered, and the filtrate concentrated. The residue after concentration was purified by chromatography on silica gel eluted with a 4:1 to 1:1 hexane-ethylacetate step gradient to yield 79 mg, 0.16 mmol (53%). MS(CI) m/e 499(m+H)$^+$, 370. $^1$H NMR(CDCl$_3$,300MHz) δ 0.81(t,J=7Hz,3H), 0.89(t,J=7Hz,3H), 0.97-1.46(m,12H), 2.78-2.88(m,1H), 2.98-3.08(m,2H), 3.30-3.49(m,3H), 5.45-5.53(m,1H), 7.08(d,J=2Hz,1H), 7.13(dt,J=1,7Hz,1H), 7.19(dt,J=1,7Hz,1H), 7.32-7.38(m,2H), 7.46(dd,J=4,8Hz,1H), 7.82-(d,J=8Hz,1H), 8.08-8.15(m,3H), 8.21(dd,J=1,8Hz,1H), 8.25(d,J=2Hz,1H), 8.99(dd,J=1,5Hz,1H). C,H,N analysis calculated for $C_{31}H_{38}N_4O_2$, 0.4 $H_2O$: C 73.60, H 7.73, N 11.08; found: C 73.51, H 7.66, N 10.91.

## Example 98

### N-Butyloxycarbonyl-S-Tryptophan-di-n-pentylamide

Boc-S-Tryptophan (1 g, 3.3 mmol), dipentylamine (1.6 mL, 8.2 mmol) and HOBt (446 mg, 3.3 mmol) were dissolved in 25 mL of methylene chloride and treated with BOPCl (841 mg, 3.3 mmol). After 1 day,

TEA (460 µL) and additional BOPCl (168 mg) were added. After 3 days, the solvents were evaporated and the residue in ethylacetate was extracted with 0.1 M citric acid, 0.1 M $NaHCO_3$, and water. After drying over $MgSO_4$ and concentration of the filtrate, the residue was purified by chromatography on silica gel eluted with 9:1 to 2:1 hexane-ethylacetate step gradient.

Example 99

S-Tryptophan-di-n-pentylamide hydrochloride

The product of example 98 (435 mg, 0.98 mmol) was dissolved in 3 mL of 4 N HCl in dioxane at 4°C and allowed to reach room temperature. After 2 hours, the excess reagent was evaporated and the residue was placed under high vacuum overnight. The product was used directly in the next step.

Example 100

N-(3′-Quinolylcarbonyl)-S-Tryptophan-di-n-pentylamide

Quinoline-3-carboxylic acid (69 mg, 0.4 mmol) and the product of example 99 (150 mg, 0.4 mmol) were coupled and extracted as in example 97. The concentrated residue was purified by chromatography on silica gel eluted with a 4:1 to 2:1 hexane-ethylacetate step gradient to yield 151 mg, 0.31 mmol (78%). MS-(CI) m/e 499(m+H)$^+$. $^1$H NMR($CDCl_3$,300MHz) δ 0.82(t,J=7Hz,3H), 0.90(t,J=7Hz,3H), 1.0-1.5(m,12H), 2.79-2.89(m,1H), 2.99-3.11(m,2H), 3.34(s,1H), 3.37(s,1H), 3.42-3.52(m,1H), 5.50(apparent q,J=7Hz,1H), 7.04-(d,J=2Hz,1H), 7.11-7.23(m,2H), 7.32-7.38(m,2H), 7.62(dt,J=1,8Hz,1H), 7.78-7.83(m,2H), 7.86-(dd,J=1,8Hz,1H), 8.04(s,1H), 8.16(d,J=8Hz,1H), 8.48(d,J=2Hz,1H), 9.31(d,J=2Hz,1H). C,H,N analysis calculated for $C_{31}H_{38}N_4O_2$, 0.4 $H_2O$: C 73.60, H 7.73, N 11.08; found: C 73.67, H 7.75, N 10.97.

Example 101

N-(7′-Chloro-4′-hydroxy-3′-quinolylcarbonyl)-R-Tryptophan-di-n-pentylamide

7-Chloro-4-hydroxyquinoline-3-carboxylic acid (590 mg, 2.6 mmol) and the product of example 2 (1.0 g, 2.6 mmol) were coupled in a manner similar to example 58. After 2 days, the reaction mixture was treated as in example 92 and the residue was crystallized from hot 80% aqueous ethanol to yield 668 mg, 1.2 mmol (47%). MS(FAB+) m/e 549(m+H)$^+$, 571(m+Na)$^+$, 419, 392, 364, 326. $^1$H NMR($DMSO_{d6}$,300MHz) δ 0.73(t,J=7Hz,3H), 0.83(t,J=7Hz,3H), 0.90-1.17(m,7H), 1.18-1.4(m,5H), 2.92-3.09(m,4H), 3.12-3.33(m,2H), 5.22-5.31(m,1H), 6.98(dt,J=1,8Hz,1H), 7.04-7.09(m,2H), 7.33(d,J=8Hz,1H), 7.53(dd,J=2,8Hz,1H), 7.68-(d,J=7Hz,1H), 7.75(d,J=2Hz,1H), 8.27(d,J=8Hz,1H), 8.81(s,1H), 10.38(d,J=8Hz,1H), 10.85(d,J=2Hz,1H), 12.74(s,1H). C,H,N analysis calculated for $C_{31}H_{37}ClN_4O_3$: C 67.80, H 6.79, N 10.20; found: C 68.13, H 6.86, N 10.27.

Example 102

N-(3′-Phenanthrylcarbonyl)-R-Tryptophan-di-n-pentylamide

Phenanthrene-3-carboxylic acid (100 mg, 0.45 mmol) and the product of example 2 (172 mg, 0.45 mmol) were coupled and treated as in example 100 after 3 days reaction time. The crude residue was purified by chromatography on silica gel eluted with a 9:1 to 2:1 hexane-ethylacetate step gradient to yield 92 mg, 0.18 mmol (40%). MS(CI) m/e 548$(m+H)^+$, 418, 326. $^1H$ NMR($CDCl_3$,300MHz) δ 0.82(t,J=7Hz,3H), 0.88(t,J=7Hz,3H), 1.0-1.08(m,2H), 1.10-1.36(m,8H), 1.38-1.48(m,2H), 2.82-2.93(m,1H), 3.0-3.11(m,2H), 3.38-3.52(m,3H), 5.52-5.54(m,1H), 7.10(d,J=2Hz,1H), 7.12-7.23(m,2H), 7.34-7.41(m,2H), 7.61-7.98(m,8H), 8.05-(m,1H), 8.72(d,J=8Hz,1H), 9.15(s,1H). C,H,N analysis calculated for $C_{36}H_{41}N_3O_2$: C 78.94, H 7.55, N 7.67; found: C 78.69, H 7.67, N 7.51.

## Example 103

### N-(5′-Nitro-2′-quinolylcarbonyl)-R-Tryptophan-di-n-pentylamide

5-Nitroquinoline-2-carboxylic acid (172 mg, 1.0 mmol) and the product of example 2 (380 mg, 1.0 mmol) were coupled as in example 100. After evaporation of the volatiles, the residue was purified by chromatography on silica gel eluted with a 4:1 to 2:1 hexane-ethylacetate step gradient to yield 387 mg, 0.73 mmol (73%). $R_f$= 0.7 (1:1 hexane-ethylacetate). MS(CI) m/e 544$(m+H)^+$, 414, 344, 326. $^1H$ NMR-($CDCl_3$,300MHz) δ 0.78(t,J=7Hz,3H), 0.88(t,J=7Hz,3H), 0.95-1.48(m,12H), 2.81-2.91(m,1H), 2.95-3.07-(m,2H), 3.38-3.49(m,3H), 5.46-5.53(m,1H), 7.11(d,J=2Hz,1H), 7.12-7.22(m,2H), 7.34(dd,J=1,7Hz,1H), 7.8-7.89(m,2H), 8.07(s,1H), 8.44-8.52(m,3H), 8.93(d,J=8Hz,1H), 9.18(dd,J=1,8Hz,1H). C,H,N analysis calculated for $C_{31}H_{37}N_5O_4$, 0.5 $H_2O$: C 67.37, H 6.93, N 12.67; found: C 67.48, H 6.82, N 12.57.

## Example 104

### N-(4′-Hydroxy-2′-quinolylcarbonyl)-R-Tryptophan-di-n-pentylamide

4-Hydroxyquinoline-2-carboxylic acid (284 mg, 1.5 mmol), the product of example 2 (500 mg, 1.3 mmol) and TEA (209 µL, 1.5 mmol) were dissolved in 10 mL DMF and treated with EDCI (287 mg, 1.5 mmol). After 1 day, additional EDCI (287 mg) and TEA (209 µL) as well as HOBt (202 mg) were added. The solvents were evaporated after 3 days and the residue in ethylacetate was extracted as in example 100. The crude product was purified by chromatography on silica gel eluted with a 20:1 to 9:1 methylene chloride-ethanol step gradient to yield 74 mg, 0.014 mmol (1.1%). MS(FAB+) m/e 515$(m+H)^+$, 330. $^1H$ NMR($DMSO_{d6}$,300MHz) δ 0.74-0.91(m,6H), 1.12-1.62(m,12H), 3.08-3.35(m,6H), 5.0-5.08(m,1H), 6.88(s,1H), 6.98(t,J=7Hz,1H), 7.06(t,J=7Hz,1H), 7.21(s,1H), 7.34(d,J=10Hz,2H), 7.62-7.69(m,2H), 7.90(d,J=8Hz,1H), 8.06(d,J=8Hz,1H), 9.42(d,J=7Hz,1H), 10.88(s,1H), 11.78(s,1H). C,H,N analysis calculated for $C_{31}H_{38}N_4O_3$, 0.5 $H_2O$: C 71.10, H 7.51, N 10.70; found: C 71.37, H 7.62, N 10.14.

## Example 105

### N-(5′-Methyl-2′-indolylcarbonyl)-R-Tryptophan-di-n-pentylamide

5-Methylindole-2-carboxylic acid (262 mg, 1.5 mmol), the product of example 2 (500 mg, 1.31 mmol) and TEA (209 µL, 1.5 mmol) were dissolved in 15 mL methylene chloride and treated with EDCI (287 mg, 1.5 mmol) at room temperature overnight. The solvents were evaporated and the residue was extracted as

in example 100. The crude product was purified by chromatography on silica gel eluted with a 3:1 to 1:1 hexane-ethylacetate step gradient to yield 550 mg, 1.1 mmol (84%). MS(CI) m/e 501(m+H)$^+$, 371, 344, 326. $^1$H NMR($CDCl_3$,300MHz) δ 0.77(t,J=7Hz,3H), 0.87(t,J=7Hz,3H), 0.91-1.45(m,13H), 2.43(s,3H), 2.78-2.88(m,1H), 2.93-3.09(m,2H), 3.31(s,1H), 3.33(s,1H), 3.34-3.45(m,1H), 5.44(apparent q,J=8Hz,1H), 6.86-(d,J=1Hz,1H), 7.04(d,J=2Hz,1H), 7.06-7.14(m,2H), 7.18(dt,J=1,6Hz,1H), 7.29(d,J=8Hz,2H), 7.33-(d,J=7Hz,1H), 7.42(d,J<1Hz,1H), 7.76(d,J=8Hz,1H), 8.02(s,1H), 9.38(s,1H). C,H,N analysis calculated for $C_{31}H_{40}N_4O_2$: C 74.36, H 8.05, N 11.19; found: C 74.07, H 8.05, N 10.98.

## Example 106

### N-(5′-Fluoro-2′-indolylcarbonyl)-R-Tryptophan-di-n-pentylamide

5-Fluoroindole-2-carboxylic acid (269 mg, 1.5 mmol), the product of example 2 (500 mg, 1.31 mmol) were coupled as in example 58 with the following modifications: 10% more EDCI and TEA were added at 6 hours, and 50% more EDCI and TEA were added at 1 day. After 2 days, the reaction mixture was processed as in example 100 to yield 546 mg, 1.08 mmol (82%). MS(CI) m/e 505(m+H)$^+$, 348, 326. $^1$H NMR($CDCl_3$,300MHz) δ 0.75(t,J=7Hz,3H), 0.86(t,J=7Hz,3H), 0.92-1.47(m,13H), 2.82-2.92(m,1H), 2.96-3.11-(m,2H), 3.32(s,1H), 3.34(s,1H), 3.38-3.46(m,1H), 5.44(apparent q,J=7Hz,1H), 6.90(d,J=2Hz,1H), 6.98-(dt,J=2,12Hz,1H), 7.05(d,J=2Hz,1H), 7.08-7.35(m,4H), 7.54(d,J=8Hz,1H), 7.73(d,J=7Hz,1H), 8.06(s,1H), 9.76(s,1H). C,H,N analysis calculated for $C_{30}H_{37}FN_4O_2$, $H_2O$, 0.3 EtOAc: C 68.25, H 7.60, N 10.20; found: C 68.13, H 7.25, N 10.11.

## Example 107

### N-(p-Chlorophenylaminothiocarbonyl)-S-Tryptophandi-n-pentylamide

4-Chlorophenylisothiocyanate (1 equiv) was added to the product of example 99 (100 mg, 0.26 mmol) and TEA (1.1 equiv) in THF and left stirring overnight. The reaction mixture was poured into ethylacetate and extracted with 0.1% citric acid, 0.1 M $NaHCO_3$ and water. The organic layer was then dried over $MgSO_4$, filtered and diluted with hexanes until slightly cloudy then left overnight to precipitate. The resulting solid was collected and rinsed with fresh hexanes to yield 89 mg, 0.173 mmol (67%). mp= 140-141 °C. MS(CI) m/e 513(m+H)$^+$, 356, 344, 329, 326. $^1$H NMR($CDCl_3$,300MHz) δ 0.82-0.88(m,6H), 1.02-1.48(m,11H), 1.46-1.54(m,1H), 2.80-3.10(m,3H), 3.26-3.43(m,3H), 5.77-5.83(m,1H), 7.00(d,J=2Hz,1H), 7.03(d,J=8Hz,2H), 7.12(dt,J=1,7Hz,1H), 7.19(dt,J=1,7Hz,1H), 7.23-7.25(m,2H), 7.30(s,1H), 7.33(dd,J=<1,8Hz,1H), 7.81-(d,J=8Hz,1H), 7.93(s,1H), 8.02(s,1H). C,H,N analysis calculated for $C_{28}H_{37}ClN_4OS$, 0.2 $H_2O$: C 65.08, H 7.30, N 10.84; found: C 65.14, H 7.18, N 10.79.

## Example 108

### N-(p-Chlorophenylaminothiocarbonyl)-R-Tryptophan-di-n-pentylamide

The product of example 2 (100 mg, 0.26 mmol) was reacted with 4-chlorophenylisothiocyanate as in example 107 to yield 62 mg, 0.12 mmol (46%). mp= 138-140 °C. MS(CI) m/e 513(m+H)$^+$, 356, 344, 326. $^1$H NMR($CDCl_3$,300MHz) δ 0.82(t,J=7Hz,3H), 0.83(t,J=7Hz,3H), 1.02-1.48(m,11H), 1.46-1.54(m,1H), 2.80-3.10(m,3H), 3.26-3.43(m,3H), 5.77-5.83(m,1H), 7.01(d,J=2Hz,1H), 7.07(d,J=8Hz,2H), 7.12(dt,J=1,7Hz,1H), 7.19(dt,J=1,7Hz,1H), 7.23-7.25(m,2H), 7.33(d,J=8Hz,1H), 7.46(bd,J=5Hz,1H), 7.81(d,J=8Hz,1H), 8.02-

(s,1H), 8.09(s,1H). C,H,N analysis calculated for $C_{28}H_{37}ClN_4OS$, 0.2 $H_2O$: C 65.08, H 7.30, N 10.84; found: C 65.09, H 7.24, N 10.75.

Example 109

N-(3′-Methylphenylaminocarbonyl)-S-Tryptophan-di-n-pentylamide

The product of example 99 (77 mg, 0.23 mmol) was reacted with 3-methylphenylisocyanate as in example 107 to yield 47 mg, 0.10 mmol (47%). mp= 158-159° C. MS(CI) m/e 477(m+H)$^+$, 344, 329. $^1$H NMR($CDCl_3$,300MHz) $\delta$ 0.78(apparent q,J=7Hz,6H), 0.95-1.38(m,12H), 2.32(s,3H), 2.98-3.12(m,3H), 3.28-(d,J=7Hz,2H), 3.33-3.40(m,1H), 5.31(apparent q,J=7Hz,1H), 6.82-6.88(m,2H), 7.02(d,J=2Hz,1H), 7.05-7.18-(m,4H), 7.28-7.32(m,2H), 7.71(s,1H), 7.75(d,J=7Hz,1H), 8.0(d,J=1Hz,1H). C,H,N analysis calculated for $C_{29}H_{40}N_4O_2$: C 73.06, H 8.46, N 11.76; found: C 73.36, H 8.47, N 11.73.

Example 110

N-(3′-Methylphenylaminocarbonyl)-R-Tryptophan-di-n-pentylamide

The product of example 2 (200 mg, 0.53 mmol) was reacted with 3-methylphenylisocyanate as in example 107 to yield 159 mg, 0.33 mmol (63%). mp= 155-6° C. MS(CI) m/e 477(m+H)$^+$, 344, 329. $^1$H NMR($CDCl_3$,300MHz) $\delta$ 0.78(apparent q,J=7Hz,6H), 0.95-1.38(m,12H), 2.32(s,3H), 2.98-3.12(m,3H), 3.28-(d,J=7Hz,2H), 3.33-3.40(m,1H), 5.31(apparent q,J=7Hz,1H), 6.82-6.86(m,2H), 7.02(d,J=2Hz,1H), 7.05-7.18-(m,4H), 7.28-7.32(m,2H), 7.69(s,1H), 7.75(d,J=7Hz,1H), 8.0(d,J=1Hz,1H). C,H,N analysis calculated for $C_{29}H_{40}N_4O_2$, 0.2 H2O: C 72.53, H 8.48, N 11.67; found: C 72.58, H 8.49, N 11.54.

Example 111

N-(4′-Methylphenylaminocarbonyl)-S-Tryptophan-di-n-pentylamide

The product of example 99 (100 mg, 0.26 mmol) was reacted with p-tolylisocyanate as in example 107 to yield 72 mg, 0.15 mmol (58%). mp= 140-1° C. MS(CI) m/e 477(m+H)$^+$, 370, 344, 326, 320. $^1$H NMR-($CDCl_3$,300MHz) $\delta$ 0.78(t,J=7Hz,3H), 0.82(t,J=7Hz,3H), 0.95-1.48(m,12H), 2.30(s,3H), 2.96-3.09(m,3H), 3.26(d,J=7Hz,2H), 3.31-3.40(m,1H), 5.28(apparent q,J=7Hz,1H), 6.72(d,J=8Hz,1H), 7.02(d,J=2Hz,1H), 7.04-7.17(m,6H),7.21-7.32(m,2H), 7.58(s,1H), 7.73(d,J=7Hz,1H), 8.01(s,1H). C,H,N analysis calculated for $C_{29}H_{40}N_4O_2$, 0.2 $H_2O$: C 72.53, H 8.48, N 11.67; found: C 72.42, H 8.38, N 11.63.

Example 112

N-(4′-Methylphenylaminocarbonyl)-R-Tryptophan-di-n-pentylamide

The product of example 2 (100 mg, 0.26 mmol) was reacted with p-tolylisocyanate as in example 107 to yield 91 mg, 0.19 mmol (73%). mp= 136-8° C. MS(CI) m/e 477(m+H)$^+$, 370, 344, 326, 320. $^1$H NMR-

($CDCl_3$,300MHz) δ 0.78(t,J=7Hz,3H), 0.82(t,J=7Hz,3H), 0.95-1.48(m,12H), 2.30(s,3H), 2.96-3.09(m,3H), 3.26(d,J=7Hz,2H), 3.31-3.40(m,1H), 5.28(apparent q,J=7Hz,1H), 6.63(d,J=8Hz,1H), 7.02(d,J=2Hz,1H), 7.04-7.17(m,6H), 7.21(m,2H), 7.51(s,1H), 7.73(d,J=7Hz,1H), 8.01(s,1H). C,H,N analysis calculated for $C_{29}H_{40}N_4O_2$, 0.2 $H_2O$: C 72.53, H 8.48, N 11.67; found: C 72.41, H 8.45, N 11.61.

## Example 113

### N-(4′-Chlorophenylaminocarbonyl)-S-Tryptophan-di-n-pentylamide

The product of example 99 (80 mg, 0.21 mmol) was reacted with 4-chlorophenylisocyanate as in example 107 to yield 89 mg, 0.18 mmol (85%). mp= 87-8° C. MS(CI) m/e 497(m+H)$^+$, 370, 344, 326. $^1$H NMR($CDCl_3$,300MHz) δ 0.76-0.83(m,6H), 0.96-1.42(m,12H), 2.99-3.12(m,3H), 3.27(d,J=7Hz,2H), 3.32-3.39-(m,1H), 5.28(apparent q,J=7Hz,1H), 6.95(d,J=8Hz,1H), 7.02(d,J=2Hz,1H), 7.07(dt,J=1,7Hz,1H), 7.13-7.20-(m,3H), 7.25-7.32(m,4H), 7.72(d,J=7Hz,1H), 7.97(s,1H), 8.03(s,1H). C,H,N analysis calculated for $C_{28}H_{37}ClN_4O_2$, 0.3 $H_2O$: C 66.93, H 7.54, N 11.15; found: C 66.80, H 7.33, N 11.12.

## Example 114

### N(4′-Chlorophenylaminocarbonyl)-R-Tryptophan-dipentylamide

The product of example 2 (100 mg, 0.30 mmol) was reacted with 4-chlorophenylisocyanate as in example 107 to yield product. mp= 73-75° C. MS(CI) m/e 497(m+H)$^+$, 344, 326. $^1$H NMR($CDCl_3$,300MHz) δ 0.76-0.83(m,6H), 0.96-1.42(m,12H), 2.99-3.12(m,3H), 3.27(d,J=7Hz,2H), 3.32-3.39(m,1H), 5.28(apparent q,J=7Hz,1H), 6.98(d,J=8Hz,1H), 7.02(d,J=2Hz,1H), 7.07(dt,J=1,8Hz,1H), 7.13-7.20(m,3H), 7.25-7.32-(m,4H), 7.72(d,J=7Hz,1H), 7.97(s,1H), 8.03(s,1H). C,H,N analysis calculated for $C_{28}H_{37}ClN_4O_2$, 0.3 $H_2O$: C 66.93, H 7.54, N 11.15; found: C 66.80, H 7.24, N 11.12.

## Example 115

### N-(3′-Chlorophenylaminocarbonyl)-S-Tryptophan-di-n-pentylamide

The product of example 99 (77 mg, 0.21 mmol) was reacted with 3-chlorophenylisocyanate as in example 107 to yield 61 mg, 0.12 mmol (58%). mp= 125-127° C. MS(CI) m/e 497(m+H)$^+$, 463, 370, 344. $^1$H NMR($CDCl_3$,300MHz) δ 0.79(t,J=7Hz,6H), 0.98-1.12(m,9H), 1.18-1.38(m,3H), 2.99-3.13(m,3H), 3.30-(d,J=7Hz,2H), 3.33-3.41(m,1H), 5.31(apparent q,J=7Hz,1H), 6.96(m,1H), 7.02(d,J=2Hz,1H), 7.05-7.18-(m,4H), 7.22-7.26(m,1H), 7.32(d,J=8Hz,1H), 7.49(t,J=1Hz,1H), 7.73(d,J=8Hz,1H), 8.03(d,J=1Hz,1H), 8.18-(s,1H). C,H,N analysis calculated for $C_{28}H_{37}ClN_4O_2$: C 67.65, H 7.50, N 11.27; found: C 67.62, H 7.57, N 11.16.

## Example 116

### N-(3′-Chlorophenylaminocarbonyl)-R-Tryptophan-di-n-pentylamide

The product of example 2 (100 mg, 0.26 mmol) was reacted with 3-chlorophenylisocyanate as in example 107 to yield 75 mg, 0.15 mmol (71%). mp= 118-121 °C. MS(CI) m/e 497(m+H)$^+$, 370, 344, 326. $^1$H NMR($CDCl_3$,300MHz) δ 0.79(t,J=7Hz,6H), 0.98-1.12(m,9H), 1.18-1.38(m,3H), 2.99-3.13(m,3H), 3.30-(d,J=7Hz,2H), 3.33-3.41(m,1H), 5.31(apparent q,J=7Hz,1H), 6.93-6.98(m,1H), 7.02(d,J=2Hz,1H), 7.05-7.09-(m,2H), 7.12-7.19(m,2H), 7.23-7.26(m,1H), 7.32(d,J=8Hz,1H), 7.49(t,J=1Hz,1H), 7.73(d,J=8Hz,1H), 8.03-(s,1H), 8.10(s,1H). C,H,N analysis calculated for $C_{28}H_{37}ClN_4O_2$, 0.3 $H_2O$: C 66.93, H 7.54, N 11.15; found: C 66.99, H 7.40, N 11.12.

## Example 117

### N-(3′-Quinolylcarbonyl)-R-Tryptophan-di-n-pentylamide mesylate salt

The product of example 21 (1.0 g, 2.0 mmol) was dissolved in 15 mL of isopropanol and treated with methanesulfonic acid (269μL, 4.0 mmol). A yellow-orange color developed immediately. Hexane was added until cloudiness persisted in the solution. As an oil began to settle the mixture was gently warmed on a steam bath and a solid formed. The mixture was cooled and the solid collected by filtration and washed with fresh hexane. The solid was dried in vacuo to yield 1.06 g, 1.78 mmol (89%). mp= 163-5 °C. $[\alpha]_D$= -24.0° (c=1.15, MeOH). MS(CI) m/e 499(m+H)$^+$, (free base). $^1$H NMR($DMSO_{d6}$,300MHz) δ 0.76-(t,J=7Hz,3H), 0.83(t,J=7Hz,3H), 1.05-1.3(m,9H), 1.33-1.55(m,3H), 2.36(s,3H), 3.06-3.20(m,3H), 3.23-3.32-(m,3H), 5.20(apparent q,J=7Hz,1H0, 6.99(dt,J<1Hz,7Hz,1H), 7.06(dt,J<1,7Hz,1H), 7.20(d,J=2Hz,1H), 7.32-(d,J=7Hz,1H), 7.67(d,=7Hz,1H), 7.81(dt,J<1,8Hz,1H), 7.99(dt,J<1,7Hz,1H), 8.16(d,J=8Hz,1H), 8.20-(dd,J<1,8Hz,1H), 9.13(d,J=1Hz,1H), 9.31(d,J=8Hz,1H), 9.40(d,J=2Hz,1H), 10.88(d,J=1Hz,1H). C,H,N analysis calculated for $C_{32}H_{42}N_4O_5S$, 0.5 $H_2O$: C 63.66, H 7.18, N 9.28; found: C 63.57, H 7.19, N 9.22.

The compounds of Formula I antagonize CCK which makes the compounds useful in the treatment and prevention of disease states wherein CCK or gastrin may be involved, for example, gastrointestinal disorders such as irritable bowel syndrome, ulcers, excess pancreatic or gastric secretion, acute pancreatitis, motility disorders, pain (potentiation of opiate analgesia), central nervous system disorders caused by CCK's interaction with dopamine such as neuroleptic disorders, tardive dyskinesia, Parkinson's disease, psychosis or Gilles de la Tourette Syndrome, disorders of appetite regulatory systems, Zollinger-Ellison syndrome, and central G cell hyperplasia.

### In Vitro Test Methods and Results

The ability of the compounds of Formula I to interact with CCK receptors and to antagonize CCK can be demonstrated in vitro using the following protocols.

$CCK_8$ [Asp-Tyr($SO_3H$)-Met-Gly-Trp Met-Asp-Phe-$NH_2$] was purchased from Beckman Instruments (Palo Alto, CA) and Peptide International (Louisville, KY). Chymostatin, L-Try-Gly, puromycin, bestatin, EGTA, HEPES and BSA were purchased from Sigma Chemical Co. (St. Louis, MO). [$^{125}$I]BH $CCK_8$ (specific activity, 2200 Ci/mmol) and Aquasol-2 scintillation cocktail were obtained from New England Nuclear (Boston, MA) Male guinea pigs, 250 to 325 g, were obtained from Scientific Small Animal Laboratory and Farm (Arlington Heights, IL). Collagenase, 300 units per mg, was purchased from Worthington (Frehold, N.J.)

### Protocol For Radioligand Binding Experiments

#### 1. Guinea Pig Cerebral Cortical and Pancreatic Membrane Preparations

Cortical and pancreatic membrances were prepared as described (Lin and Miller; J. Pharmacol. EXP. Ther. 232, 775-780, 1985). In brief, cortex and pancreas were removed and rinsed with ice-cold saline. Visible fat and connective tissues were removed from the pancreas. Tissues were weighed and homogenized separately in approximately 25 mL of ice-cold 50 mM Tris-HCl buffer, pH 7.4 at 4 °C, with a

Brinkman Polytron for 30 sec, setting 7. The homogenates were centrifuged for 10 min at 1075 x g and pellets were discarded. the supernatants were saved and centrifuged at 38,730 x g for 20 min. The resultant pellets were rehomogenized in 25 mL of 50 mM Tris-HCl buffer with a Teflon-glass homogenizer, 5 up and down strokes. The homogenates were centrifuged again at 38,730 x g for 20 min. Pellets were then resuspended in 20 mM HEPES, containing 1 mM EGTA, 118 mM NaCl, 4.7 mM KCl, 5 mM $MgCl_2$, 100 uM bestatin, 3 uM phosphoramidon, pH 7.4 at 22°C, with a Teflon-glass homogenizer, 15 up and down strokes. Resuspension volume for the cortex was 15-18 mL per gm of original wet weight and 60 mL per gm for the pancreas.

2. Incubation Conditions

[$^{125}$I]Bolton-Hunter $CCK_8$, and test compounds were diluted with HEPES-EGTA-salt buffer (see above) containing 0.5% bovine serum albumin (BSA). To 1 mL Skatron polystyrene tubes were added 25 uL of test compounds, 25 uL of [$^{125}$I]BH-$CCK_8$ and 200 uL of membrane suspension. the final BSA concentration was 0.1%. The cortical tissues were incubated at 30°C for 150 min and pancreatic tissues were incubated at 37°C for 30 min. Incubations were terminated by filtration using Skatron Cell Harvester and SS32 microfiber filter mats The specific binding of [$^{125}$I]BH-$CCK_8$, defined as the difference between binding in the absence and presence of 1 uM $CCK_8$, was 85-90% of total binding in cortex and 90-95% in pancreas. $IC_{50}$'s were determined from the Hill analysis. The results of these binding assays are shown in Table 1.

Protocol for Amylase

This assay was performed using the modified protocol of Lin et al., J. Pharmacol Exp. Ther. 236, 729-734, 1986.

1. Guinea Pig Acini Preparation

Guinea pig acini were prepared by the method of Bruzzone et al. (Biochem. J. 226, 621-624, 1985) as follows. Pancreas was dissected out and connective tissues and blood vessels were removed. The pancreas was cut into small pieces (2mm) by a scissor and placed in a 15 mL conical plastic tube containing 2 5 mL of Krebs-Ringer HEPES (KRH) buffer plus 400 units per mL of collagense. The composition of the KRH buffer was: HEPES, 12.5 mM: NaCl, 118 mM; KCl, 4.8 mM; $CaCl_2$, 1 mM; $KH_2PO_4$, 1.2 mM; $MgSO_4$, 1.2 mM; $NaHCO_3$, 5 mM; glucose, 10 mM, pH 7.4. The buffer was supplemented with 1% MEM vitamins, 1% MEM amino acids and 0.001% aprotinin. The tube was shaken by hand until the suspension appeared homogeneous, usually 5 to 6 min. 5 mL of the KRH, without collagenase and with 0.1% BSA, were added and the tube was centrifuged at 50 x g for 35 sec. The supernatant was discarded and 6 mL of the KRH were added to the cell pellet. Cells were triturated by a glass pipet and centrifuged at 50 x g for 35 sec. This wash procedure was repeated once. The cell pellet from the last centrifugation step was then resuspended in 15 mL of KRH containing 0.1% BSA. The contents were filtered through a dual nylon mesh, size 275 and 75 um. The filtrate, containing the acini, were centrifuged at 50 x g for 3 min. The acini were then resuspended in 5 mL of KRH-BSA buffer for 30 min at 37°C, under 100% $O_2$, with a change of fresh buffer at 15 min.

2. Amylase Assay

After the 30 min incubation time, the acini were resuspended in 100 volumes of KRH-BSA buffer, containing 3 uM phosphoramidon and 100 M bestatin. While stirring, 400 uL of acini were added to 1.5 mL microcentrifuge tubes containing 50 uL of $CCK_8$, buffer, or test compounds. The final assay volume was 500 uL. Tubes were vortexed and placed in a 37°C waterbath, under 100% $O_2$, for 30 min. Afterward, tubes were centrifuged at 10,000 g for 1 min. Amylase activity in the supernatant and the cell pellet were separately determined after appropriate dilutions in 0.1% Triton X-100, 10 mM $NaH_2PO_4$, pH 7.4 by Abbott Amylase A-gent test using the Abbott Bichromatic Analyzer 200. The reference concentration for $CCK_8$ in determining the $IC_{50}$'s of the compounds of Formula I was $3x10^{-10}$M. The results of this assay are shown in Table 2.

TABLE 1

| Compound of Example | $IC_{50}$ (nM) | |
|---|---|---|
| | [125I]-BH-$CCK_8$ Pancreas | [125I]-BH-$CCK_8$ Cortex |
| 3 | 51 | 8,000 |
| 4 | 330 | >10,000 |
| 7 | 350 | >10,000 |
| 8 | 420 | >10,000 |
| 18 | 680 | 10,000 |
| 19 | 1,000 | >10,000 |
| 21 | 14 | 15,000 |
| 25 | 150 | 10,000 |
| 28 | 280 | 10,000 |
| 29 | 88 | 9,200 |
| 31 | 300 | >10,000 |
| 32 | 120 | >10,000 |
| 33 | 530 | 10,000 |
| 36 | 380 | >10,000 |
| 39 | 110 | >10,000 |
| 40 | 1,000 | 10,000 |
| 45 | 1,500 | >10,000 |
| 50 | 1,200 | >10,000 |
| 53 | 67 | >10,000 |
| 57 | 750 | >10,000 |
| 63 | 62 | >10,000 |
| 64 | 330 | 10,000 |
| 69 | 10 | 4,100 |
| 78 | 370 | >10,000 |
| 84 | 2.6 | <10,000 |
| 90 | 230 | >10,000 |
| 91 | 5 | 4,200 |
| 96 | 630 | >10,000 |
| 97 | 180 | >10,000 |
| 100 | 670 | 6,300 |
| 105 | 620 | >10,000 |
| 106 | 82 | 10,000 |
| 117 | 17 | 13,000 |

TABLE 2

| Compound of Example | $IC_{50}$ (nM) Inhibition of Amylase Release |
|---|---|
| 3 | 670 |
| 7 | <30,000 |
| 8 | <10,000 |
| 18 | <30,000 |
| 19 | <10,000 |
| 21 | 42 |
| 25 | <10,000 |
| 28 | <10,000 |
| 29 | <10,000 |
| 31 | <100,000 |
| 32 | <30,000 |
| 33 | <30,000 |
| 36 | <10,000 |
| 39 | <10,000 |
| 40 | <100,000 |
| 46 | <10,000 |
| 54 | <100,000 |
| 93 | 100,000 |
| 96 | <100,000 |
| 97 | <100,000 |
| 100 | <100,000 |

The results of the assays indicate that the compounds of the invention inhibit specific [$^{125}$I]-BH-CCK-8 receptor binding in the concentration range of $10^{-9}$ to $10^{-6}$ M and that the compounds antagonize the actions of CCK..

In Vivo Test Methods and Results

The ability of the compounds of Formula I to interact with CCK receptors and to antagonize CCK in vivo can be dmonstrated using the following protocols

Inhibition of CCK Induced Gastric Emptying

Three fasted mice are dosed (p.o.) with the test compound. $CCK_8$ (80 ug/kg s.c.) is administered with 60 minutes and charcoal meal (0.1 ml of 10% suspension) is given orally 5 minutes later. The animals are sacrificed within an additional 5 minutes.

Gastric emptying, defined as the presence of charcoal within the intestine beyond the pyloric sphincter, is inhibited by $CCK_8$. Gastric emptying observed in more than one mouse indicates antagonism of $CCK_8$.

Measurement of Plasma Insulin Level Following Treatment with $CCK_8$ and a Compound of Formula I

The ability of the compounds of Formula I to antagonize CCK induced hyperinsulinemia can be demonstrated in vivo using the following protocol.

Male mice, 20 30 g, are used in all experiments. The animals are fed with laboratory lab chow and water ad libitum. The compound of Formula I (1-100 mg/kg in 0.2 ml of 0.9% saline) was administered i.p. Ten minutes later $CCK_8$ (0.2 to 200 nmole/kg in 0 2 ml of 0.9% saline) or saline is injected into the tail vein. Two minutes later the animals are sacrificed and blood is collected into 1.5 ml heparinized polypropylene tubes. The tubes are centrifuged at 10,000 x g for 2 minutes. Insulin levels are determined in the supernatant (plasma) by an RIA method using kits from Radioassy Systems Laboratory (Carson, CA ) or

Novo Biolabs (MA.).

Antagonism of CCK Mediated Behavioral Effect in Mice with Compounds of Formula I

Male Swiss CD-1 mice (Charles River) (22-27 g) were provided with ample food (Purina Lab (show) and water until the time of their injection with the test compound.

ICV injections were given by a free-hand method similar to that previously described (Haley, and McCormick, Br. J. Pharmacol. Chemother. 12 12-15 (1957)). The animals were placed on a slightly elevated metal grid and restrained by the thumb and forefinger at the level of the shoulders, thus immobilizing their heads. Injections were made with a 30 gauge needle with a "stop" consisting of a piece of tygon tubing to limit penetration of the needle to about 4.5 mm below the surface of the skin. The needle was inserted perpendicular to the skull at a midline point equidistant from thje eye and an equal distance posterior from the level of the eyes such that the injection site and the two eyes form an equilateral triangle. The injection volme (5 ul) was expelled smoothly over a period of approximately 1 second.

Immediately after the injections, the mice were placed in their cages and allowed a 15 minute recovery period prior to the beginning of the behavioral observations.

For the behavioral observations, the mice were placed in clear plastic cages. Each cage measured 19 x 26 x 15 centimeters and contained a 60-tube polypropylene test tube rack (NALGENE #5970-0020) placed on end in the center of the cage to enhance exploratory activity. Observations were made evcery 30 seconds for a period of 30 minutes. Behavior was compared between drug and $CCK_8$ treated mice; $CCK_8$ treated mice; a;nd mice treated with an equal volume of carrier (usually 0.9% saline or 5% dimethylsulfoxide in water). Locomotion as reported here consisted of either floor locomotion or active climbing on the rack. Differences among groups were analyzed by Newman-Kewels analysis and a probability level of $p<0.05$ was accepted as significant. Each group tested consisted of 10 animals. The results of this test indicate that compounds of Formula I are antagonists of CCK in vivo. Minimally effective doses (MED) are defined as that dose at which a statistically significant reversal of CCK-induced inactivity was observed when the test compound of Formula I and $CCK_8$ were coadministered.

| Compound of Example | Dose of $CCK_8$ | MED |
|---|---|---|
| 21 | 3 nmol | 0.3 nmol |

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptonate, glycerophosphate, hemisulfate, heptonate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

The pharmaceutically acceptable salts of the present invention can be synthesized from the compounds of Formula I which contain a basic or acidic moiety by conventional chemical methods. Generally, the salts are prepared by reacting the free base or acid with stoichiometric amounts or with an excess of the desired salt forming inorganic or organic acid or base in a suitable solvent or various combinations of solvents.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

The pharmaceutically acceptable salts of the acid of Formula I are also readily prepared by conventional procedures such as treating an acid of Formula I with an appropriate amount of a base, such as an alkali or alkaline earth metal hydroxide e.g. sodium, potassium, lithium, calcium, or magnesium, or an

organic base such as an amine, e.g., dibenzylethylenediamine, trimethylamine, piperidine, pyrrolidine, benzylamine nd the like, or a quaternary ammonium hydroxide such as tetramethylammonium hydroxide and the like.

When a compound of Formula I is used as an antagonist of CCK or gastrin in a human subject, the total daily dose administered in single or divided doses may be in amounts, for example, from 0.001 to 1000 mg a day and more usually 1 to 1000 mg. Dosage unit compositions may contain such amounts of submultiples thereof to make up the daily dose.

The amount of active ingredient that may be combined with the carrier materials to produce a single dosage form will vary depending upon the host treated, the particular treatment and the particular mode of administration.

It will be understood, however, that the specific dose level for any particular patient will depend upon a variety of factors including the activity of the specific compound employed, the age, body weight, general health, sex, diet, time of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable prepartion may also be a sterile injectable solution or suspension in a nontoxic parenterally acceptable diluent or solvent, for example, as a solution in 1,3 butanediol. Among the acceptable vehicles and solvents that may be employed are water, Ringer's solution, and isotonic sodium chloride solution. In additon, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsion, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The present agents can also be administered in the form of liposomes. As is known in the art, liposomes are generally derived from phospholipids or other lipid substances. Liposomes are formed by mono-or multi-lamellar hydrated liquid crystals that are dispersed in an aqueous medium. Any non-toxic, physiologically acceptable and metabolizable lipid capable of forming liposomes can be used. The present compositons in liposome form can contain, in addition to the compounds of the present invention, stabilizers, preservatives, excipients and the like. The preferred lipids are the phospholipids and the phophatidyl cholines (lecithins), both natural and synthetic.

Methods to form liposomes are known in the art. See, for example, Prescott, ed., Methods in Cell Biology, Vol. XIV, Academic Press, New York, N.Y pp. 33- (1976).

The foregoing is merely illustrative of the invention and is not intended to limit the invention to the disclosed compounds. Variations and changes which are obvious to one skilled in the art are intended to be within the scope and nature of the invention which are defined in the appended claims

## Claims

1. A compound of the formula:

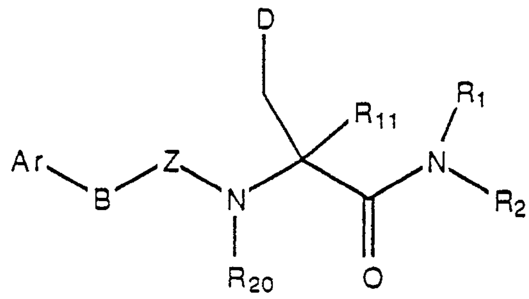

wherein
$R_1$ and $R_2$ are independently selected from
i) hydrogen,
ii) loweralkyl,
iii) cycloalkyl,
iv) loweralkenyl,
v) adamantyl,
vi) aryl,
vii) substituted aryl,
viii) heterocyclic group,
ix) substituted alkyl,
x) substituted amide,
xi) functionalized carbonyl, and
xii) nitrogen containing ring wherein $R_1$, $R_2$ and the adjacent nitrogen atom form a ring;
$R_{11}$ is
i) hydrogen,
ii) loweralkyl, or
iii) loweralkenyl;
$R_{20}$ is
i) hydrogen,
ii) loweralkyl, or
iii) loweralkenyl;
B is
i) $-(CH_2)_m-$,
ii) substituted alkenylene,
iii) $-QCH_2-$ wherein Q is 0, S, or
$-N(R_8)-$ wherein $R_8$ is selected from hydrogen, $-(C=O)_r(C_1-C_6$loweralkyl), $-(C=O)_r$, cycloalkyl, $-(C=O)-_r$loweralkenyl, $-(C=O)_r(CH_2)_m$aryl, $-(C=O)_r(CH_2)_m$(substituted aryl) wherein substituted aryl is as defined above, $-(CH_2)_m$ $SR_4$ and $-(CH_2)_mOR_4$ wherein $R_4$ is hydrogen, $-(C=O)_r$loweralkyl, $-(C=O)_r$loweralkenyl, --$(C=O)_r$cycloalkyl, $-(C=O)_r(CH_2)_m$aryl or $-(C=O)_r(CH_2)_m$ (substituted aryl),
iv) $-CH_2Q-$ wherein Q is as defined above, or
v) NH;
Z is
i) C=O,
ii) $S(O)_2$
or
iii) C=S;
Ar is a heterocyclic group, aryl or substituted aryl;
D is unsubstituted or substituted indol-3-yl, indolin-3-yl or oxindol-3-yl; and
m is 0 to 4 and r is 0 or 1;
or a pharmaceutically acceptable salt thereof; with the proviso that when the compound is of the formula:

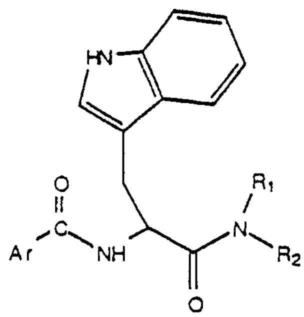

wherein Ar is phenyl or phenyl substituted with one or two substituents independently selected from hydrogen, loweralkyl, hydroxy substituted alkyl, alkoxy substituted alkyl, alkoxy, halogen, amino, hydroxy, nitro, cyano, carboxy, ethoxycarbonyl and trihalomethyl; and $R_1$ is $-(CH_2)_a$phenyl wherein a is 1 to 4; then $R_2$ is not loweralkyl, cycloalkyl, $-(CH_2)_b$aryl wherein b is 0 to 4, $-(CH_2)_b$(substituted aryl) wherein b is 0 to 4 and substituted aryl is as defined above, or $-(CH_2)_c(CO)R_3$ wherein c is 1 to 4 and $R_3$ is hydroxy, alkoxy, $-O(CH_2)_c$aryl, $-O(CH_2)_c$(substituted aryl) wherein c is 1 to 4, or $-NR_6R_7$ wherein $R_6$ and $R_7$ are independently selected from hydrogen and loweralkyl.

2. The compound of Claim 1 wherein $R_1$ and $R_2$ are independently selected from loweralkyl; Z is C=O; B is absent; $R_{11}$ is hydrogen or loweralkyl; D is indol-3-yl, hydroxy-substituted indol-3-yl or halo-substituted indol-3-yl; and Ar is a heterocyclic group selected from indolyl, quinolyl, naphthyl and substituted derivatives thereof.

3. The compound of Claim 1 wherein $R_1$ is loweralkyl; $R_2$ is $-(CH_2)_sO$(loweralkyl) wherein s is 1 to 4; Z is C=O; B is absent; $R_{11}$ is hydrogen or loweralkyl; D is indol-3-yl, hydroxy-substituted indol-3-yl or halo-substituted indol-3-yl; and Ar is a heterocyclic group selected from indolyl, quinolyl, naphthyl and substituted derivatives thereof

4. The compound of Claim 1 wherein $R_1$ is hydrogen or $-(CH_2)_s(C=O)O$(loweralkyl) wherein s is 1 to 4; $R_2$ is benzyl or $-CHR'R''$ wherein $R'$ is carboalkoxy or loweralkyl and $R''$ is phenyl; Z is C=O; B is absent; $R_{11}$ is hydrogen or loweralkyl; D is indol-3-yl, hydroxy-substituted indol-3-yl or halo-substituted indol-3-yl; and Ar is indolyl, quinolyl or naphthyl.

5. A compound selected from the group consisting of:
N-(2′-Indolylcarbonyl)-R-Tryptophan-di-n-pentylamide,
N-(3′-Quinolylcarbonyl)-R-Tryptophan-di-n-pentylamide,
N-(3′-Quinolylcarbonyl)-R-(beta-Oxindolyl)Alanine-di-n-pentylamide
N(alpha)-(3′-Quinolylcarbonyl)-R-5-Hydroxytryptophan-di-n-pentylamide,
N-(4′,8′-Dihydroxy-2′-quinolylcarbonyl)-R-Tryptophan-di-n-pentylamide, and
N(alpha)-(4′,8′-Dihydroxy-2′-quinolylcarbonyl)-R-5-Hydroxytryptophan-di-n-pentylamide.

6. A pharmaceutical composition for antagonizing CCK, comprising a pharmaceutical carrier and a therapeutically effective amount of a compound of Claim 1.

7. A method for antagonizing CCK comprising administering to a host in need of such treatment a therapeutically effective amount of a compound of Claim 1.

8. A method for the treatment and prevention of gastrointestinal ulcers, cancers of the gall bladder and pancreas, pancreatitis, Zollinger-Ellison syndrome, central G cell hyperplasia, irritable bowel syndrome, the treatment or prevention of neuroleptic disorders, tardive dyskinesia, Parkinson's disease, psychosis, Gilles de la Tourette syndrome, disorders of appetite regulatory systems, the treatment of pain and the treatment of substance abuse comprising administering to a host in need of such treatment a therapeutically effective amount of a compound of Claim 1.

9. A pharmaceutical composition for the treatment and prevention of gastrointestinal ulcers, cancers of the gall bladder and pancreas, pancreatitis, Zollinger-Ellison syndrome, central G cell hyperplasia, irritable bowel syndrome, the treatment or prevention of neuroleptic disorders, tardive dyskinesia, Parkinson's disease, psychosis, Gilles de la Tourette syndrome, disorders of appetite regulatory systems, the treatment of pain and the treatment of substance abuse comprising a pharmaceutical carrier and a therapeutically effective amount of a compound of Claim 1.

10. A process for the preparation of a compound of the formula:

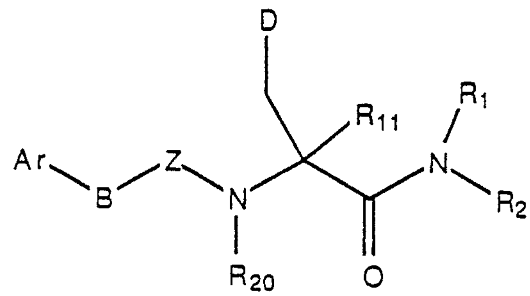

wherein
$R_1$ and $R_2$ are independently selected from
i) hydrogen,
ii) loweralkyl,
iii) cycloalkyl,
iv) loweralkenyl,
v) adamantyl,
vi) aryl,
vii) substituted aryl,
viii) heterocyclic group,
ix) substituted alkyl,
x) substituted amide,
xi) functionalized carbonyl, and
xii) nitrogen containing ring wherein $R_1$ $R_2$ and the adjacent nitrogen atom form a ring;
$R_{11}$ is
i) hydrogen,
ii) loweralkyl, or
iii) loweralkenyl;
$R_{20}$ is
i) hydrogen,
ii) loweralkyl, or
iii) loweralkenyl;
B is
i) $-(CH_2)_m-$,
ii) substituted alkenylene,
iii) $-QCH_2-$ wherein Q is O, S, or
$-N(R_8)-$ wherein $R_8$ is selected from hydrogen, $-(C=O)_r(C_1\text{-}C_6\text{loweralkyl})$, $-(C=O)_r-$, cycloalkyl, $-(C=O)_r$loweralkenyl, $-(C=O)_r(CH_2)_m$aryl, $-(C=O)_r(CH_2)_m$(substituted aryl) wherein substituted aryl is as defined above, $-(CH_2)_mSR_4$ and $-(CH_2)_mOR_4$ wherein $R_4$ is hydrogen, $-(C=O)_r$loweralkyl, $-(C=O)_r$loweralkenyl, $-(C=O)_r$cycloalkyl, $-(C=O)_r(CH_2)_m$aryl or $-(C=O)_r(CH_2)_m$(substituted aryl),
iv) $-CH_2Q$ wherein Q is as defined above, or
v) NH;
Z is
i) C=O,
ii) $S(O)_2$ ,
or
iii) C=S;
Ar is a heterocyclic group, aryl or substituted aryl;
D is unsubstituted or substituted indol-3-yl, indolin-3-yl or oxindol 3-yl; and
m is 0 to 4 and r is 0 ro 1;
or a pharmaceutically acceptable salt thereof; comprising coupling an amine of the formula

a)

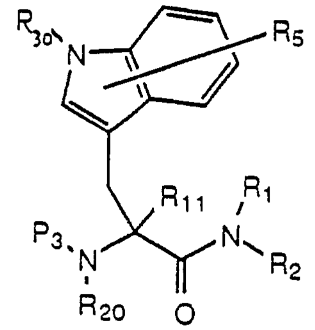

)

b)

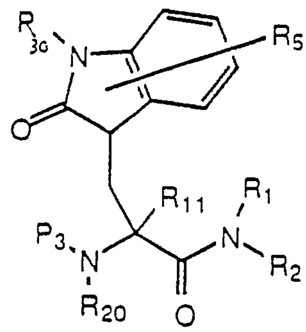

or

c)

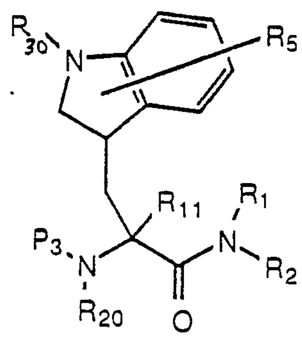

wherein $P_3$ is hydrogen with a compound of the formula

Ar-B-Z-Z′

wherein Z′ is an activating group; or

-B-Z-Z′ taken together represent -N=C=O, -N=C=S, $-CH_2-N=C=O$ or $CH_2-N=C=S$.

11. A process for the preparation of a compound of the formula

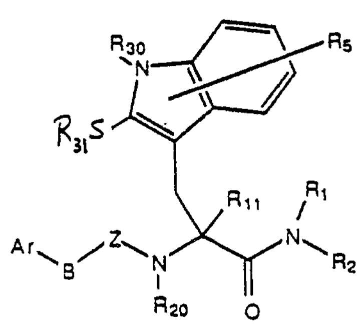

wherein

$R_1$ and $R_2$ are independently selected from

i) hydrogen,

ii) loweralkyl,
iii) cycloalkyl,
iv) loweralkenyl,
v) adamantyl,
vi) aryl,
vii) substituted aryl,
viii) heterocyclic group,
ix) substituted alkyl,
x) substituted amide,
xi) functionalized carbonyl, and
xii) nitrogen containing ring wherein $R_1$, $R_2$ and the adjacent nitrogen atom form a ring; $R_5$ is hydrogen or $R_5$ is one, two or three of the substituents independently selected from the group $-(C_1-C_6$loweralkyl), loweralkenyl, $-(O)_t(CH_2)_m$carboxyl, $-(O)_t(CH_2)_m$carboalkoxy, $-(O)_t(CH_2)_m$carboaryloxy, haloalkyl, nitro, alkoxy, cyano, $-N_3$, $-NHP_4$ wherein $P_4$ is an N-protecting group, $-OP_5$ wherein $P_5$ is an O-protecting group, alkylaminocarbonyl, dialkylaminocarbonyl, aryloxy, thioalkoxy, thioaryloxy, halogen, CN, -OH, $-NH_2$ $-NR_6R_7$ wherein $R_6$ and $R_7$ are independently selected from hydrogen, loweralkyl, cycloalkyl, loweralkenyl, $-(CH_2)_m$aryl, $-(CH_2)_m$(substituted aryl) wherein the aryl group is substituted with one, two or three substituents independently selected from loweralkyl, alkoxy, thioalkoxy, carboxy, carboalkoxy, cyano, haloalkyl, $-N_3$ $-NHP_4$ wherein $P_4$ is an N-protecting group, $-OP_5$ wherein $P_5$ is an O-protecting group, nitro, halogen, hydroxy, amino and -NH(loweralkyl); $-(CH_2)_mOR_4$ wherein $R_4$ is hydrogen, $-(C=O)_r$loweralkyl. $-(C=O)_r$loweralkenyl, $-(C=O)_r$cycloalkyl, $-(C=O)_r(CH_2)_m$aryl or $-(C=O)_r(CH_2)_m$(substituted aryl), and $-(CH_2)_mSR_4$ wherein $R_4$ is independently as defined above;

$R_{11}$ is
i) hydrogen,
ii) loweralkyl, or
iii) loweralkenyl;

$R_{20}$ is
i) hydrogen,
ii) loweralkyl, or
iii) loweralkenyl;

B is
i) $-(CH_2)_m-$,
ii) substituted alkenylene,
iii) $-QCH_2-$ wherein Q is 0, S, or $-N(R_8)-$ wherein $R_8$ is selected from hydrogen, $-(C=O)_r(C_1-C_6$loweralkyl), --$(C=O)_r$-, cycloalkyl, $-(C=O)_r$loweralkenyl, $-(C=O)_r(CH_2)_m$aryl, $-(C=O)_r(CH_2)_m$(substituted aryl) wherein substituted aryl is as defined above, $-(CH_2)_mSR_4$ and $-(CH_2)_mOR_4$ wherein $R_4$ is hydrogen, $-(C=O)_r$loweralkyl, $-(C=O)_r$loweralkenyl, $-(C=O)_r$cycloalkyl, $-(C=O)_r(CH_2)_m$aryl or $-(C=O)_r(CH_2)_m$(substituted aryl),
iv) $-CH_2Q-$ wherein Q is as defined above, or
v) NH;

Z is
i) C=O,
ii) $S(O)_2$ ,
or
iii) C=S;

Ar is a heterocyclic group, aryl or substituted aryl;
$R_{30}$ is hydrogen, loweralkyl, arylalkyl, (substituted aryl)alkyl or an N-protecting group;
$R_{31}$ is loweralkyl, aryl or substituted aryl;
and m, t and r are independently selected at each occurrence from the values m is 0 to 4, t is 0 or and r is 0 or 1;
or a pharmaceutically acceptable salt thereof;
comprising reacting a compound of the formula

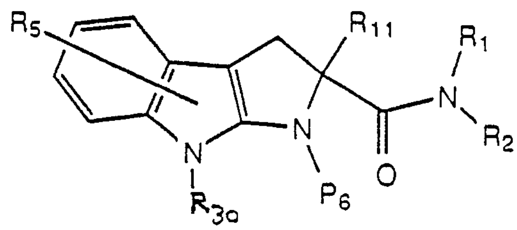

wherein $P_6$ is an N-protecting group; and $R_1$, $R_2$, $R_5$, $R_{11}$ and $R_{30}$ are independently as defined above, with a compound of the formula $R_{31}SH$ wherein $R_{31}$ is loweralkyl, aryl or substituted aryl.